# EUROPEAN PATENT SPECIFICATION

(11) **EP 3 415 508 B1**
(45) Date of publication and mention of the grant of the patent: **21.04.2021**
(21) Application number: 18180522.7
(22) Date of filing: 17.03.2015
(51) Int. Cl.: C07D 401/04, C07D 403/04, C07D 409/14, C07D 405/14, C07D 403/14, C07D 417/14, C07F 7/08, C07D 403/10, C07D 487/04, C07D 495/04, C07D 491/048, C09K 11/06, H01L 51/50

(54) **ELECTRON BUFFERING MATERIAL AND ORGANIC ELECTROLUMINESCENT DEVICE**
ELEKTRONENPUFFERMATERIAL UND ORGANISCHE ELEKTROLUMINESZENTE VORRICHTUNG
MATÉRIAU TAMPON D'ÉLECTRONS ET DISPOSITIF ÉLECTROLUMINESCENT ORGANIQUE

(30) Priority: 17.03.2014 KR 20140031295; 16.03.2015 KR 20150036207
(43) Date of publication of application: 19.12.2018
(62) Divisional of application: 15764804.9
(73) Proprietor: Rohm And Haas Electronic Materials Korea Ltd., Chungcheongnam-do 331-980 (KR)
(72) Inventor: KIM, Chi-Sik, 445-752 Gyeonggi-do (KR); LEE, Hyo-Jung, 445-160 Gyeonggi-do (KR); LEE, Su-Hyun, 440-200 Gyeonggi-do (KR); JUN, Ji-Song, 331-980 Chungcheongnam-do (KR); YANG, Jeong-Eun, 442-870 Gyeonggi-do (KR); SHIM, Jae-Hoon, 331-980 Chungcheongnam-do (KR); CHOI, Kyung-Hoon, 445-160 Gyeonggi-do (KR); CHO, Young-Jun, 463-40 Gyeonggi-do (KR); JEON, Jeong-Hwan, 430-838 Gyeonggi-do (KR); NA, Hong-Yoep, 150-806 Seoul (KR); CHO, Sang-Hee, 443-390 Gyeonggi-do (KR)
(74) Representative: Houghton, Mark Phillip

(56) References cited:
- WO-A1-2013/183851
- WO-A1-2015/084021
- JIANG, H.: "Hosts for High-Performance Phosphorescent Organic Light-Emitting Diodes Based on Carbazole Derivatives", ASIAN J. ORG. CHEM., vol. 3, 4 February 2014 (2014-02-04), pages 102-112, XP002772477,
- JIANG, H. ET AL.: "A Review on Synthesis of Carbazole-based Chromophores as Organic Light-emitting Materials", CURRENT ORGANIC CHEMISTRY, vol. 16, 2012, pages 2014-2025, XP009195057,

## Description

### Technical Field

The present disclosure relates to an electron buffering material and an organic electroluminescent device.

### Background Art

An organic electroluminescent device (OLED) emitting green light was first proposed by Tang et al. of Eastman Kodak in 1987, which employs a double layer of TPD/Alq₃, composed of a light-emitting layer and a charge transport layer. Afterward, an organic electroluminescent device had been rapidly researched, and has now become commercialized. At present, a phosphorescent material, which has excellent luminous efficiency, is mainly used for a panel of an organic electroluminescent device. An organic electroluminescent device emitting red or green light has been successfully commercialized by using a phosphorous material. However, a phosphorous material for emitting blue light has the following disadvantages, which are blocking realization of full color display: roll-off is reduced at high current due to loss of excessively formed excitons, thereby deteriorating performances; the blue-emitting phosphorous material itself has a problem in long term stability of lifespan; and color purity is rapidly decreasing by lapse of time.

A fluorescent material has been used until the present, but has several problems. First, when exposed to high-temperature during a panel production process, a current feature can be changed, which can cause a change in luminance. Furthermore, due to a structural characteristic, an interface feature between a light-emitting layer and an electron injection layer can deteriorate, which can cause a decrease of luminance. In addition, a fluorescent material provides lower efficiencies than a phosphorescent material. Accordingly, there have been attempts to improve efficiencies by developing a specific fluorescent material such as a combination of an anthracene-based host and a pyrene-based dopant. However, the proposed combination makes holes become greatly trapped, which can cause light-emitting sites in a light-emitting layer to shift to the side close to a hole transport layer, thereby light being emitted at an interface. The light-emission at an interface decreases lifespan of a device, and efficiencies are not satisfactory.

It is not easy to solve the aforementioned problems of a fluorescent material by improvement of a light-emitting material itself. Accordingly, recently, there has been research to solve the problems, which include improvement of a charge transport material to change a charge transport feature, and a development of optimized device structure.

Korean Patent Application Laying-Open No. 10-2012-0092550 discloses an organic electroluminescent device in which a blocking layer is interposed between an electron injection layer and a light-emitting layer, wherein the blocking layer comprises an aromatic heterocyclic derivative comprising an azine ring. However, the prior art reference fails to disclose an electron buffering material comprising a compound in which a fluorene is connected to a carbazole or fused with an indole to form a backbone of the compound, and an organic electroluminescent device employing the compound in an electron buffering layer.

WO2013/183851 discloses an organic electroluminescent element compound represented by structural formula 1 or 2 below; 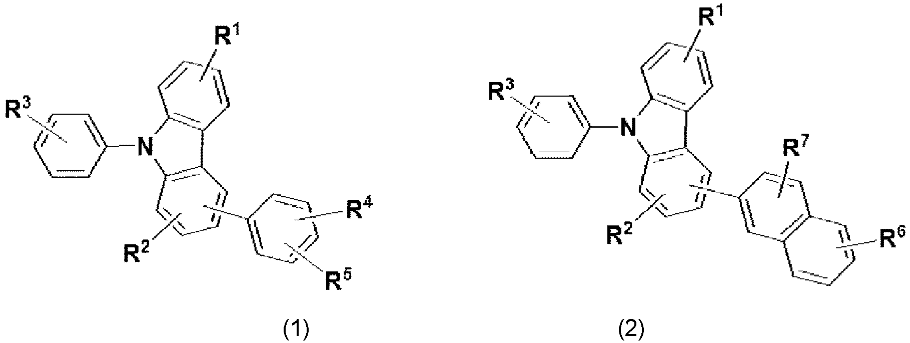 and an organic electroluminescent element comprising same. WO2015/084021 discloses a novel organic electroluminescent compound and an organic electroluminescent device comprising the same. *"*Hosts for High-Performance Phosphorescent Organic Light-Emitting Diodes Based on Carbazole Derivatives" to Jiang, H. in ASIAN J.ORG.CHEM. (2014), Vol. 3, pp.102-112 discloses compounds that contain late transition-metal complexes as emitters are particularly attractive due to their ability to harvest singlet and triplet excitons, which makes it possible to achieve an internal quantum efficiency of 100%. *"*A Review on Synthesis of Carbazole-based Chromophores as Organic Light-emitting Materials" to Jiang, H. et al in CURRENT ORGANIC CHEMISTRY (2012), Vol. 16, pp.2014-2025 discloses different chemical modifying methods to improve the performance of carbazole-based electroluminescent materials including different linkage methods, synthesis of molecules with non-conjugation linkage conformation, bipolar characteristic, heterocycle replacements and multifunctional integration strategies.

### Disclosure of the Invention

### Problems to be Solved

The objective of the present disclosure is to provide an electron buffering material which can provide an organic electroluminescent device having low driving voltage, excellent luminous efficiency, and long lifespan; and an organic electroluminescent device comprising the electron buffering material.

### Solution to Problems

The invention is set out in accordance with the appended claims. The present inventors found that the objective above can be achieved by an electron buffering material comprising a compound represented by the following formula 1; and an organic electroluminescent device comprising a first electrode, a second electrode facing the first electrode, a light-emitting layer between the first electrode and the second electrode, and an electron transport zone and an electron buffering layer between the light-emitting layer and the second electrode, wherein the electron buffering layer comprises a compound represented by the following formula 1. 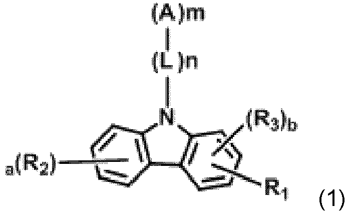 wherein
A represents a substituted or unsubstituted (5- to 30-membered)heteroaryl selected from the group consisting of a substituted or unsubstituted triazinyl, tetrazinyl, triazolyl, tetrazolyl, furazanyl, pyridyl, pyrazinyl, pyrimidinyl, pyridazinyl, benzoimidazolyl, benzothiazolyl, benzoisothiazolyl, benzoisoxazolyl, benzoxazolyl, quinolyl, isoquinolyl, cinnolinyl, quinazolinyl, quinoxalinyl, naphthyridinyl or phenanthridinyl group;
L represents a single bond, a substituted or unsubstituted (C6-C30)arylene, or a substituted or unsubstituted (5- to 30-membered)heteroarylene;
R₁ represents formula 2a: 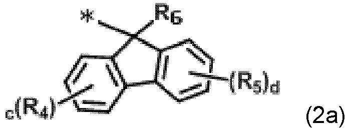
R₂ represents hydrogen, deuterium, a halogen, a cyano, a substituted or unsubstituted (C1-C30)alkyl, a substituted or unsubstituted (C6-C30)aryl, a substituted or unsubstituted (5-to 30-membered)heteroaryl, a substituted or unsubstituted (C3-C30)cycloalkyl, a substituted or unsubstituted (C1-C30)alkoxy, a substituted or unsubstituted tri(C1-C30)alkylsilyl, a substituted or unsubstituted tri(C6-C30)arylsilyl, a substituted or unsubstituted di(C1-C30)alkyl(C6-C30)arylsilyl, a substituted or unsubstituted (C1-C30)alkyldi(C6-C30)arylsilyl, a substituted or unsubstituted mono- or di-(C1-C30)alkylamino, a substituted or unsubstituted mono- or di-(C6-C30)arylamino, a substituted or unsubstituted (C1-C30)alkyl(C6-C30)arylamino, or the following formula 3; or may be fused with the carbazole backbone to form a substituted or unsubstituted benzocarbazole; 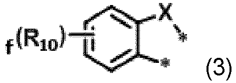
X represents O, S, CR₁₁R₁₂, NR₁₃ or SiR₁₃R₁₄;
R₃ represents hydrogen, deuterium, a halogen, a cyano, a substituted or unsubstituted (C1-C30)alkyl, a substituted or unsubstituted (C6-C30)aryl, a substituted or unsubstituted (5-to 30-membered)heteroaryl, a substituted or unsubstituted (C3-C30)cycloalkyl, a substituted or unsubstituted (C1-C30)alkoxy, a substituted or unsubstituted tri(C1-C30)alkylsilyl, a substituted or unsubstituted tri(C6-C30)arylsilyl, a substituted or unsubstituted di(C1-C30)alkyl(C6-C30)arylsilyl, a substituted or unsubstituted (C1-C30)alkyldi(C6-C30)arylsilyl, a substituted or unsubstituted mono- or di-(C1-C30)alkylamino, a substituted or unsubstituted mono- or di-(C6-C30)arylamino, or a substituted or unsubstituted (C1-C30)alkyl(C6-C30)arylamino;
R₄, R₅ and R₁₀, each independently, represent hydrogen, deuterium, a halogen, a cyano, a substituted or unsubstituted (C1-C30)alkyl, a substituted or unsubstituted (C6-C30)aryl, a substituted or unsubstituted (5- to 30-membered)heteroaryl, a substituted or unsubstituted (C3-C30)cycloalkyl, a substituted or unsubstituted (C1-C30)alkoxy, a substituted or unsubstituted tri(C1-C30)alkylsilyl, a substituted or unsubstituted tri(C6-C30)arylsilyl, a substituted or unsubstituted di(C1-C30)alkyl(C6-C30)arylsilyl, a substituted or unsubstituted (C1-C30)alkyldi(C6-C30)arylsilyl, a substituted or unsubstituted mono- or di-(C1-C30)alkylamino, a substituted or unsubstituted mono- or di-(C6-C30)arylamino, or a substituted or unsubstituted (C1-C30)alkyl(C6-C30)arylamino; or may be linked to an adjacent substituent(s) to form a substituted or unsubstituted (C3-C30), mono- or polycyclic, alicyclic or aromatic ring whose carbon atom(s) may be replaced with at least one hetero atom selected from nitrogen, oxygen, and sulfur;
R₆ represents a substituted or unsubstituted (C1-C30)alkyl, a substituted or unsubstituted (C6-C30)aryl, a substituted or unsubstituted (5- to 30-membered)heteroaryl, a substituted or unsubstituted (C3-C30)cycloalkyl, a substituted or unsubstituted mono- or di-(C1-C30)alkylamino, a substituted or unsubstituted mono- or di-(C6-C30)arylamino, or a substituted or unsubstituted (C1-C30)alkyl(C6-C30)arylamino;
R₁₁ to R₁₄, each independently, represent a substituted or unsubstituted (C1-C30)alkyl, a substituted or unsubstituted (C6-C30)aryl, or a substituted or unsubstituted (5- to 30-membered)heteroaryl; or may be linked to an adjacent substituent(s) to form a substituted or unsubstituted (C3-C30), mono- or polycyclic, alicyclic or aromatic ring whose carbon atom(s) may be replaced with at least one hetero atom selected from nitrogen, oxygen, and sulfur;
a, c, d and f, each independently, represent an integer of 0 to 4; where a, c, d, e, or f is an integer of 2 or more, each of R₂, R₄, R₅, R₇ or R₁₀ may be the same or different;
b represents an integer of 0 to 3; where b is an integer of 2 or more, each of R₃ may be the same or different;
n represents an integer of 0 or 1; m represents an integer of 1 or 2;
* represents a bonding site to the carbazole backbone; and
the heteroaryl(ene) contains one or more hetero atoms selected from B, N, O, S, P(=O), Si, and P;
wherein the substituents of the substituted (C1-C30)alkyl, the substituted (C1-C30)alkoxy, the substituted (C3-C30)cycloalkyl, the substituted (C6-C30)aryl(ene), the substituted (5- to 30-membered)heteroaryl(ene) and the substituted (C6-C30)aryl(C1-C30)alkyl in A, L, R2 to R6 and R10 to R14 of formula 1 of the present disclosure, each independently, are at least one selected from the group consisting of deuterium, a halogen, a cyano, a carboxy, a nitro, a hydroxy, a (C1-C30)alkyl, a halo(C1-C30)alkyl, a (C2-C30)alkenyl, a (C2-C30)alkynyl, a (C1-C30)alkoxy, a (C1-C30)alkylthio, a (C3-C30)cycloalkyl, a (C3-C30)cycloalkenyl, a (3- to 7-membered)heterocycloalkyl, a (C6-C30)aryloxy, a (C6-C30)arylthio, a (3- to 30-membered)heteroaryl unsubstituted or substituted with a (C6-C30)aryl, a (C6-C30)aryl unsubstituted or substituted with a (3- to 30-membered)heteroaryl, a tri(C1-C30)alkylsilyl, a tri(C6-C30)arylsilyl, a di(C1-C30)alkyl(C6-C30)arylsilyl, a (C1-C30)alkyldi(C6-C30)arylsilyl, an amino, a mono- or di-(C1-C30)alkylamino, a mono- or di-(C6-C30)arylamino, a (C1-C30)alkyl(C6-C30)arylamino, a (C1-C30)alkylcarbonyl, a (C1-C30)alkoxycarbonyl, a (C6-C30)arylcarbonyl, a di(C6-C30)arylboronyl, a di(C1-C30)alkylboronyl, a (C1-C30)alkyl(C6-C30)arylboronyl, a (C6-C30)aryl(C1-C30)alkyl, and a (C1-C30)alkyl(C6-C30)aryl.

### Effects of the Invention

By using the electron buffering material of the present disclosure, an organic electroluminescent device can have low driving voltage, excellent luminous efficiency, and long lifespan.

### Brief Description of the Drawings

Fig. 1 is a schematic sectional view illustrating a structure of an organic electroluminescent device according to one embodiment of the present disclosure;
Fig. 2 is an energy band diagram among a hole transport layer, a light-emitting layer, an electron buffering layer, and an electron transport zone of an organic electroluminescent device according to one embodiment of the present disclosure; and
Fig. 3 is a graph illustrating a current efficiency versus a luminance of organic electroluminescent devices of Examples 1 to 3, and Comparative Example 1.

### Embodiments of the Invention

Hereinafter, the present disclosure will be described in detail. However, the following description is intended to explain the invention, and is not meant in any way to restrict the scope of the invention.

LUMO ("Lowest Unoccupied Molecular Orbital") and HOMO ("Highest Occupied Molecular Orbital") have negative energy levels. However, for convenience, LUMO energy level and HOMO energy level are indicated by absolute values in the present disclosure. Thus, upon comparison between the LUMO energy level and the HOMO energy level, the comparison is conducted on the basis of their absolute values. In the present disclosure, the LUMO energy level and the HOMO energy level are calculated by Density Functional Theory (DFT).

Herein, "(C1-C30)alkyl(ene)" indicates a linear or branched alkyl(ene) having 1 to 30, preferably 1 to 20, and more preferably 1 to 10 carbon atoms, and includes methyl, ethyl, n-propyl, isopropyl, n-butyl, isobutyl, tert-butyl, etc. "(C2-C30) alkenyl" indicates a linear or branched alkenyl having 2 to 30, preferably 2 to 20, and more preferably 2 to 10 carbon atoms and includes vinyl, 1-propenyl, 2-propenyl, 1-butenyl, 2-butenyl, 3-butenyl, 2-methylbut-2-enyl, etc. "(C2-C30)alkynyl" indicates a linear or branched alkynyl having 2 to 30, preferably 2 to 20, and more preferably 2 to 10 carbon atoms and includes ethynyl, 1-propynyl, 2-propynyl, 1-butynyl, 2-butynyl, 3-butynyl, 1-methylpent-2-ynyl, etc. "(C3-C30)cycloalkyl" indicates a mono- or polycyclic hydrocarbon having 3 to 30, preferably 3 to 20, and more preferably 3 to 7 carbon atoms and includes cyclopropyl, cyclobutyl, cyclopentyl, cyclohexyl, etc. "(3- to 7-membered) heterocycloalkyl" indicates a cycloalkyl having 3 to 7, preferably 5 to 7 ring backbone atoms including at least one hetero atom selected from B, N, O, S, P(=O), Si, and P, preferably O, S, and N, and includes tetrahydrofuran, pyrrolidine, thiolan, tetrahydropyran, etc. Furthermore, "(C6-C30)aryl(ene)" indicates a monocyclic or fused ring derived from an aromatic hydrocarbon and having 6 to 30, preferably 6 to 20, and more preferably 6 to 15 ring backbone carbon atoms, and includes phenyl, biphenyl, terphenyl, naphthyl, binaphthyl, phenylnaphthyl, naphthylphenyl, fluorenyl, phenylfluorenyl, benzofluorenyl, dibenzofluorenyl, phenanthrenyl, phenylphenanthrenyl, anthracenyl, indenyl, triphenylenyl, pyrenyl, tetracenyl, perylenyl, chrysenyl, naphthacenyl, fluoranthenyl, spirofluorenyl, etc. "(5- to 30-membered) heteroaryl(ene)" indicates an aryl group having 5 to 30 ring backbone atoms including at least one, preferably 1 to 4, hetero atom selected from the group consisting of B, N, O, S, P(=O), Si, and P; may be a monocyclic ring, or a fused ring condensed with at least one benzene ring; may be partially saturated; may be one formed by linking at least one heteroaryl or aryl group to a heteroaryl group via a single bond(s); and includes a monocyclic ring-type heteroaryl such as furyl, thiophenyl, pyrrolyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl, pyridazinyl, etc., and a fused ring-type heteroaryl such as benzofuranyl, benzothiophenyl, isobenzofuranyl, dibenzofuranyl, dibenzothiophenyl, benzonaphthofuranyl, benzonaphthothiophenyl, benzoimidazolyl, benzothiazolyl, benzoisothiazolyl, benzoisoxazolyl, benzoxazolyl, isoindolyl, indolyl, indazolyl, benzothiadiazolyl, quinolyl, isoquinolyl, cinnolinyl, quinazolinyl, quinoxalinyl, naphthyridinyl, carbazolyl, benzocarbazolyl, phenoxazinyl, phenanthridinyl, benzodioxolyl, dihydroacridinyl, etc. Furthermore, "halogen" includes F, CI, Br, and I.

Herein, "substituted" in the expression, "substituted or unsubstituted," means that a hydrogen atom in a certain functional group is replaced with another atom or group, i.e. a substituent. The substituents of the substituted (C1-C30)alkyl, the substituted (C1-C30)alkoxy, the substituted (C3-C30)cycloalkyl, the substituted (C6-C30)aryl(ene), the substituted (5- to 30-membered)heteroaryl(ene) and the substituted (C6-C30)aryl(C1-C30)alkyl in A, L, and R₂ to R₁₄ of formula 1 of the present disclosure, each independently, are at least one selected from the group consisting of deuterium, a halogen, a cyano, a carboxy, a nitro, a hydroxy, a (C1-C30)alkyl, a halo(C1-C30)alkyl, a (C2-C30)alkenyl, a (C2-C30)alkynyl, a (C1-C30)alkoxy, a (C1-C30)alkylthio, a (C3-C30)cycloalkyl, a (C3-C30)cycloalkenyl, a (3- to 7-membered)heterocycloalkyl, a (C6-C30)aryloxy, a (C6-C30)arylthio, a (3- to 30-membered)heteroaryl unsubstituted or substituted with a (C6-C30)aryl, a (C6-C30)aryl unsubstituted or substituted with a (3- to 30-membered)heteroaryl, a tri(C1-C30)alkylsilyl, a tri(C6-C30)arylsilyl, a di(C1-C30)alkyl(C6-C30)arylsilyl, a (C1-C30)alkyldi(C6-C30)arylsilyl, an amino, a mono- or di-(C1-C30)alkylamino, a mono- or di-(C6-C30)arylamino, a (C1-C30)alkyl(C6-C30)arylamino, a (C1-C30)alkylcarbonyl, a (C1-C30)alkoxycarbonyl, a (C6-C30)arylcarbonyl, a di(C6-C30)arylboronyl, a di(C1-C30)alkylboronyl, a (C1-C30)alkyl(C6-C30)arylboronyl, a (C6-C30)aryl(C1-C30)alkyl, and a (C1-C30)alkyl(C6-C30)aryl; and preferably, each independently, are at least one selected from the group consisting of a (C1-C30)alkyl, a (3- to 30-membered)heteroaryl unsubstituted or substituted with a (C6-C30)aryl, a (C6-C30)aryl unsubstituted or substituted with a (3- to 30-membered)heteroaryl, a (C6-C30)aryl(C1-C30)alkyl, and a (C1-C30)alkyl(C6-C30)aryl.

According to one aspect of the present disclosure, an electron buffering material comprising the compound represented by formula 1 is provided. The electron buffering material indicates a material controlling an electron flow. Therefore, the electron buffering material may be, for example, a material trapping electrons, blocking electrons, or lowering an energy barrier between an electron transport zone and a light-emitting layer. Specifically, the electron buffering material may be for an organic electroluminescent device. In the organic electroluminescent device, the electron buffering material may be used for preparing an electron buffering layer, or may be added to another area such as an electron transport zone or a light-emitting layer. The electron buffering layer may be formed between a light-emitting layer and an electron transport zone, or between an electron transport zone and a second electrode of an organic electroluminescent device. The electron buffering material may be a mixture or composition which may further comprise a conventional material for preparing an organic electroluminescent device.

The compound of formula 1 may be represented, preferably, by any one of the following formulae 4 to 10, and specifically, by the following formula 4, 5, or 7. 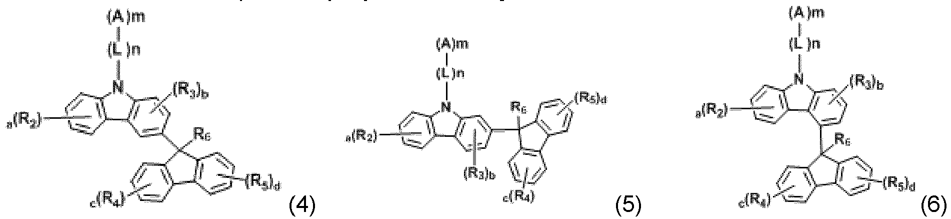 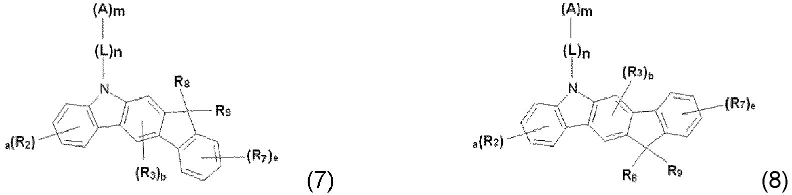 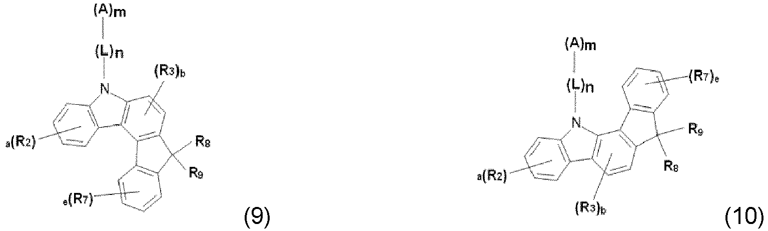 wherein, A, L, R₂ to R₉, a, b, c, d, e, m, and n are as defined in formula 1.

In formulae 1 and 4 to 10, A may represent preferably, a substituted or unsubstituted nitrogen-containing (5- to 30-membered)heteroaryl; and, more preferably, a substituted or unsubstituted nitrogen-containing (6- to 20-membered)heteroaryl. Specifically, A may represent a substituted or unsubstituted pyridyl, a substituted or unsubstituted pyrimidinyl, a substituted or unsubstituted triazinyl, a substituted or unsubstituted pyrazinyl, a substituted or unsubstituted quinolyl, a substituted or unsubstituted isoquinolyl, a substituted or unsubstituted quinolinyl, a substituted or unsubstituted quinazolinyl, a substituted or unsubstituted quinoxalinyl, or a substituted or unsubstituted naphthyridinyl; and more specifically, a substituted or unsubstituted pyridyl, a substituted or unsubstituted pyrimidinyl, a substituted or unsubstituted triazinyl, or a substituted or unsubstituted pyrazinyl. Preferably, the substituent of the substituted heteroaryl of A may be at least one selected from the group consisting of a (C1-C10)alkyl; a (C6-C20)aryl unsubstituted or substituted with deuterium, a cyano, a halogen, a (C1-C10)alkyl, a (C6-C20)cycloalkyl, a (C6-C20)aryl, a (6- to 20-membered)heteroaryl, a di(C6-C20)arylamino, or a tri(C6-C20)arylsilyl; and, a (6- to 20-membered)heteroaryl unsubstituted or substituted with deuterium, a cyano, a halogen, a (C1-C10)alkyl, a (C6-C20)cycloalkyl, a (C6-C20)aryl, a (6- to 20-membered)heteroaryl, a di(C6-C20)arylamino, or a tri(C6-C20)arylsilyl.

In formulae 1 and 4 to 10, L may represent preferably, a single bond or a substituted or unsubstituted (C6-C20)arylene; and more preferably, a single bond or an unsubstituted (C6-C18)arylene. Specifically, L may represent a single bond, a substituted or unsubstituted phenyl, a substituted or unsubstituted naphthyl, a substituted or unsubstituted biphenyl, a substituted or unsubstituted terphenyl, a substituted or unsubstituted phenylnaphthyl, or a substituted or unsubstituted naphthylphenyl.

In formulae 1 and 4 to 10, R₂ may represent preferably, hydrogen, deuterium, a substituted or unsubstituted (C6-C20)aryl, a substituted or unsubstituted (5- to 20-membered)heteroaryl, a substituted or unsubstituted mono- or di-(C6-C20)arylamino, or formula 3, or may be fused with the carbazole backbone to form a substituted or unsubstituted benzocarbazole. Specifically, R₂ may represent hydrogen, or a substituted or unsubstituted (C6-C20)aryl. More specifically, R₂ may represent hydrogen, a substituted or unsubstituted phenyl, a substituted or unsubstituted dibenzothiophenyl, a substituted or unsubstituted dibenzofuranyl, a substituted or unsubstituted carbazolyl, a substituted or unsubstituted diphenylamino, a substituted or unsubstituted fluorenyl, or formula 3, or may be fused with the carbazole backbone to form a substituted or unsubstituted benzocarbazole.

In formula 3, X may represent preferably, O, S, CR₁₁R₁₂, or NR₁₃. R₁₀ may represent preferably, hydrogen or a (C1-C20)alkyl. R₁₁ to R₁₄, each independently, may represent preferably, hydrogen, a substituted or unsubstituted (C1-C10)alkyl, a substituted or unsubstituted (C6-C20)aryl, or a substituted or unsubstituted (5- to 20-membered)heteroaryl; and specifically, hydrogen, a (C1-C6)alkyl, phenyl, naphthyl, or biphenyl.

In formulae 1 and 4 to 10, R₃, R₄, R₅, and R₇, each independently, may represent preferably, hydrogen or a substituted or unsubstituted (C1-C20)alkyl. Specifically, R₃, R₄, R₅, and R₇ may represent hydrogen.

In formulae 1 and 4 to 10, R₆, R₈, and R₉, each independently, may represent preferably, a substituted or unsubstituted (C1-C20)alkyl, a substituted or unsubstituted (C6-C20)aryl, or a substituted or unsubstituted (6- to 20-membered)heteroaryl; and more preferably, a substituted or unsubstituted (C6-C20)aryl. Specifically, R₆, R₈, and R₉, each independently, may represent a substituted or unsubstituted phenyl, a substituted or unsubstituted biphenyl, or a substituted or unsubstituted naphthyl.

In formulae 1 and 4 to 10, specifically, a may represent 0 or 1; b may represent 0; c, d, e, and f, each independently, may represent an integer of 0 to 2; n may represent 0 or 1; and m may represent 1.

According to one embodiment of the present disclosure, A represents a substituted or unsubstituted nitrogen-containing (5- to 30-membered)heteroaryl; L represents a single bond or a substituted or unsubstituted (C6-C20)arylene selected from the group consisting of a substituted or unsubstituted triazinyl, tetrazinyl, triazolyl, tetrazolyl, furazanyl, pyridyl, pyrazinyl, pyrimidinyl, pyridazinyl, benzoimidazolyl, benzothiazolyl, benzoisothiazolyl, benzoisoxazolyl, benzoxazolyl, quinolyl, isoquinolyl, cinnolinyl, quinazolinyl, quinoxalinyl, naphthyridinyl or phenanthridinyl group; R₁ is represented by formula 2a; R₂ represents hydrogen, deuterium, a substituted or unsubstituted (C6-C20)aryl, a substituted or unsubstituted (5- to 20-membered)heteroaryl, a substituted or unsubstituted mono- or di-(C6-C20)arylamino or formula 3, or is fused with the carbazole backbone to form a substituted or unsubstituted benzocarbazole; X represents O, S, CR₁₁R₁₂, or NR₁₃; R₁₀ represents hydrogen or a (C1-C20)alkyl; R₃, R₄, R₅, and R₇, each independently, represent hydrogen or a substituted or unsubstituted (C1-C20)alkyl; R₆, R₈ and R₉, each independently, represent a substituted or unsubstituted (C1-C20)alkyl, a substituted or unsubstituted (C6-C20)aryl, or a substituted or unsubstituted (6- to 20-membered)heteroaryl; R₁₁ to R₁₄, each independently, represent hydrogen, a substituted or unsubstituted (C1-C10)alkyl, a substituted or unsubstituted (C6-C20)aryl, or a substituted or unsubstituted (5- to 20-membered)heteroaryl; and the heteroaryl contains one or more hetero atoms selected from N, O, and S.

Specifically, the compound of formula 1 includes the following, but is not limited thereto.

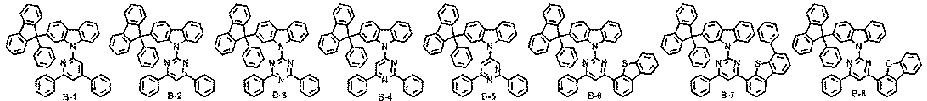

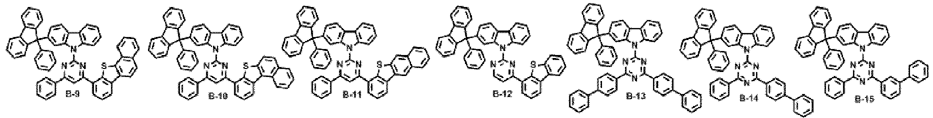

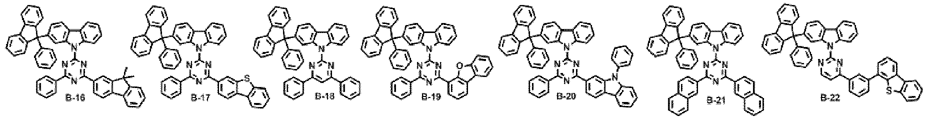

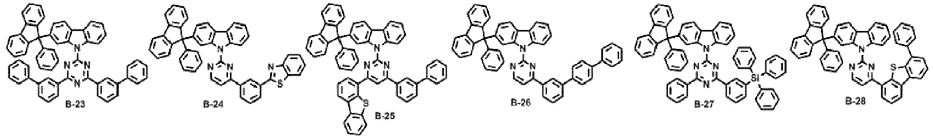

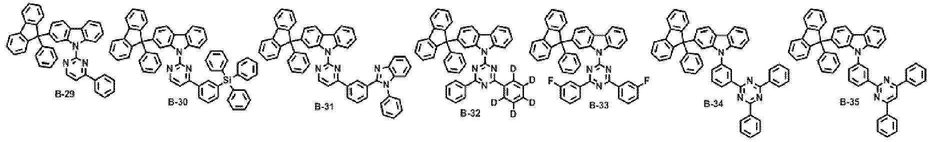

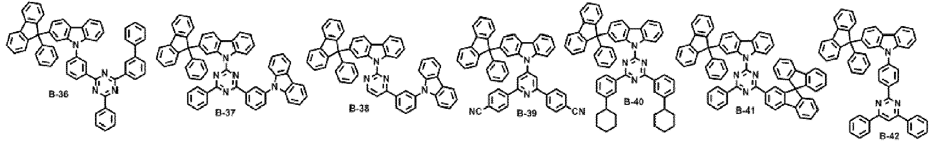

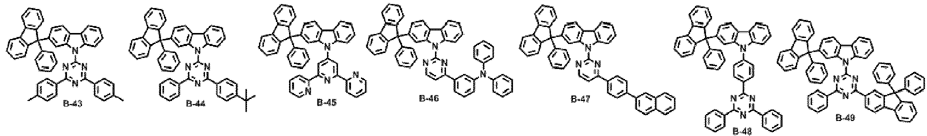

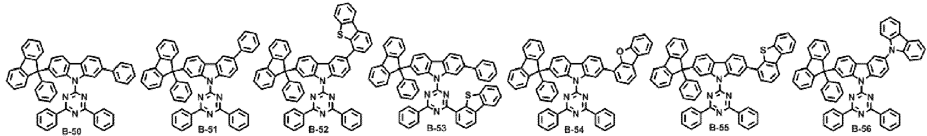

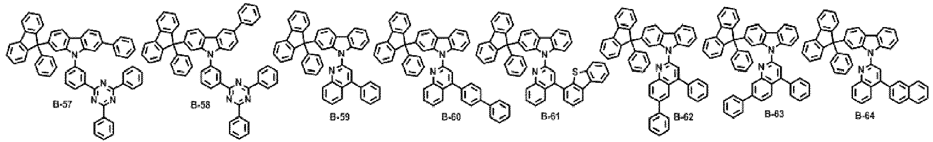

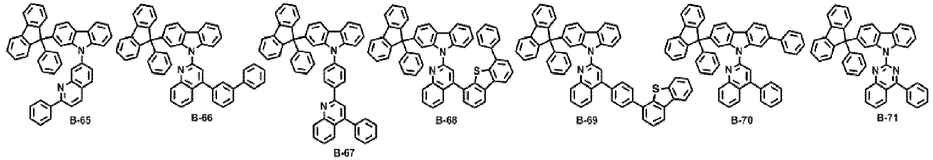

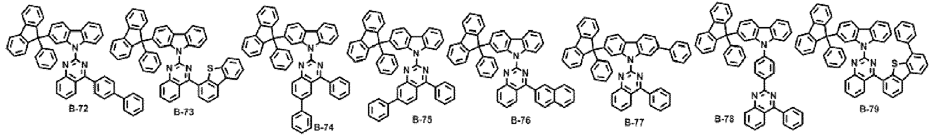

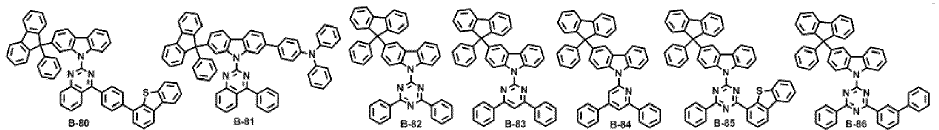

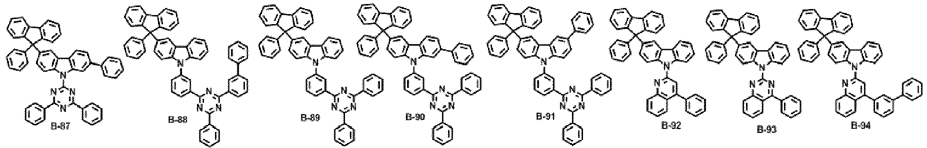

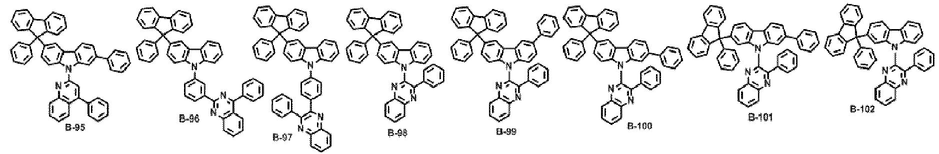

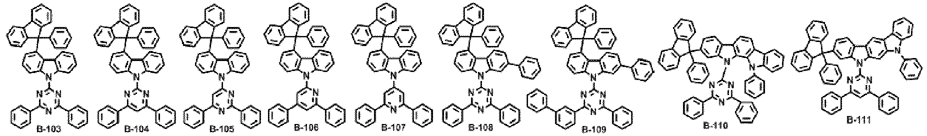

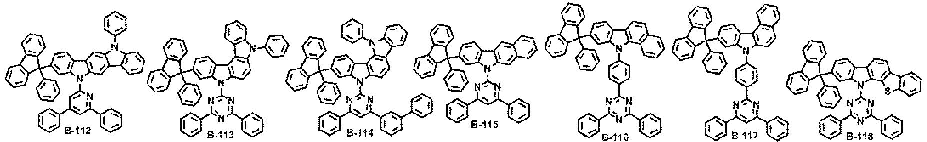

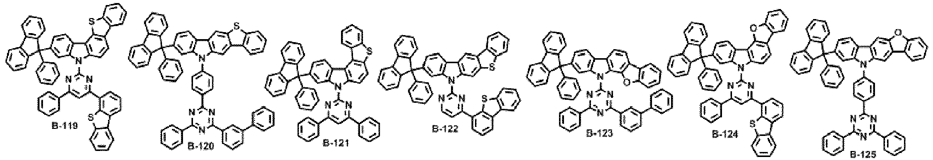

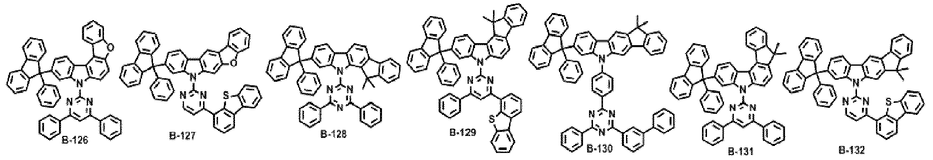

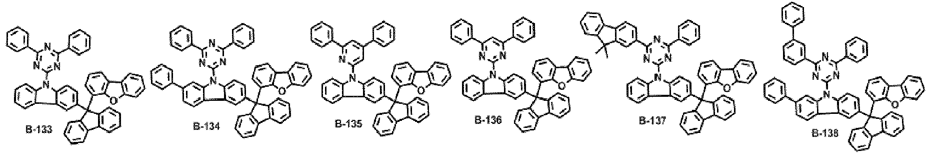

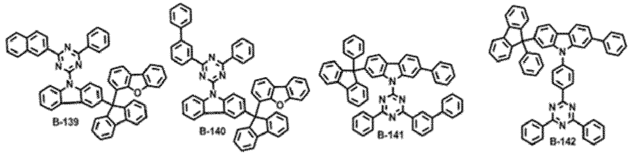

The compound of the present disclosure can be prepared by a synthetic method known to one skilled in the art. For example, it can be prepared according to the following reaction scheme 1 or 2.

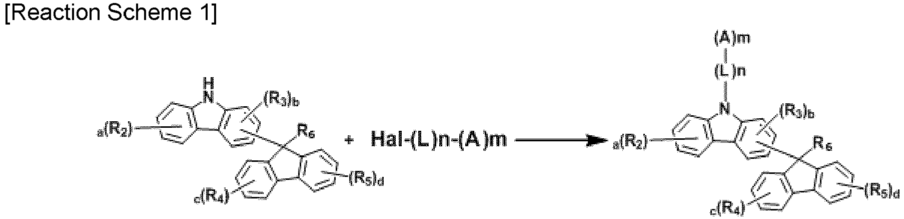

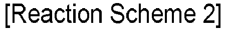

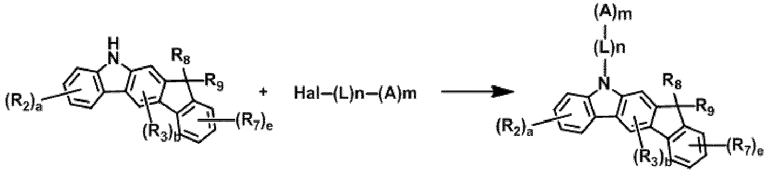

In reaction schemes 1 and 2 above, A, L, R₂ to R₉, a, b, c, d, e, m, and n are as defined in formula 1, and Hal represents a halogen. For more specific methods for preparing the compound of the present disclosure, please refer to Korean Patent Application No. 2013-0149733.

The electron buffering material may further comprise a compound represented by the following formula 11. 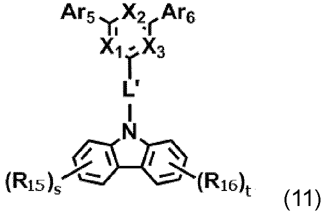 wherein
Ar₅ and Ar₆, each independently, represent a substituted or unsubstituted (5- to 30-membered)heteroaryl, or a substituted or unsubstituted (C6-C30)aryl;
L' represents a single bond, a substituted or unsubstituted (C6-C30)arylene, or a substituted or unsubstituted (3- to 30-membered)heteroarylene;
X₁ to X₃, each independently, represent N or C, with the proviso that at least one of X₁ to X₃ represents N;
R₁₅ and R₁₆, each independently, represent hydrogen, deuterium, a halogen, a cyano, a carboxy, a nitro, a hydroxy, a substituted or unsubstituted (C1-C30)alkyl, a substituted or unsubstituted (C6-C30)aryl, a substituted or unsubstituted (5- to 30-membered)heteroaryl, a substituted or unsubstituted (C3-C30)cycloalkyl, a substituted or unsubstituted (C3-C30)cycloalkenyl, a substituted or unsubstituted (3- to 7-membered)heterocycloalkyl, a substituted or unsubstituted (C1-C30)alkoxy, a substituted or unsubstituted tri(C1-C30)alkylsilyl, a substituted or unsubstituted tri(C6-C30)arylsilyl, a substituted or unsubstituted di(C1-C30)alkyl(C6-C30)arylsilyl, a substituted or unsubstituted (C1-C30)alkyldi(C6-C30)arylsilyl, a substituted or unsubstituted mono- or di-(C1-C30)alkylamino, a substituted or unsubstituted mono- or di-(C6-C30)arylamino, or a substituted or unsubstituted (C1-C30)alkyl(C6-C30)arylamino, or may be linked to an adjacent substituent(s) to form a substituted or unsubstituted (C3-C30), mono- or polycyclic, alicyclic or aromatic ring whose carbon atom(s) may be replaced with at least one hetero atom selected from nitrogen, oxygen, and sulfur;
the heteroaryl(ene) and heterocycloalkyl, each independently, contain one or more hetero atoms selected from B, N, O, S, P(=O), Si and P; and
s and t, each independently, represent an integer of 1 to 4; where s or t is an integer of 2 or more, each of R₁₅ or R₁₆ may be the same or different.

In formula 11, preferably, Ar₅ and Ar₆, each independently, may represent a substituted or unsubstituted (6- to 20-membered)heteroaryl, or a substituted or unsubstituted (C6-C20)aryl. Specifically, Ar₅ and Ar₆, each independently, represent a substituted or unsubstituted phenyl, a substituted or unsubstituted biphenyl, a substituted or unsubstituted naphthyl, a substituted or unsubstituted terphenyl, a substituted or unsubstituted phenylnaphthyl, a substituted or unsubstituted naphthylphenyl, a substituted or unsubstituted anthracenyl, a substituted or unsubstituted phenanthrenyl, a substituted or unsubstituted fluorenyl, a substituted or unsubstituted carbazolyl, a substituted or unsubstituted dibenzothiophenyl, or a substituted or unsubstituted dibenzofuranyl. Preferably, the substituents of the substituted heteroaryl and the substituted aryl in Ar₅ and Ar₆, each independently, may be a (C1-C10)alkyl, a (C6-C20)aryl, a (6- to 20-membered)heteroaryl, or a di(C6-C20)arylamino.

In formula 11, preferably, L' may represent a single bond, or a substituted or unsubstituted (C6-C20)arylene. Specifically, L' may represent a single bond, a substituted or unsubstituted phenylene, a substituted or unsubstituted naphthylene, or a substituted or unsubstituted biphenylene.

In formula 11, preferably, R₁₅ and R₁₆, each independently, may represent hydrogen, a substituted or unsubstituted (C6-C20)aryl, or a substituted or unsubstituted (5- to 20-membered)heteroaryl, or may be linked to an adjacent substituent(s) to form a substituted or unsubstituted (C5-C20), mono- or polycyclic aromatic ring whose carbon atom(s) may be replaced with at least one hetero atom selected from nitrogen, oxygen, and sulfur; and s and t represent 1. In formula 11, R₁₅ and R₁₆ may not be a substituted or unsubstituted fluorenyl. In formula 11, R₁₅ and R₁₆, each independently, may not form a substituted or unsubstituted fluorene ring with a benzene ring of the carbazole backbone linked to R₁₅ and R₁₆. Specifically, R₁₅ and R₁₆, each independently, may represent hydrogen, a substituted or unsubstituted phenyl, a substituted or unsubstituted naphthyl, a substituted or unsubstituted biphenyl, a substituted or unsubstituted dibenzothiophenyl, a substituted or unsubstituted dibenzofuranyl, or a substituted or unsubstituted carbazolyl, or may be linked to an adjacent substituent(s) to form a substituted or unsubstituted benzene ring, a substituted or unsubstituted indene ring, a substituted or unsubstituted indole ring, a substituted or unsubstituted benzothiophene ring, or a substituted or unsubstituted benzofuran ring. Preferably, the substituents of the substituted groups such as the substituted heteroaryl and the substituted aryl in R₁₅ and R₁₆, each independently, may be a (C1-C10)alkyl, a (C6-C20)aryl, a (6- to 20-membered)heteroaryl unsubstituted or substituted with a (C6-C20)aryl, a di(C6-C20)arylamino, or a tri(C6-C20)arylsilyl.

According to one embodiment of the present disclosure, Ar₅ and Ar₆, each independently, may represent a substituted or unsubstituted (6- to 20-membered)heteroaryl, or a substituted or unsubstituted (C6-C20)aryl; L' represents a single bond, or a substituted or unsubstituted (C6-C20)arylene; R₁₅ and R₁₆, each independently, represent hydrogen, a substituted or unsubstituted (C6-C20)aryl, or a substituted or unsubstituted (5- to 20-membered)heteroaryl, or may be linked to an adjacent substituent(s) to form a substituted or unsubstituted (C5-C20), mono- or polycyclic aromatic ring whose carbon atom(s) may be replaced with at least one hetero atom selected from nitrogen, oxygen, and sulfur; and s and t may represent 1.

Specifically, the compound of formula 11 includes the following, but is not limited thereto:

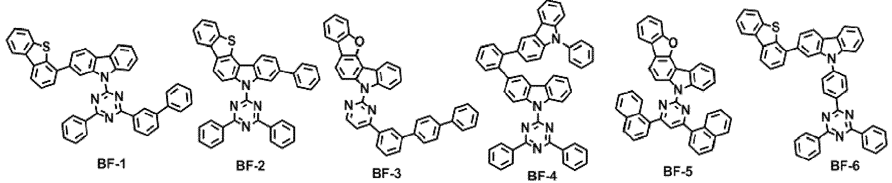

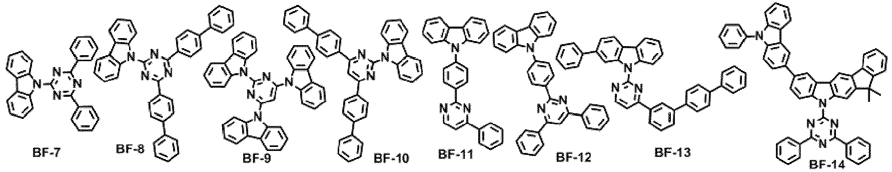

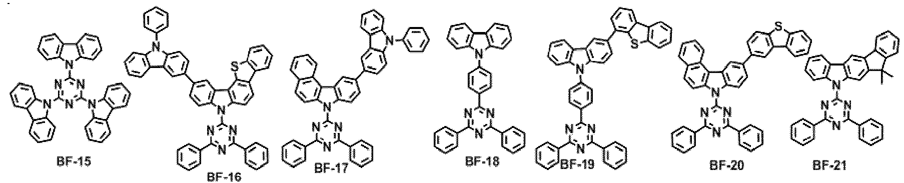

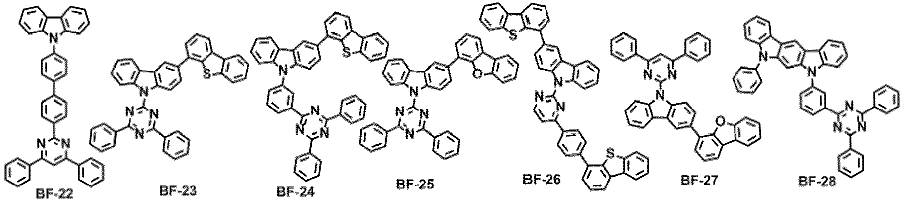

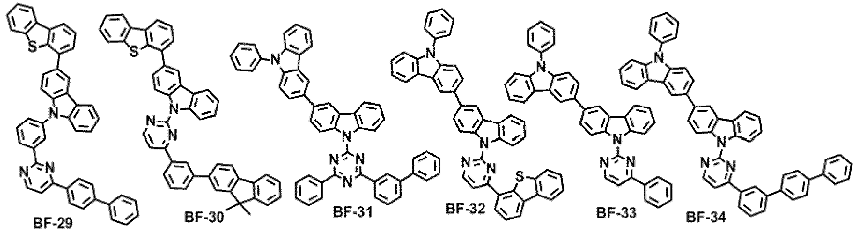

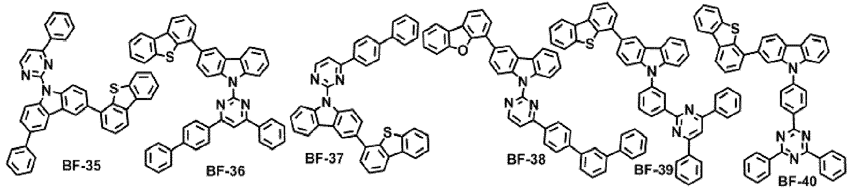

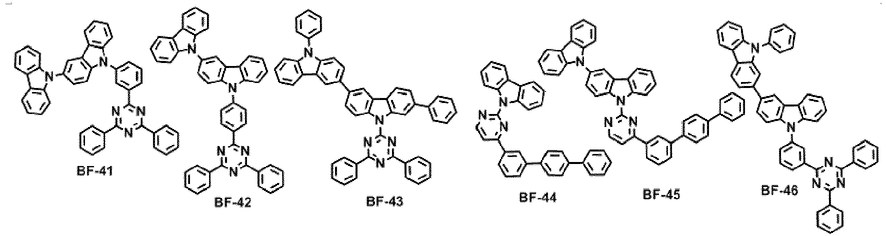

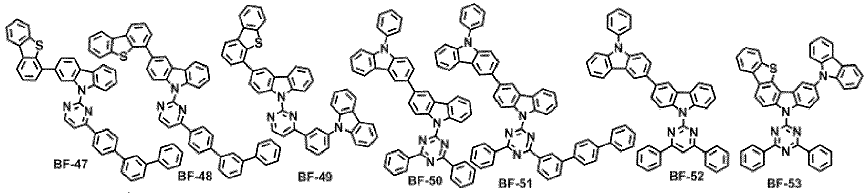

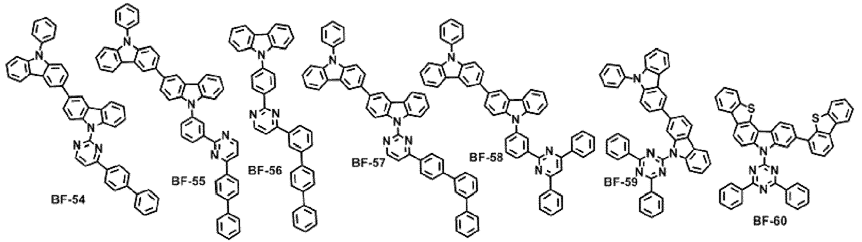

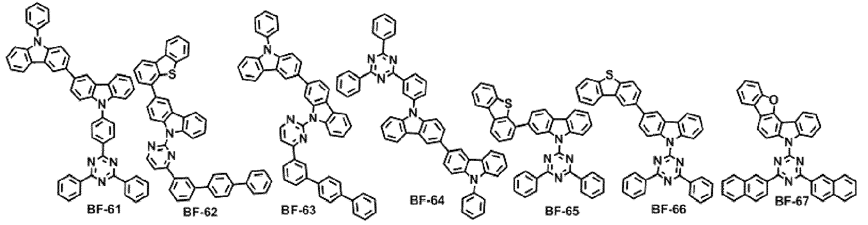

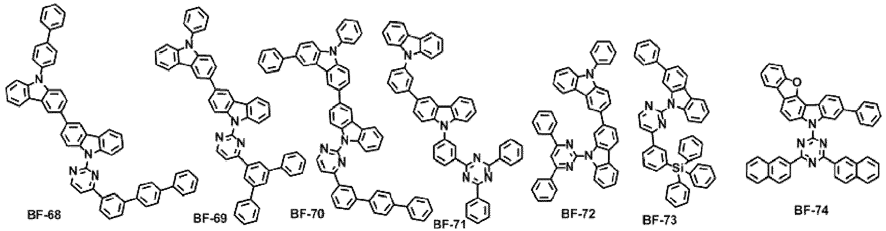

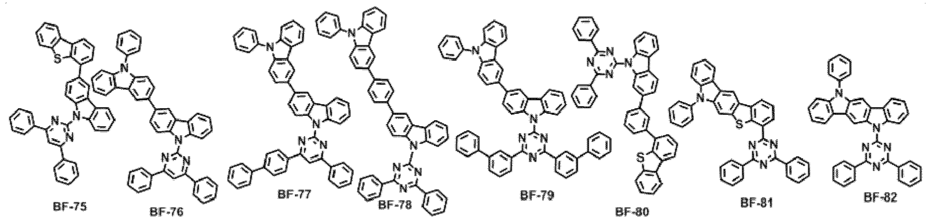

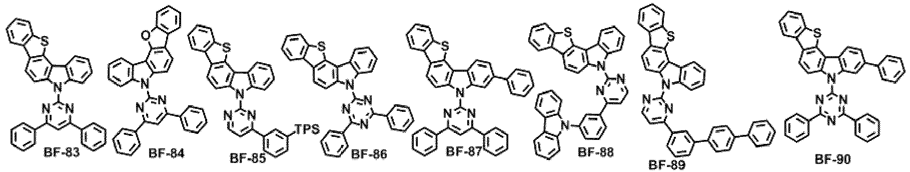

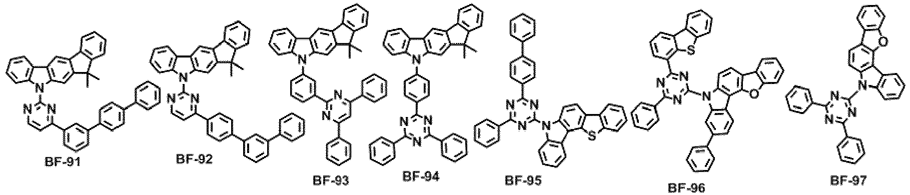

According to another aspect of the present disclosure, it is provided an organic electroluminescent device comprising a first electrode, a second electrode facing the first electrode, a light-emitting layer between the first electrode and the second electrode, and an electron transport zone and an electron buffering layer between the light-emitting layer and the second electrode, wherein the electron buffering layer comprises the compound represented by formula 1 above.

By interposing the electron buffering layer between the light-emitting layer and the second electrode in the organic electroluminescent device comprising the first and second electrodes and the light-emitting layer, an electron injection can be controlled by electron affinity LUMO energy of the electron buffering layer.

The electron buffering layer in the organic electroluminescent device may further comprise the compound of formula 11, in addition to the compound of formula 1. The weight ratio between the compound of formula 1 and compound of formula 11 is in the range of 1:99 to 99:1, preferably 10:90 to 90:10, and more preferably 30:70 to 70:30 in view of driving voltage and luminous efficiency.

When the electron buffering layer comprises both the compound of formula 1 and the compound of formula 11, these compounds for the electron buffering layer may be co-evaporated or mixture-evaporated. Herein, a co-evaporation indicates a process for two or more materials to be deposited as a mixture, by introducing each of the two or more materials into respective crucible cells, and applying electric current to the cells for each of the materials to be evaporated. Herein, a mixture-evaporation indicates a process for two or more materials to be deposited as a mixture, by mixing the two or more materials in one crucible cell before the deposition, and applying electric current to the cell for the mixture to be evaporated.

In the organic electroluminescent device, a light-emitting layer may comprise a host compound and a dopant compound. The host compound may be a phosphorescent host compound or a fluorescent host compound. The dopant compound may be a phosphorescent dopant compound or a fluorescent dopant compound. Preferably, the host compound and the dopant compound may be a fluorescent host compound and a fluorescent dopant compound, respectively. LUMO energy level of the electron buffering layer may be higher than LUMO energy level of the host compound. Specifically, the difference in LUMO energy levels between the electron buffering layer and the host compound may be 0.7 eV or less. For example, LUMO energy levels of the electron buffering layer and the host compound may be 1.9 eV and 1.6 eV, respectively, and thus the difference in LUMO energy level may be 0.3 eV. Although LUMO barrier between the host compound and the electron buffering layer can cause an increase in driving voltage, electrons can be more easily transferred to the host compound due to the compound of formula 1 comprised in the electron buffering layer. Therefore, the organic electroluminescent device of the present disclosure can have low driving voltage, high luminous efficiency, and long lifespan. Herein, specifically, LUMO energy level of an electron buffering layer may indicate the level of the compound of formula 1 comprised in the electron buffering layer.

LUMO energy level of the electron buffering layer may be preferably in the range of 1.6 to 2.3 eV, and more preferably in the range of 1.75 to 2.05 eV. When LUMO energy level of the electron buffering layer is in the range as above, electrons cannot be easily injected to another layer. However, if the electron buffering layer comprises the compound of formula 1, the organic electroluminescent device can have low driving voltage, high luminous efficiency, and long lifespan.

In the organic electroluminescent device of the present disclosure, the electron transport zone indicates a zone transporting electrons from the second electrode to the light-emitting layer. The electron transport zone may comprise an electron transport compound, a reductive dopant, or a combination thereof. The electron transport compound may be at least one selected from the group consisting of oxazole-based compounds, isoxazole-based compounds, triazole-based compounds, isothiazole-based compounds, oxadiazole-based compounds, thiadiazole-based compounds, perylene-based compounds, anthracene-based compounds, aluminum complexes, and gallium complexes. The reductive dopant may be at least one selected from the group consisting of alkali metals, alkali metal compounds, alkaline earth metals, rare-earth metals, and halides, oxides, and complexes thereof. The electron transport zone may comprise an electron transport layer, an electron injection layer, or both of them. The electron transport layer and the electron injection layer, each independently, may be composed of two or more layers. LUMO energy level of the electron buffering layer may be higher or lower than LUMO energy level of the electron transport zone. For example, the electron buffering layer and the electron transport zone may have LUMO energy levels of 1.9 eV and 1.8 eV, respectively, and a difference between them in LUMO energy level may be 0.1 eV. When the electron buffering layer has LUMO energy level as in said numerical range, electrons can be easily injected to a light-emitting layer through the electron buffering layer. LUMO energy level of the electron transport zone may be 1.7 eV or more, or 1.9 eV or more. In the present disclosure, specifically, LUMO energy level of the electron transport zone may indicate the level of an electron transport material comprised in the electron transport zone. When the electron transport zone has two or more layers, LUMO energy level of the electron transport zone may be the one of a material comprised in a layer which is in the electron transport zone and is adjacent to the electron buffering layer.

Specifically, LUMO energy level of the electron buffering layer may be higher than those of the host compound and the electron transport zone. For example, LUMO energy levels may have the following relationship: the electron buffering layer > the electron transport zone > the host compound. According to the aforementioned LUMO relationship, electrons are trapped between the light-emitting layer and the electron buffering layer, which inhibits an injection of electrons to a light-emitting layer, and thus can cause an increase in driving voltage. However, an electron buffering layer comprising the compound of formula 1 can easily transport electrons to the light-emitting layer, and thus the organic electroluminescent device of the present disclosure can have low driving voltage, high luminous efficiency, and long lifespan.

LUMO energy level can be easily measured by known various methods. Generally, cyclic voltametry or ultraviolet photoelectron spectroscopy (UPS) may be used. Therefore, one skilled in the art can easily understand and determine the electron buffering layer, the host material, and the electron transport zone which satisfy the aforementioned relationship for LUMO energy levels, so that he/she can easily practice the invention. HOMO energy level can be easily measured in the same manner as LUMO energy level.

The layers of the organic electroluminescent device of the present disclosure can be formed in the order of the light-emitting layer, the electron buffering layer, the electron transport zone, and the second electrode, or in the order of the light-emitting layer, the electron transport zone, the electron buffering layer, and the second electrode.

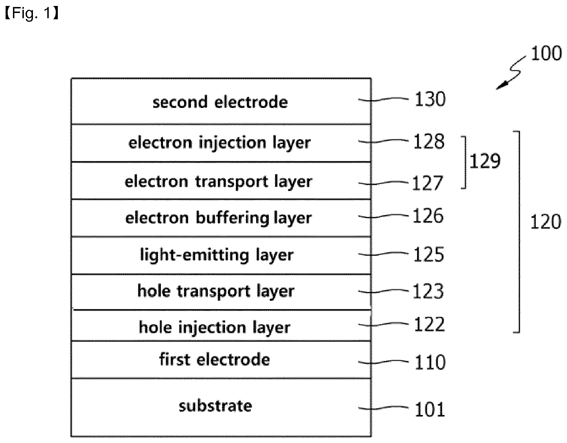

Hereinafter, referring to Figure 1, the structure of an organic electroluminescent device, and a method for preparing it will be described in detail.

Figure 1 shows an organic electroluminescent device 100 comprising a substrate 101, a first electrode 110 formed on the substrate 101, an organic layer 120 formed on the first electrode 110, and a second electrode 130 formed on the organic layer 120 and facing the first electrode 110.

The organic layer 120 comprises a hole injection layer 122, a hole transport layer 123 formed on the hole injection layer 122, a light-emitting layer 125 formed on the hole transport layer 123, an electron buffering layer 126 formed on the light-emitting layer 125, and an electron transport zone 129 formed on the electron buffering layer 126; and the electron transport zone 129 comprises an electron transport layer 127 formed on the electron buffering layer 126, and an electron injection layer 128 formed on the electron transport layer 127.

The substrate 101 may be any conventional substrate for an organic electroluminescent device, such as a glass substrate, a plastic substrate, or a metal substrate.

The first electrode 110 may be an anode, and may be prepared with a high work-function material. The first electrode 110 may be formed by any method known in the art, such as vacuum deposition, sputtering, etc.

The hole injection layer 122 may be prepared with any hole injection material known in the art. For example, the hole injection layer 122 may be formed of a compound represented by the following formula 12. 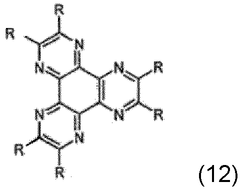 wherein R may be selected from the group consisting of a cyano(-CN), a nitro(-NO₂), a phenylsulfonyl(-SO₂(C₆H₅)), a cyano- or nitro-substituted (C2-C5) alkenyl, and a cyano- or nitro-substituted phenyl.

The compound of formula 12 has a characteristic to be crystallized. Thus, by using the compound, the hole injection layer 122 can have strength. The example of the compound of formula 12 includes HAT-CN (1,4,5,8,9,11-hexaazatriphenylene-hexacarbonitrile) of the following formula:

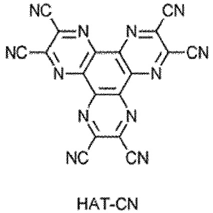

The hole injection layer 122 may be a single layer, or may be composed of two or more layers. In the latter case, one of the two or more layers may comprise the compound of formula 12. The thickness of the hole injection layer 122 may be in the range of from about 1 nm to about 1,000 nm, and preferably from about 5 nm to about 100 nm. The hole injection layer 122 may be formed on the first electrode 110 by using known various methods such as vacuum deposition, wet film-forming methods, laser induced thermal imaging, etc.

The hole transport layer 123 may be prepared with any hole transport material known in the art. The hole transport layer 123 may be a single layer, or may be composed of two or more layers. The thickness of the hole transport layer 123 may be in the range of from about 1 nm to about 100 nm, and preferably from about 5 nm to about 80 nm. The hole transport layer 123 may be formed on the hole injection layer 122 by using known various methods such as vacuum deposition, wet film-forming methods, laser induced thermal imaging, etc.

The light-emitting layer 125 may be prepared with a host compound and a dopant compound. The host compound may be a phosphorescent host compound or a fluorescent host compound. The dopant compound may be a phosphorescent dopant compound or a fluorescent dopant compound. The kinds of host compound and dopant compound to be used are not particularly limited, and may be compounds having the aforementioned LUMO energy level and selected from compounds known in the art. Preferably, the host compound may be a fluorescent host compound. The fluorescent host compound may be an anthracene-based compound represented by the following formula 13. 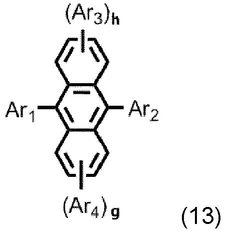 wherein Ar₁ and Ar₂, each independently, represent a substituted or unsubstituted (C6-C30)aryl, or a substituted or unsubstituted (5- to 30-membered) heteroaryl; Ar₃ and Ar₄, each independently, represent hydrogen, deuterium, a halogen, a cyano, a nitro, a hydroxy, a substituted or unsubstituted (C1-C30)alkyl, a substituted or unsubstituted (C6-C30)aryl, a substituted or unsubstituted (5- to 30-membered) heteroaryl, a substituted or unsubstituted (C3-C30)cycloalkyl, a substituted or unsubstituted (C1-C30)alkoxy, a substituted or unsubstituted (C1-C30)alkylsilyl, a substituted or unsubstituted (C6-C30)arylsilyl, a substituted or unsubstituted (C6-C30)aryl(C1-C30)alkylsilyl, or-NR₂₁R₂₂; R₂₁ and R₂₂, each independently, represent hydrogen, a substituted or unsubstituted (C6-C30)aryl, or a substituted or unsubstituted (5- to 30-membered) heteroaryl, or may be bonded to each other to form a (3-to 30-membered), mono- or polycyclic, alicyclic or aromatic ring whose carbon atom(s) may be replaced with at least one hetero atom selected from nitrogen, oxygen, and sulfur; g and h, each independently, represent an integer of 1 to 4; and where g or h is an integer of 2 or more, each of Ar₃ or Ar₄ may be the same or different.

In formula 13, Ar₁ and Ar₂, each independently, may represent preferably, a substituted or unsubstituted (C6-C30)aryl. Specifically, Ar₁ and Ar₂, each independently, may represent a substituted or unsubstituted phenyl, a substituted or unsubstituted biphenyl, a substituted or unsubstituted naphthyl, a substituted or unsubstituted anthracenyl, a substituted or unsubstituted phenanthrenyl, a substituted or unsubstituted naphthacenyl, a substituted or unsubstituted fluoranthenyl, a substituted or unsubstituted pyrenyl, or a substituted or unsubstituted chrysenyl. In formula 13, Ar₃ and Ar₄, each independently, may represent preferably, hydrogen, a substituted or unsubstituted (C6-C21)aryl, a substituted or unsubstituted (5- to 21-membered) heteroaryl or -NR₂₁R₂₂.

Specifically, the compound of formula 13 includes the following, but is not limited thereto:

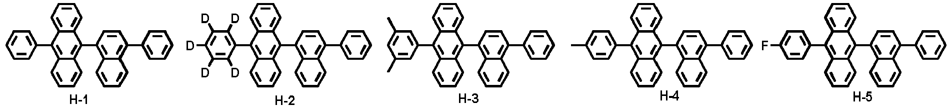

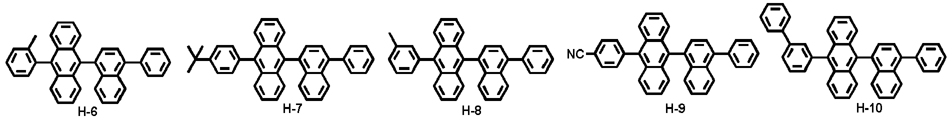

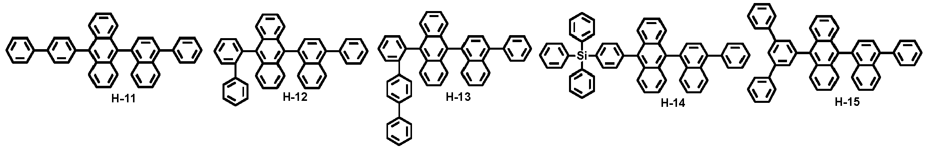

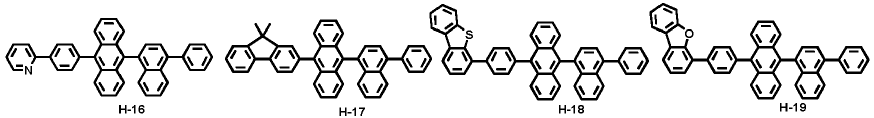

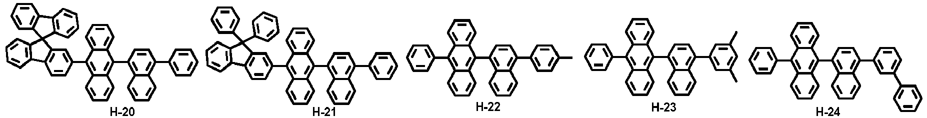

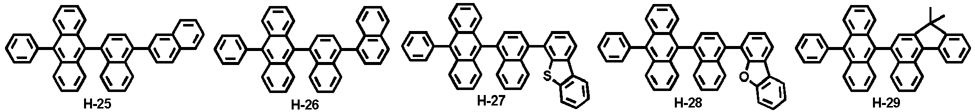

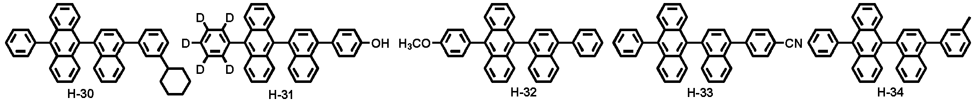

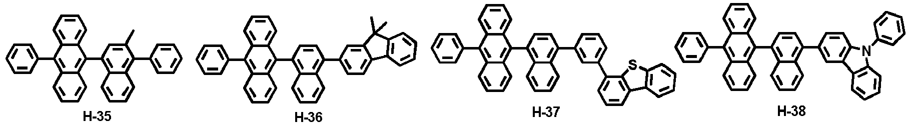

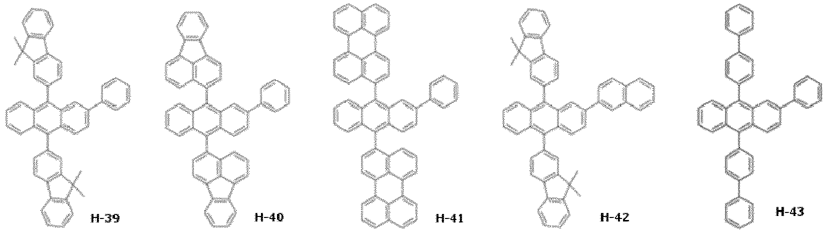

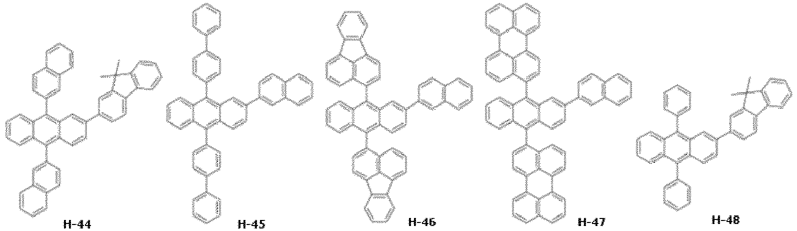

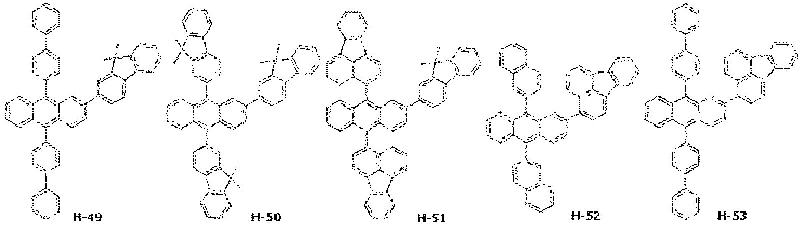

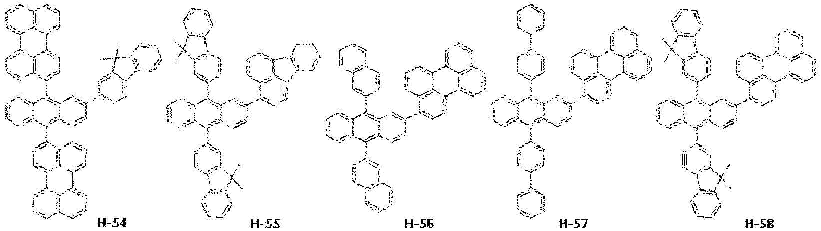

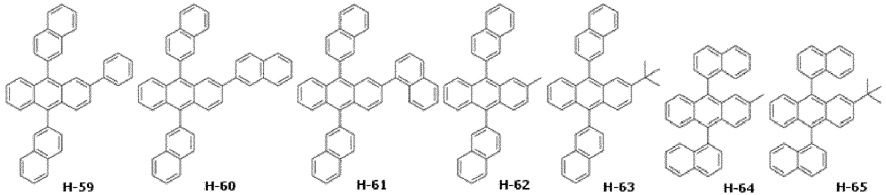

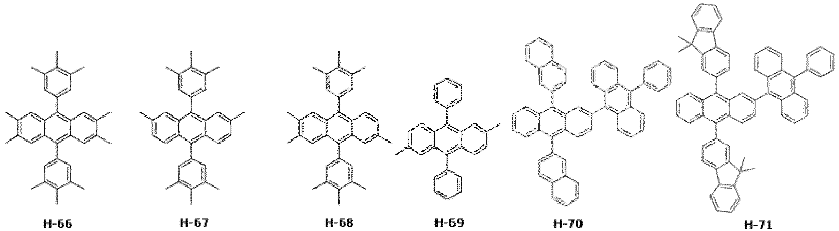

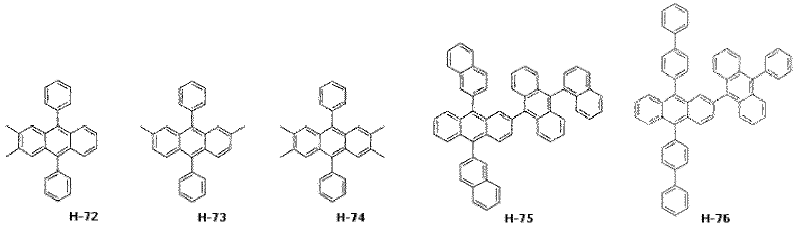

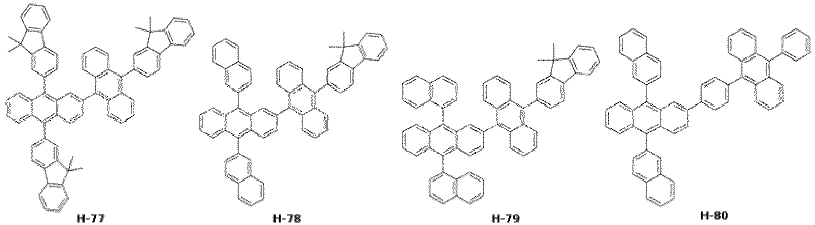

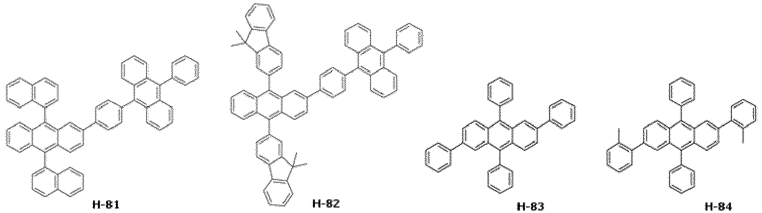

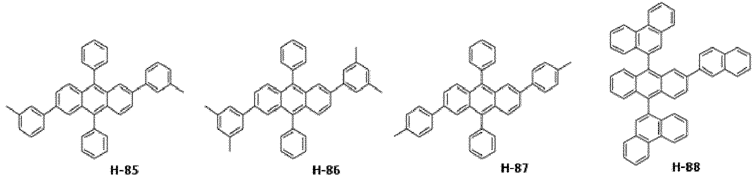

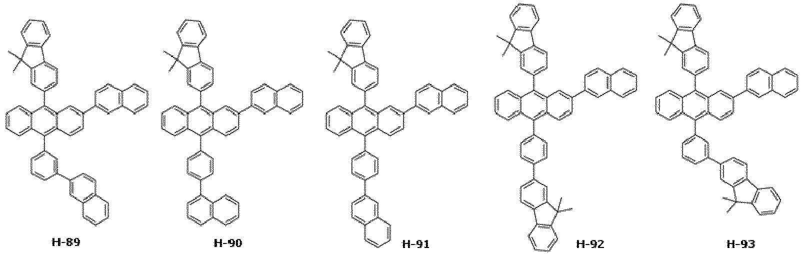

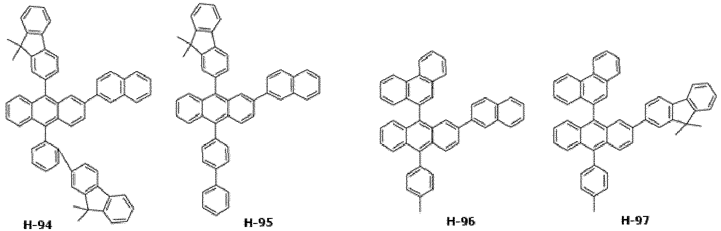

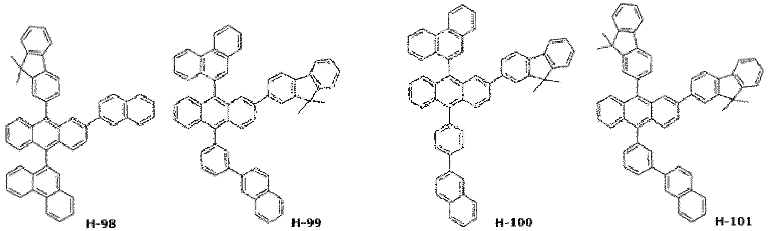

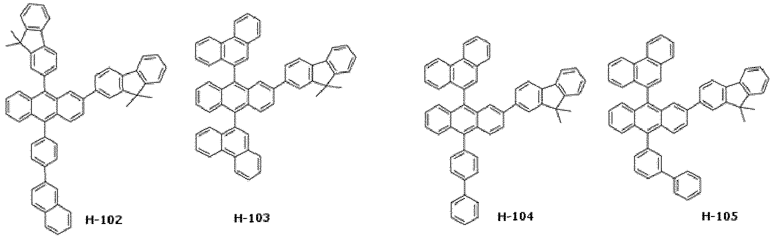

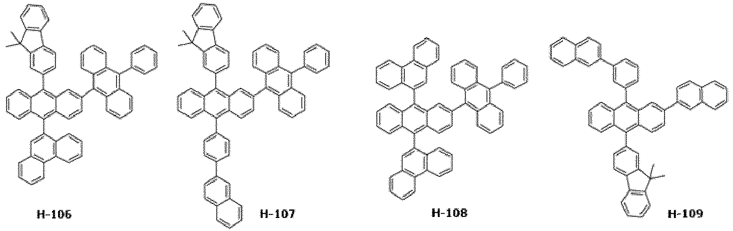

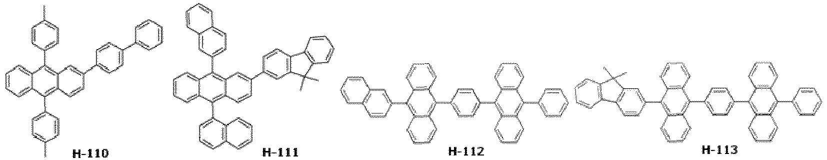

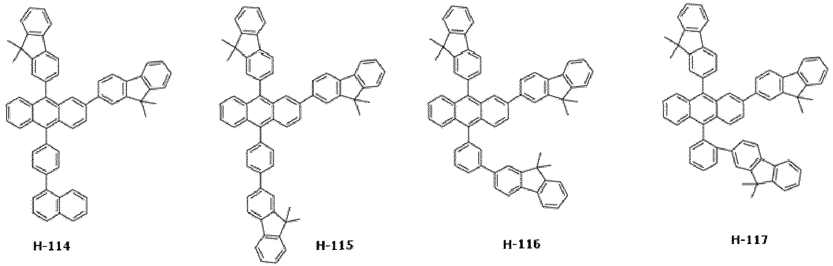

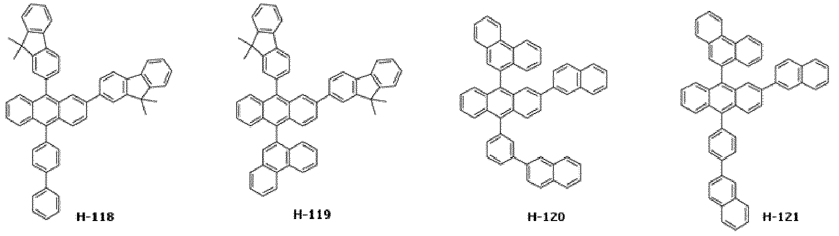

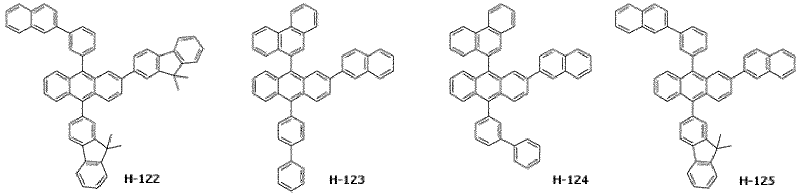

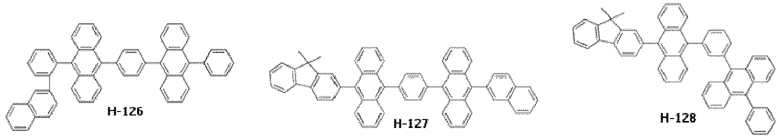

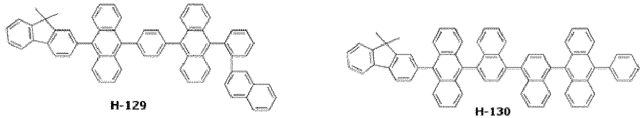

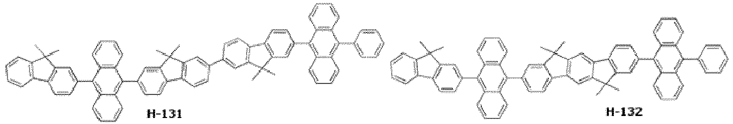

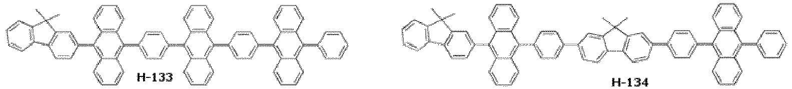

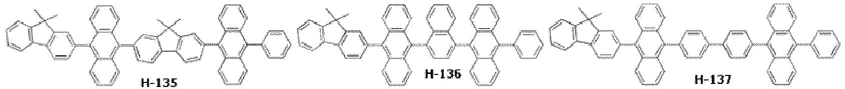

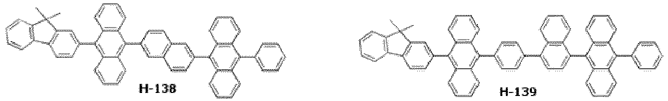

Preferably, the dopant compound may be a fluorescent dopant compound. In view of light color required to be emitted, the fluorescent dopant compound may be selected preferably, from amine-based compounds, aromatic compounds, chelate complexes such as tris(8-quinolinolate)aluminum complexes, coumarine derivatives, tetraphenylbutadiene derivatives, bis-styrylarylene derivatives, oxadiazole derivatives, etc.; more preferably, styrylamine compounds, styryldiamine compounds, arylamine compounds, and aryldiamine compounds; and even more preferably, condensed polycyclic amide derivatives. The fluorescent dopant can be used solely or as a combination of two or more compounds.

The fluorescent dopant compound may be a condensed polycyclic amine derivative represented by the following formula 14. 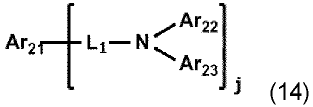 wherein Ar₂₁ represents a substituted or unsubstituted (C6-C50)aryl or styryl; L₁ represents a single bond, a substituted or unsubstituted (C6-C30)arylene, or a substituted or unsubstituted (3- to 30-membered)heteroarylene; Ar₂₂ and Ar₂₃, each independently, represent hydrogen, deuterium, a halogen, a substituted or unsubstituted (C1-C30)alkyl, a substituted or unsubstituted (C6-C30)aryl, or a substituted or unsubstituted (3- to 30-membered)heteroaryl, or may be linked to an adjacent substituent(s) to form a (3- to 30-membered), mono- or polycyclic, alicyclic or aromatic ring whose carbon atom(s) may be replaced with at least one hetero atom selected from nitrogen, oxygen, and sulfur; j represents 1 or 2; and where j is 2, each of 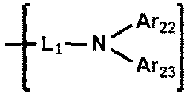 may be the same or different.

A preferable aryl for Ar₂₁ includes a substituted or unsubstituted phenyl, a substituted or unsubstituted fluorenyl, a substituted or unsubstituted anthryl, a substituted or unsubstituted pyrenyl, a substituted or unsubstituted chrysenyl, a substituted or unsubstituted benzofluorenyl, and spiro[fluoren-benzofluorene], etc.

Specifically, the compound of formula 14 includes the following, but is not limited thereto:

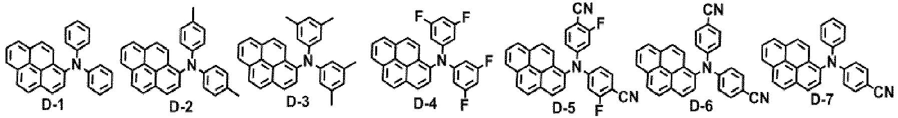

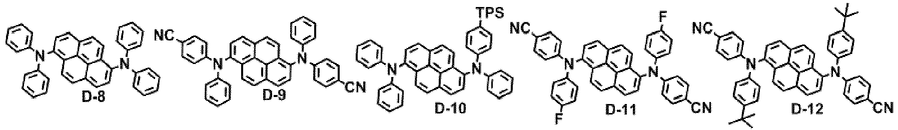

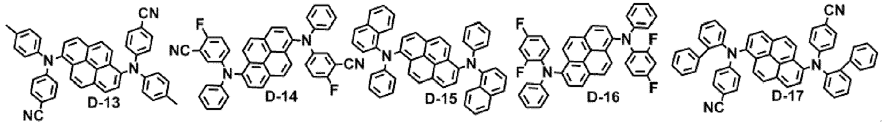

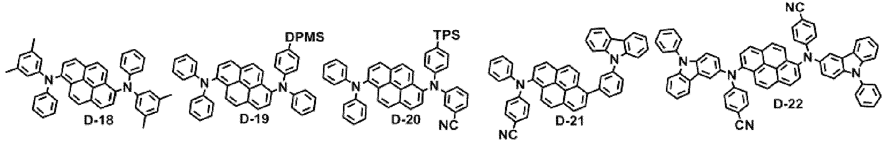

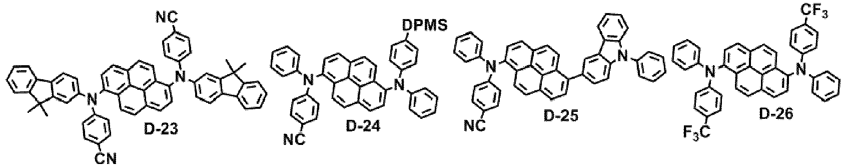

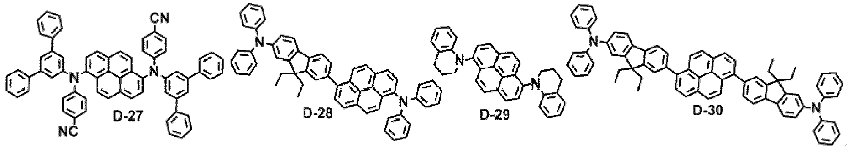

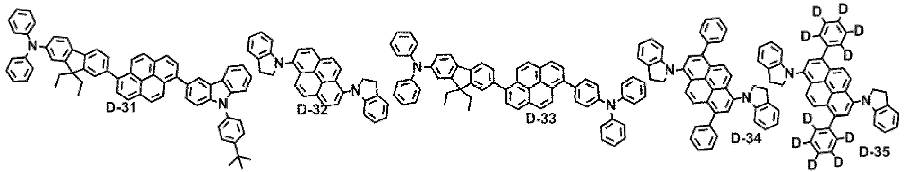

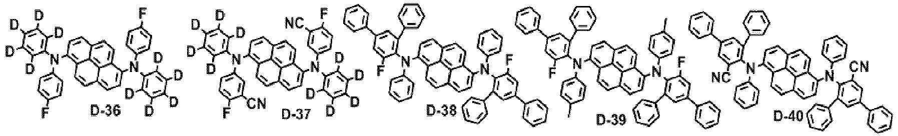

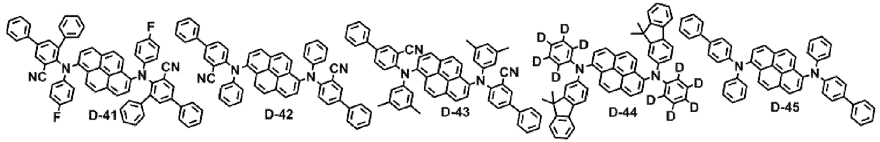

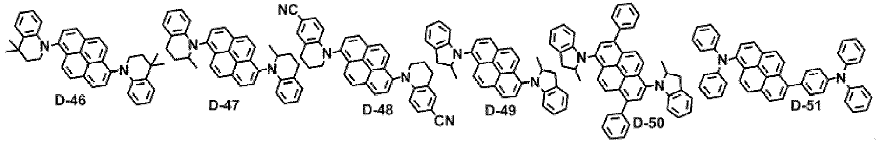

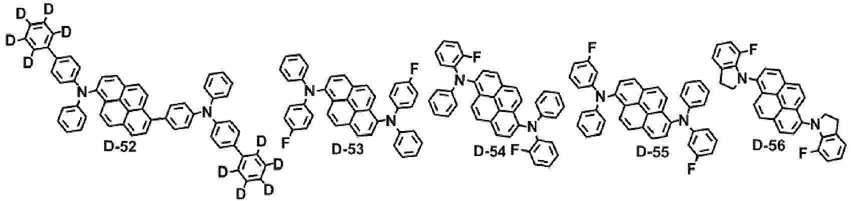

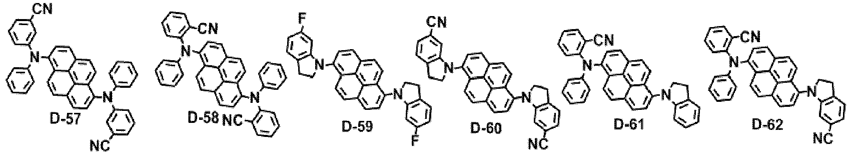

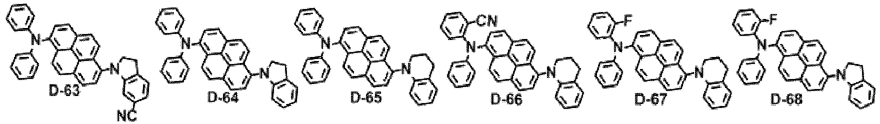

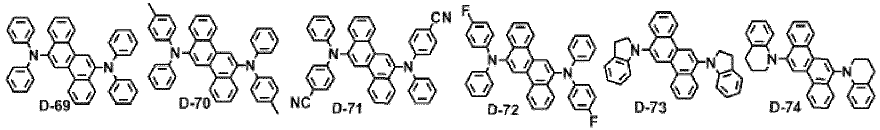

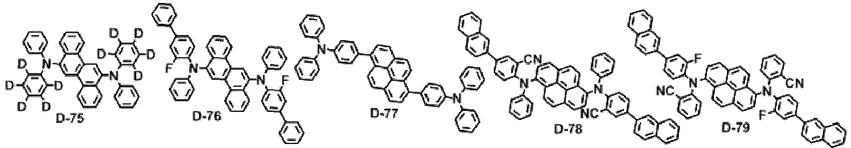

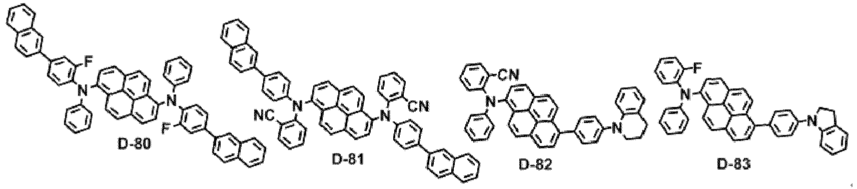

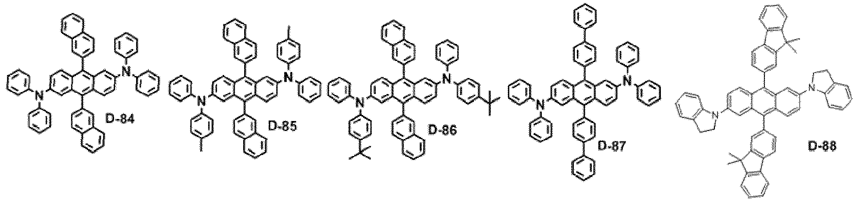

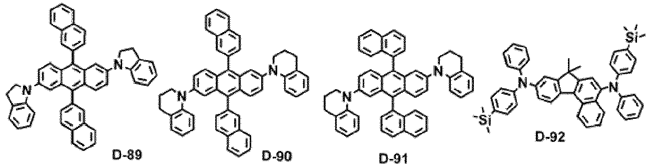

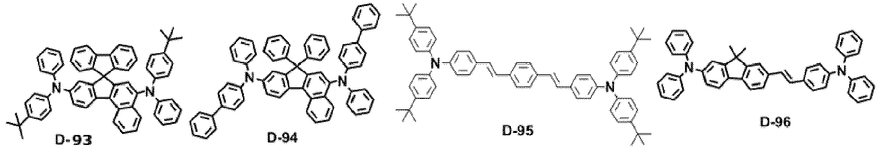

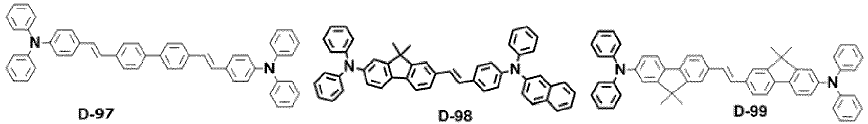

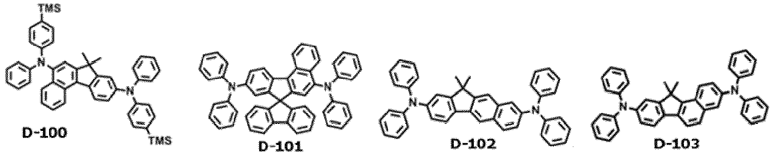

When the light-emitting layer 125 comprises a host and a dopant, the dopant can be doped in an amount of less than about 25 wt%, and preferably less than 17 wt%, based on the total amount of the dopant and host of the light-emitting layer. The thickness of the light-emitting layer 125 can be in the range of from about 5 nm to about 100 nm, and preferably from about 10 nm to about 60 nm. Light emission occurs at the light-emitting layer 125. The light-emitting layer 125 may be a single layer, or may be composed of two or more layers. When the light emitting layer 125 is composed of two or more layers, each of the layers may be prepared to emit color different from one another. For example, the device may emit white light by preparing three light-emitting layers 125 which emit blue, red, and green, respectively. The light-emitting layer 125 may be formed on the hole transport layer 123 by using known various methods such as vacuum deposition, wet film-forming methods, laser induced thermal imaging, etc.

The electron buffering layer 126 employs the compound of formula 1 of the present disclosure. The details of the compound of formula 1 are as previously described. The thickness of the electron buffering layer 126 is 1 nm or more, but is not particularly limited thereto. Specifically, the thickness of the electron buffering layer 126 may be in the range of from 2 nm to 200 nm. The electron buffering layer 126 may be formed on the light-emitting layer 125 by using known various methods such as vacuum deposition, wet film-forming methods, laser induced thermal imaging, etc.

The electron transport layer 127 may be prepared with any electron transport material known in the art, which includes oxazole-based compounds, isoxazole-based compounds, triazole-based compounds, isothiazole-based compounds, oxadiazole-based compounds, thiadiazole-based compounds, perylene-based compounds, anthracene-based compounds, aluminum complexes, and gallium complexes, but is not limited thereto. The anthracene-based compound may be a compound represented by the following formula 15. 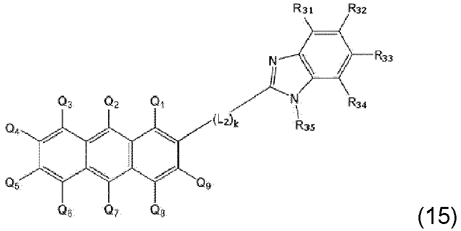 wherein R₃₁ to R₃₅, each independently, represent hydrogen, a halogen, a hydroxy, a cyano, a substituted or unsubstituted (C1-C30)alkyl, a substituted or unsubstituted (C1-C30)alkoxy, a substituted or unsubstituted (C1-C30)alkylcarbonyl, a (C1-C30)alkylcarbonyl, a substituted or unsubstituted (C1-C30)alkoxycarbonyl, a substituted or unsubstituted (C6-C30)arylcarbonyl, a substituted or unsubstituted (C2-C30)alkenyl, a substituted or unsubstituted (C2-C30)alkynyl, a substituted or unsubstituted (C6-C30)aryl, or a substituted or unsubstituted (3- to 30-membered)heteroaryl, or may be linked to an adjacent substituent(s) to form a (3- to 30-membered), mono- or polycyclic, alicyclic or aromatic ring whose carbon atom(s) may be replaced with at least one hetero atom selected from nitrogen, oxygen, and sulfur; L₂ represents a single bond, a substituted or unsubstituted (C1-C30)alkylene, a substituted or unsubstituted (C6-C30)arylene, or a substituted or unsubstituted (3- to 30-membered)heteroarylene; Q₁ to Q₉, each independently, represent hydrogen, a substituted or unsubstituted (C6-C30)aryl or a substituted or unsubstituted (3- to 30-membered)heteroaryl; and k represents an integer of 1 to 10.

In formula 15, preferably, one of R₃₁ to R₃₅ may be a substituted or unsubstituted (C6-C18)aryl, or a substituted or unsubstituted (5- to 18-membered)heteroaryl, the rest of R₃₁ to R₃₅ may be hydrogen; Q₂ and Q₇, each independently, may represent a substituted or unsubstituted (C6-C30)aryl or a substituted or unsubstituted (3- to 30-membered)heteroaryl; Q₁, Q₃ to Q₆, Q₈, and Q₉ may represent hydrogen; and k may represent 1.

The specific compound of formula 15 includes the following compound:

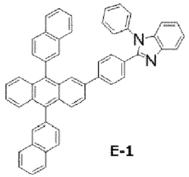

Preferably, the electron transport layer 127 may be a mixed layer comprising an electron transport compound and a reductive dopant. In this case, the electron transport compound is reduced to an anion, and thus it becomes easier to inject and transport electrons to an electroluminescent medium. The electron transport compound for the mixed layer is not particularly limited, and may be any of the aforementioned known electron transport materials. The reductve dopant may be selected from alkali metals, alkali metal compounds, alkaline earth metals, rare-earth metals, and halides, oxides, and complexes thereof. Specifically, the reductive dopant includes lithium quinolate, sodium quinolate, cesium quinolate, potassium quinolate, LiF, NaCl, CsF, Li₂O, BaO, and BaF₂, but are not limited thereto. The thickness of the electron transport layer 127 may be in the range of from about 5 to about 100 nm, and preferably from about 10 to about 60 nm. The electron transport layer 127 may be formed on the electron buffering layer 126 by using known various methods such as vacuum deposition, wet film-forming methods, laser induced thermal imaging, etc.

The electron injection layer 128 may be prepared with any electron injection material known in the art, which includes lithium quinolate, sodium quinolate, cesium quinolate, potassium quinolate, LiF, NaCl, CsF, Li₂O, BaO, and BaF₂, but is not limited thereto. The thickness of the electron injection layer may be in the range of from about 0.1 to about 10 nm, and preferably from about 0.3 to about 9 nm. The electron injection layer 128 may be formed on the electron transport layer by using known various methods such as vacuum deposition, wet film-forming methods, laser induced thermal imaging, etc.

The second electrode 130 may be a cathode, and may be prepared with a low work-function material. The material for the second electrode 130 includes aluminum (Al), calcium (Ca), magnesium (Mg), silver (Ag), cesium (Cs), lithium (Li), and a combination thereof. The second electrode 130 may be formed by any method known in the art, such as vacuum deposition, sputtering, etc.

The aforementioned description regarding the organic electroluminescent device shown in Figure 1 is intended to explain one embodiment of the invention, and is not meant in any way to restrict the scope of the invention. The organic electroluminescent device can be constructed in another way. For example, any one optional component such as a hole injection layer may not be comprised in the organic electroluminescent device of Figure 1, except for a light-emitting layer and an electron buffering layer. In addition, an optional component may be further comprised therein, which includes an impurity layer such as n-doping layer and p-doping layer. The organic electroluminescent device may be a both side emission type in which a light-emitting layer is placed on each of both sides of the impurity layer. The two light-emitting layers on the impurity layer may emit different colors. The organic electroluminescent device may be a bottom emission type in which a first electrode is a transparent electrode and a second electrode is a reflective electrode. The organic electroluminescent device may be a top emission type in which a first electrode is a reflective electrode and a second electrode is a transparent electrode. The organic electroluminescent device may have an inverted type structure in which a cathode, an electron transport layer, a light-emitting layer, a hole transport layer, a hole injection layer, and an anode are sequentially stacked on a substrate.

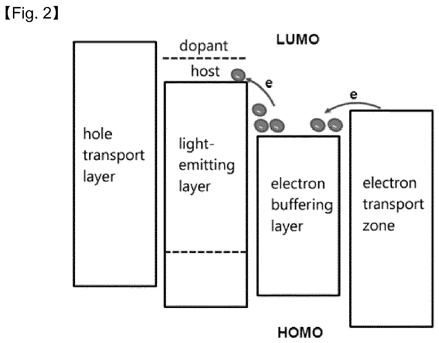

Figure 2 illustrates an energy band diagram among a hole transport layer, a light-emitting layer, an electron buffering layer, and an electron transport zone of an organic electroluminescent device according to one embodiment of the present disclosure.

In Figure 2, a hole transport layer 123, a light-emitting layer 125, an electron buffering layer 126, and an electron transport zone 129 are sequentially stacked. Electrons (e) injected from a cathode are transported to a light-emitting layer through an electron transport zone 129 and an electron buffering layer 126. LUMO energy level of an electron buffering layer 126 may be higher than those of a host compound and a dopant compound of a light-emitting layer 125, and an electron transport layer 127. Specifically, LUMO energy levels may have the following relationship: the electron buffering layer > the electron transport zone > the host compound. According to prior arts, light-emitting sites in a light-emitting layer are shifted to a hole transport layer due to hole trap, thereby light being emitted at an interface. However, according to the present disclosure, electrons(e) are trapped due to the aforementioned LUMO energy level of an electron buffering layer 126, so that light-emitting sites in a light-emitting layer can be shifted to an electron transport zone 129, and thus lifespan and efficiencies of an organic electroluminescent device can be improved. Meanwhile, HOMO energy level of an electron buffering layer 126 is higher than those of a host compound and a dopant compound of a light-emitting layer 125, but may be lower or higher than HOMO energy level of an electron transport zone 129.

Hereinafter, a preparation method of an organic electroluminescent device according to one embodiment of the present disclosure, and luminescent properties of the device will be explained in detail with reference to the following examples.

### [Comparative Example 1] Preparation of a Blue-Emitting OLED in which an electron buffering layer is not comprised

OLED was produced as follows. A transparent electrode indium tin oxide (ITO) thin film (15 Ω/sq) on a glass substrate for an OLED (Samsung Corning) was subjected to an ultrasonic washing with trichloroethylene, acetone, ethanol, and distilled water, sequentially, and then was stored in isopropanol. The ITO substrate was then mounted on a substrate holder of a vacuum vapor depositing apparatus. N⁴,N^{4'}-diphenyl-N⁴,N^{4'}-bis(9-phenyl-9H-carbazol-3-yl)-[1,1'-biphenyl]-4,4'-diamine was introduced into a cell of the vacuum vapor depositing apparatus, and then the pressure in the chamber of said apparatus was controlled to 10⁻⁶ torr. Thereafter, an electric current was applied to the cell to evaporate the above introduced material, thereby forming a first hole injection layer having a thickness of 60 nm on the ITO substrate. 1,4,5,8,9,11-hexaazetriphenylene-hexacarbonitrile (HAT-CN) was then introduced into another cell of the vacuum vapor depositing apparatus, and was evaporated by applying an electric current to the cell, thereby forming a second hole injection layer having a thickness of 5 nm on the first hole injection layer. N-([1,1'-biphenyl]-4-yl)-9,9-dimethyl-N-(4-(9-phenyl-9H-carbazol-3-yl)phenyl)-9H-fluoren-2-amine **(HT-1)** was then introduced into another cell of the vacuum vapor depositing apparatus, and was evaporated by applying an electric current to the cell, thereby forming a first hole transport layer having a thickness of 20 nm on the second hole injection layer. Thereafter, HT-2 was introduced into another cell of the vacuum vapor depositing apparatus, and was evaporated by applying an electric current to the cell, thereby forming a second hole transport layer having a thickness of 5 nm on the first hole transport layer. Thereafter, compound **H-1** was introduced into one cell of the vacuum vapor depositing apparatus, as a host material, and compound **D-38** was introduced into another cell as a dopant. The two materials were evaporated at different rates, so that the dopant was deposited in a doping amount of 2 wt% based on the total amount of the host and dopant to form a light-emitting layer having a thickness of 20 nm on the hole transport layer. 2-(4-(9,10-di(naphthalen-2-yl)anthracen-2-yl)phenyl)-1-phenyl-1H-benzo[d]imidazole was then introduced into one cell, and lithium quinolate was introduced into another cell. The two materials were evaporated at the same rate, so that they were respectively deposited in a doping amount of 50 wt% to form an electron transport layer having a thickness of 35 nm on the light-emitting layer. After depositing lithium quinolate as an electron injection layer having a thickness of 2 nm on the electron transport layer, an Al cathode having a thickness of 80 nm was then deposited by another vacuum vapor deposition apparatus on the electron injection layer. Thus, an OLED was produced. All the material used for producing the OLED device were those purified by vacuum sublimation at 10⁻⁶ torr.

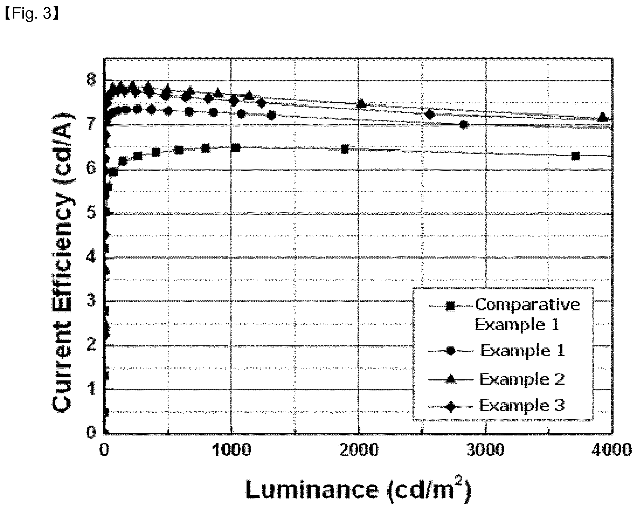

Figure 3 shows a graph illustrating a current efficiency versus a luminance of the prepared organic electroluminescent device. In addition, luminous efficiency, CIE color coordinate, a driving voltage at 1,000 nit of luminance, and a lifespan for 10 hours at 2,000 nit and a constant current are shown in Table 1 below.

### [Examples 1 to 3] Preparation of a blue-emitting OLED comprising an electron buffering layer of an electron buffering material according to the present disclosure

OLEDs were produced and evaluated in the same manner as in Comparative Example 1, except that a thickness of an electron transport layer was 30 nm, and an electron buffering layer having a thickness of 5 nm was interposed between a light-emitting layer and an electron transport layer. Electron buffering materials used in Examples 1 to 3 are shown in Tables 1 and 4 below. Figure 3 shows a graph illustrating a current efficiency versus a luminance of the prepared organic electroluminescent device. In addition, evaluation results of the devices prepared in Examples 1 to 3 are shown in Table 1 below.

### [Comparative Examples 2 and 3] Prepartion of a blue-emitting OLED comprising an electron buffering layer of a conventional electron buffering material

OLEDs were produced and evaluated in the same manner as in Example 1, except that BF-1 and BF-65 were used for an electron buffering material and HT-3 was used for a second hole transport layer. Evaluation results of the devices prepared in Comparative Examples 2 and 3 were shown in Table 1 below.

**[Table 1]**

| | Second Hole Transport Layer | Electron Buffering Material | Voltage (V) | Current Efficiency (cd/A) | Color coordinate (x) | Color coordinate (y) | Lifespan (hr) | LUMO (eV) | HOMO (eV) |
|---|---|---|---|---|---|---|---|---|---|
| Comparative Ex. 1 | HT-2 | - | 4.2 | 6.3 | 0.140 | 0.097 | 95.6 | | |
| Comparative Ex. 2 | HT-3 | **BF-1** | 4.5 | 6.6 | 0.140 | 0.095 | 95.4 | 1.95 | 5.48 |
| Comparative Ex. 3 | HT-3 | **BF-65** | 4.4 | 6.8 | 0.140 | 0.098 | 95.8 | 1.92 | 5.55 |
| Example 1 | HT-2 | **B-34** | 4.3 | 7.2 | 0.140 | 0.095 | 95.6 | 1.96 | 5.33 |
| Example 2 | HT-2 | **B-57** | 4.1 | 7.6 | 0.139 | 0.097 | 96.4 | 1.94 | 5.34 |
| Example 3 | HT-2 | **B-90** | 4.1 | 7.6 | 0.139 | 0.098 | 95.7 | 1.97 | 5.32 |

From Table 1 above, it is recognized that due to speediness of electron current by the electron buffering material of the present disclosure, the devices of Examples 1 to 3 show higher efficiencies and longer lifespan than those of Comparative Examples 1 to 3 in which an electron buffering layer is not comprised or an electron buffering material of the present disclosure is not used to prepare an electron buffering layer. LUMO energy levels of the electron buffering layer of Examples 1 to 3 were about 1.9 eV; LUMO energy level of the host compound was about 1.6 eV; and LUMO energy level of the electron transport layer was about 1.8 eV. From Figure 3, it is recognized that the organic electroluminescent devices of Examples 1 to 3 show higher current efficiencies over the whole range of luminance than the organic electroluminescent device of Comparative Example 1.

### [Comparative Example 4] Preparation of a blue-emitting OLED in which an electron buffering layer is not comprised

OLEDs were produced in the same manner as in Comparative Example 1, except that a compound for a second hole transport layer was changed to HT-3 shown in Table 4 below. Luminous efficiency, CIE color coordinate, a driving voltage at 1,000 nit of luminance, and time taken for luminance to be reduced from 100% to 90% at 2,000 nit and a constant current are shown in Table 2 below.

### [Examples 4 to 8] Preparation of a blue-emitting OLED comprising an electron buffering layer of an electron buffering material according to the present disclosure

OLEDs were produced and evaluated in the same manner as in Comparative Example 4, except that a thickness of an electron transport layer was 30 nm, and an electron buffering layer having a thickness of 5 nm and comprising an electron buffering material was interposed between a light-emitting layer and an electron transport layer. Electron buffering materials used in Examples 4 to 8 are shown in Tables 2 and 4 below. Evaluation results of the devices prepared in Examples 4 to 8 are shown in Table 2 below.

**[Table 2]**

| | Second Hole Transport Layer | Electron Buffering Material | Voltage (V) | Current Efficiency (cd/A) | Color coordinate (x) | Color coordinate (y) | Lifespan (hr) | LUMO (eV) | HOMO (eV) |
|---|---|---|---|---|---|---|---|---|---|
| Comparative Ex. 4 | HT-3 | - | 4.1 | 6.5 | 0.139 | 0.094 | 27.9 | | |
| Example 4 | HT-3 | **B-143** | 4.3 | 6.6 | 0.139 | 0.095 | 37.0 | 1.95 | 5.21 |
| Example 5 | HT-3 | **B-145** | 4.4 | 6.9 | 0.139 | 0.096 | 47.3 | 1.90 | 5.43 |
| Example 6 | HT-3 | **B-144** | 4.3 | 6.9 | 0.139 | 0.098 | 39.7 | 1.97 | 5.15 |
| Example 7 | HT-3 | **B-147** | 4.3 | 6.8 | 0.139 | 0.095 | 35.1 | 1.89 | 5.40 |
| Example 8 | HT-3 | **B-149** | 4.2 | 7.2 | 0.139 | 0.097 | 34.8 | 1.88 | 5.43 |

It is recognized that due to speediness of electron current by the electron buffering material of the present disclosure, the devices of Examples 4 to 8 have similar current characteristics to one of Comparative Example 4, but show higher efficiencies and longer lifespan than those of Comparative Example 4.

### [Comparative Example 5] Preparation of a green-emitting OLED in which an electron buffering layer is not comprised

OLEDs were produced and evaluated in the same manner as in Comparative Example 1, except that a thickness of a first hole transport layer was 10 nm; HT-4 was used to form a second hole transport layer having a thickness of 30 nm on a first hole transport layer; and H-44 and D-88 were used as a host and a dopant, respectively. The prepared OLED was evaluated as to luminous efficiency, CIE color coordinate, a driving voltage at 1,000 nit of luminance, and time taken for luminance to be reduced from 100% to 90% at 2,000 nit and a constant current, and the results are shown in Table 3 below.

### [Examples 9 to 10] Preparation of a green-emitting OLED comprising an electron buffering layer of an electron buffering material according to the present disclosure

OLEDs were produced and evaluated in the same manner as in Comparative Example 5, except that a thickness of an electron transport layer was 30 nm, and an electron buffering layer having a thickness of 5 nm and comprising an electron buffering material was interposed between a light-emitting layer and an electron transport layer. Electron buffering materials used in Examples 9 and 10 are shown in Table 3 below, along with evaluation results of the prepared devices.

**[Table 3]**

| | Second Hole Transport Layer | Electron Buffering Material | Voltage (V) | Current Efficiency (cd/A) | Color coordinate (x) | Color coordinate (y) | Lifespan (hr) | LUMO (eV) | HOMO (eV) |
|---|---|---|---|---|---|---|---|---|---|
| Comparative Ex. 5 | HT-4 | - | 3.4 | 28.9 | 0.291 | 0.677 | 25.0 | | |
| Example 9 | HT-4 | **B-57** | 3.4 | 30.7 | 0.291 | 0.677 | 25.0 | 1.94 | 5.34 |
| Example 10 | HT-4 | **B-145** | 3.4 | 30.3 | 0.291 | 0.676 | 27.0 | 1.90 | 5.43 |

It is recognized that due to speediness of electron current by the electron buffering material of the present disclosure, the devices of Examples 9 to 10 have similar current characteristics to one of Comparative Example 5 in which an electron buffering layer is not comprised, but show higher efficiencies and longer lifespan than those of Comparative Example 5.

**[Table 4] Compounds for a hole transport layer of the Comparative Examples and the Examples**

| | | | | |
|---|---|---|---|---|
| Hole Transport Layer | 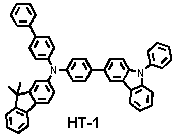 | 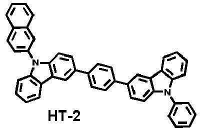 | 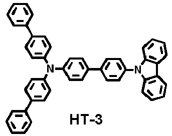 | 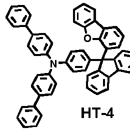 |

### [Examples 11 to 16] Preparation of a blue-emitting OLED comprising an electron buffering layer of an electron buffering material according to the present disclosure

OLEDs were produced in the same manner as in Example 1, except that compounds shown in Table 5 below were used as an electron buffering material. The electron buffering layer was formed by a co-evaporation of the two compounds shown in Table 5. The prepared OLEDs were evaluated as to luminous efficiency, CIE color coordinate, a driving voltage at 1,000 nit of luminance, and lifespan for 10 hours at 2,000 nit and a constant current, and the results are shown in Table 5 below.

**[Table 5]**

| | Hole Injection Layer | Electron buffering material (mixing ratio = 5:5) | Voltage (V) | Current Efficiency (cd/A) | Color coordinate (x) | Color coordinate (y) | Lifespan (10hr) (%) |
|---|---|---|---|---|---|---|---|
| Example 11 | HT-2 | **BF-3:B-57** | 4.4 | 7.1 | 0.139 | 0.095 | 96.2 |
| Example 12 | HT-2 | **BF-4:B-57** | 4.2 | 7.3 | 0.139 | 0.094 | 95.9 |
| Example 13 | HT-2 | **BF-5:B-57** | 4.3 | 7.2 | 0.139 | 0.095 | 96.3 |
| Example 14 | HT-2 | **BF-3:B-145** | 4.4 | 7.2 | 0.139 | 0.097 | 96.5 |
| Example 15 | HT-2 | **BF-4:B-145** | 4.3 | 7.3 | 0.139 | 0.098 | 96.5 |
| Example 16 | HT-2 | **BF-5:B-145** | 4.4 | 7.1 | 0.139 | 0.095 | 96.5 |

From Table 5 above, it is recognized that due to speediness of electron current by the electron buffering material of the present disclosure, the devices of Examples 11 to 16 show higher efficiency and longer lifespan than those of Comparative Examples 1 to 3 shown in Table 1 in which an electron buffering layer was not comprised or an electron buffering material of the present disclosure is not used to prepare an electro buffer layer.

### [Examples 17 to 18] Preparation of a blue-emitting OLED comprising an electron buffering layer of an electron buffering material according to the present disclosure

OLEDs were produced in the same manner as in Example 1, except that compounds shown in Table 6 below were used as an electron buffering material. The electron buffering layer of Example 17 was formed by a co-evaporation, while the electron buffering layer of Example 18 was formed by a mixture-evaporation. The prepared OLEDs were evaluated as to luminous efficiency, CIE color coordinate, a driving voltage at 1,000 nit of luminance, and lifespan for 10 hours at 2,000 nit and a constant current, and the results are shown in Table 6 below.

**[Table 6]**

| | Hole Injection Layer | Electron buffering material (mixing ratio = 5:5) | Voltage (V) | Current Efficiency (cd/A) | Color coordinate (x) | Color coordinate (y) | Lifespan (10hr) (%) |
|---|---|---|---|---|---|---|---|
| Example 17 | HT-2 | **B-145:BF-6** | 4.5 | 6.7 | 0.139 | 0.095 | 97.4 |
| Example 18 | HT-2 | **B-145:BF-6** | 4.5 | 6.7 | 0.139 | 0.095 | 97.4 |

From Table 6 above, it is recognized that due to speediness of electron current by the electron buffering material of the present disclosure, the devices of Examples 17 and 18 show higher efficiency than those of Comparative Examples 1 to 3 shown in Table 1 in which an electron buffering layer was not comprised or an electron buffering material of the present disclosure is not used to prepare an electro buffer layer. In particular, lifespan was significantly enhanced by 97.4%. Furthermore, B-145 and BF-6 have the same deposition temperature, and thus are suitable for a mixture-evaporation. Example 18 shows that an electron buffering layer can be formed by a mixture-evaporation with the material. A respective deposition temperature of the compounds in a vacuum vapor depositing apparatus are shown in Table 7 below. The mixture-evaporation method for an electron buffering layer has an advantage in that device characteristics can be maintained until the whole material is consumed in a crucible.

**[Table 7]**

| Electron buffering compound | Deposition Temperature (°C) (0.5 Å/s at 10⁻⁷ torr) |
|---|---|
| B-145 | 245 |
| BF-6 | 244 |

**Description of Reference Numerals**

| | |
|---|---|
| 100: organic electroluminescent device | 101: substrate |
| 110: first electrode | 120: organic layer |
| 122: hole injection layer | 123: hole transport layer |
| 125: light-emitting layer | 126: electron buffering layer |
| 127: electron transport layer | 128: electron injection layer |
| 129: electron transport zone | 130: second electrode |

## Claims

1. An electron buffering material comprising a compound represented by the following formula 1: 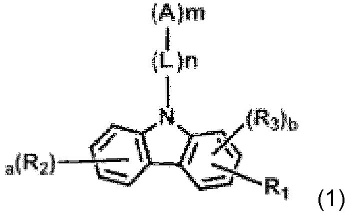 wherein
A represents a substituted or unsubstituted (5- to 30-membered)heteroaryl selected from the group consisting of a substituted or unsubstituted triazinyl, tetrazinyl, triazolyl, tetrazolyl, furazanyl, pyridyl, pyrazinyl, pyrimidinyl, pyridazinyl, benzoimidazolyl, benzothiazolyl, benzoisothiazolyl, benzoisoxazolyl, benzoxazolyl, quinolyl, isoquinolyl, cinnolinyl, quinazolinyl, quinoxalinyl, naphthyridinyl or phenanthridinyl group;
L represents a single bond, a substituted or unsubstituted (C6-C30)arylene, or a substituted or unsubstituted (5- to 30-membered)heteroarylene;
R₁ represents the formula 2a: 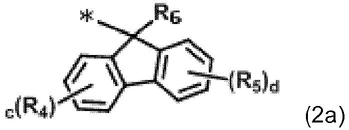
R₂ represents hydrogen, deuterium, a halogen, a cyano, a substituted or unsubstituted (C1-C30)alkyl, a substituted or unsubstituted (C6-C30)aryl, a substituted or unsubstituted (5-to 30-membered)heteroaryl, a substituted or unsubstituted (C3-C30)cycloalkyl, a substituted or unsubstituted (C1-C30)alkoxy, a substituted or unsubstituted tri(C1-C30)alkylsilyl, a substituted or unsubstituted tri(C6-C30)arylsilyl, a substituted or unsubstituted di(C1-C30)alkyl(C6-C30)arylsilyl, a substituted or unsubstituted (C1-C30)alkyldi(C6-C30)arylsilyl, a substituted or unsubstituted mono- or di-(C1-C30)alkylamino, a substituted or unsubstituted mono- or di-(C6-C30)arylamino, a substituted or unsubstituted (C1-C30)alkyl(C6-C30)arylamino, or the following formula 3; or may be fused with the carbazole backbone to form a substituted or unsubstituted benzocarbazole; 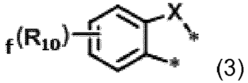
X represents O, S, CR₁₁R₁₂, NR₁₃ or SiR₁₃R₁₄;
R₃ represents hydrogen, deuterium, a halogen, a cyano, a substituted or unsubstituted (C1-C30)alkyl, a substituted or unsubstituted (C6-C30)aryl, a substituted or unsubstituted (5-to 30-membered)heteroaryl, a substituted or unsubstituted (C3-C30)cycloalkyl, a substituted or unsubstituted (C1-C30)alkoxy, a substituted or unsubstituted tri(C1-C30)alkylsilyl, a substituted or unsubstituted tri(C6-C30)arylsilyl, a substituted or unsubstituted di(C1-C30)alkyl(C6-C30)arylsilyl, a substituted or unsubstituted (C1-C30)alkyldi(C6-C30)arylsilyl, a substituted or unsubstituted mono- or di-(C1-C30)alkylamino, a substituted or unsubstituted mono- or di-(C6-C30)arylamino, or a substituted or unsubstituted (C1-C30)alkyl(C6-C30)arylamino;
R₄, R₅ and R₁₀, each independently, represent hydrogen, deuterium, a halogen, a cyano, a substituted or unsubstituted (C1-C30)alkyl, a substituted or unsubstituted (C6-C30)aryl, a substituted or unsubstituted (5- to 30-membered)heteroaryl, a substituted or unsubstituted (C3-C30)cycloalkyl, a substituted or unsubstituted (C1-C30)alkoxy, a substituted or unsubstituted tri(C1-C30)alkylsilyl, a substituted or unsubstituted tri(C6-C30)arylsilyl, a substituted or unsubstituted di(C1-C30)alkyl(C6-C30)arylsilyl, a substituted or unsubstituted (C1-C30)alkyldi(C6-C30)arylsilyl, a substituted or unsubstituted mono- or di-(C1-C30)alkylamino, a substituted or unsubstituted mono- or di-(C6-C30)arylamino, or a substituted or unsubstituted (C1-C30)alkyl(C6-C30)arylamino; or may be linked to an adjacent substituent(s) to form a substituted or unsubstituted (C3-C30), mono- or polycyclic, alicyclic or aromatic ring whose carbon atom(s) may be replaced with at least one hetero atom selected from nitrogen, oxygen, and sulfur;
R₆ represents a substituted or unsubstituted (C1-C30)alkyl, a substituted or unsubstituted (C6-C30)aryl, a substituted or unsubstituted (5- to 30-membered)heteroaryl, a substituted or unsubstituted (C3-C30)cycloalkyl, a substituted or unsubstituted mono- or di-(C1-C30)alkylamino, a substituted or unsubstituted mono- or di-(C6-C30)arylamino, or a substituted or unsubstituted (C1-C30)alkyl(C6-C30)arylamino;
R₁₁ to R₁₄, each independently, represent a substituted or unsubstituted (C1-C30)alkyl, a substituted or unsubstituted (C6-C30)aryl, or a substituted or unsubstituted (5- to 30-membered)heteroaryl; or may be linked to an adjacent substituent(s) to form a substituted or unsubstituted (C3-C30), mono- or polycyclic, alicyclic or aromatic ring whose carbon atom(s) may be replaced with at least one hetero atom selected from nitrogen, oxygen, and sulfur;
a, c, d and f, each independently, represent an integer of 0 to 4; where a, c, d or f is an integer of 2 or more, each of R₂, R₄, R₅ or R₁₀ may be the same or different;
b represents an integer of 0 to 3; where b is an integer of 2 or more, each of R₃ may be the same or different;
n represents an integer of 0 or 1; m represents an integer of 1 or 2;
* represents a bonding site to the carbazole backbone; and
the heteroaryl(ene) contains one or more hetero atoms selected from B, N, O, S, P(=O), Si, and P;
wherein the substituents of the substituted (C1-C30)alkyl, the substituted (C1-C30)alkoxy, the substituted (C3-C30)cycloalkyl, the substituted (C6-C30)aryl(ene), the substituted (5- to 30-membered)heteroaryl(ene) and the substituted (C6-C30)aryl(C1-C30)alkyl in A, L, R2 to R6 and R10 to R14 of formula 1 of the present disclosure, each independently, are at least one selected from the group consisting of deuterium, a halogen, a cyano, a carboxy, a nitro, a hydroxy, a (C1-C30)alkyl, a halo(C1-C30)alkyl, a (C2-C30)alkenyl, a (C2-C30)alkynyl, a (C1-C30)alkoxy, a (C1-C30)alkylthio, a (C3-C30)cycloalkyl, a (C3-C30)cycloalkenyl, a (3- to 7-membered)heterocycloalkyl, a (C6-C30)aryloxy, a (C6-C30)arylthio, a (3- to 30-membered)heteroaryl unsubstituted or substituted with a (C6-C30)aryl, a (C6-C30)aryl unsubstituted or substituted with a (3- to 30-membered)heteroaryl, a tri(C1-C30)alkylsilyl, a tri(C6-C30)arylsilyl, a di(C1-C30)alkyl(C6-C30)arylsilyl, a (C1-C30)alkyldi(C6-C30)arylsilyl, an amino, a mono- or di-(C1-C30)alkylamino, a mono- or di-(C6-C30)arylamino, a (C1-C30)alkyl(C6-C30)arylamino, a (C1-C30)alkylcarbonyl, a (C1-C30)alkoxycarbonyl, a (C6-C30)arylcarbonyl, a di(C6-C30)arylboronyl, a di(C1-C30)alkylboronyl, a (C1-C30)alkyl(C6-C30)arylboronyl, a (C6-C30)aryl(C1-C30)alkyl, and a (C1-C30)alkyl(C6-C30)aryl.

2. The electron buffering material according to claim 1, wherein A represents a substituted or unsubstituted nitrogen-containing (5- to 30-membered)heteroaryl selected from the group consisting of a substituted or unsubstituted triazinyl, tetrazinyl, triazolyl, tetrazolyl, furazanyl, pyridyl, pyrazinyl, pyrimidinyl, pyridazinyl, benzoimidazolyl, benzothiazolyl, benzoisothiazolyl, benzoisoxazolyl, benzoxazolyl, quinolyl, isoquinolyl, cinnolinyl, quinazolinyl, quinoxalinyl, naphthyridinyl or phenanthridinyl group; L represents a single bond or a substituted or unsubstituted (C6-C20)arylene; R₁ represents formula 2a; R₂ represents hydrogen, deuterium, a substituted or unsubstituted (C6-C20)aryl, a substituted or unsubstituted (5- to 20-membered)heteroaryl, a substituted or unsubstituted mono- or di-(C6-C20)arylamino or formula 3, or may be fused with the carbazole backbone to form a substituted or unsubstituted benzocarbazole; X represents O, S, CR₁₁R₁₂, or NR₁₃; R₁₀ represents hydrogen or (C1-C20)alkyl; R₃, R₄ and R₅, each independently, represent hydrogen or a substituted or unsubstituted (C1-C20)alkyl; R₆ represents a substituted or unsubstituted (C1-C20)alkyl, a substituted or unsubstituted (C6-C20)aryl, or a substituted or unsubstituted (6- to 20-membered)heteroaryl; R₁₁ to R₁₄, each independently, represent hydrogen, a substituted or unsubstituted (C1-C10)alkyl, a substituted or unsubstituted (C6-C20)aryl, or a substituted or unsubstituted (5- to 20-membered)heteroaryl; and the heteroaryl contains one or more hetero atoms selected from N, O and S.

3. The electron buffering material according to claim 1, wherein the compound of formula 1 is selected from the group consisting of:

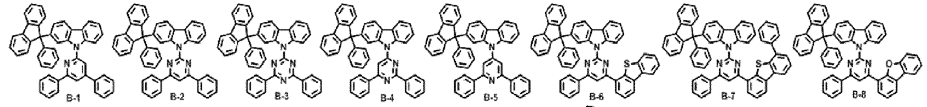

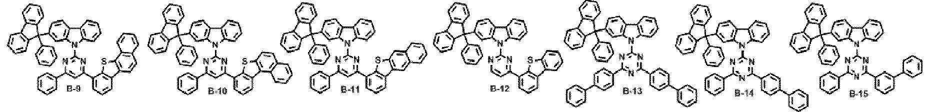

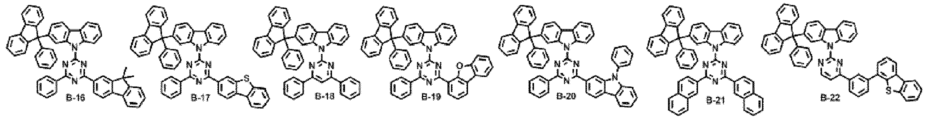

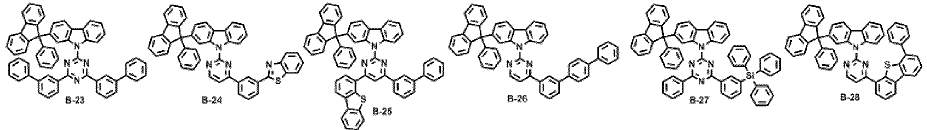

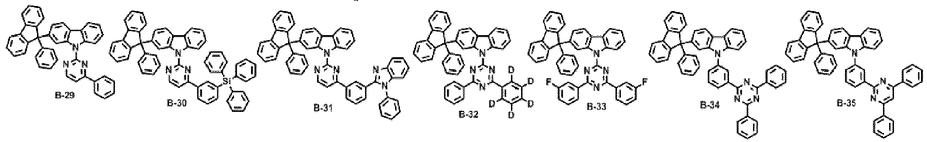

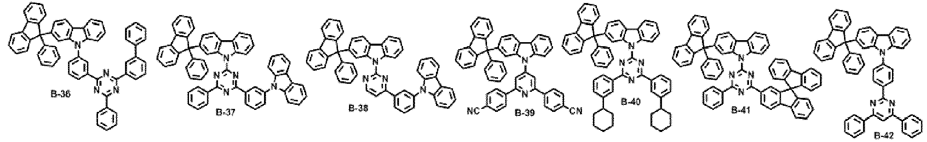

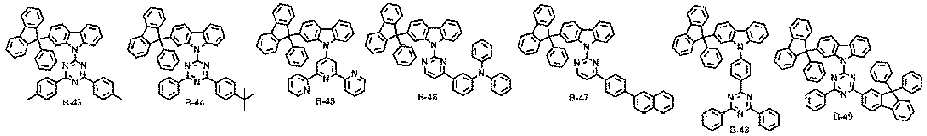

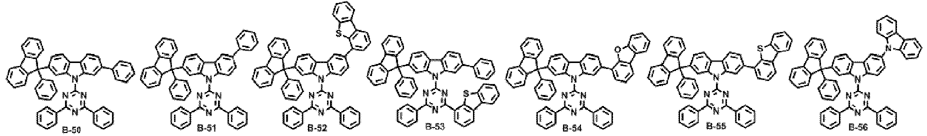

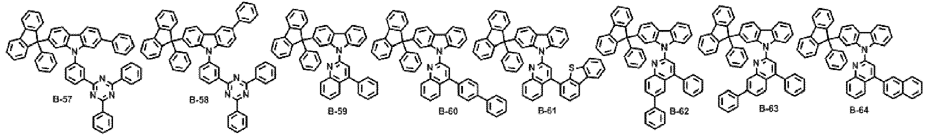

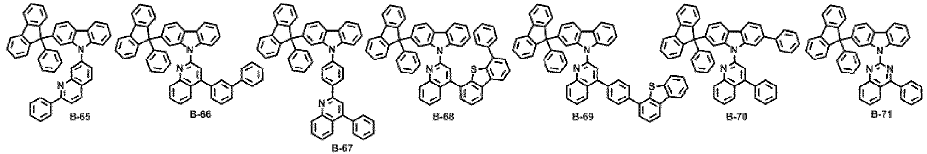

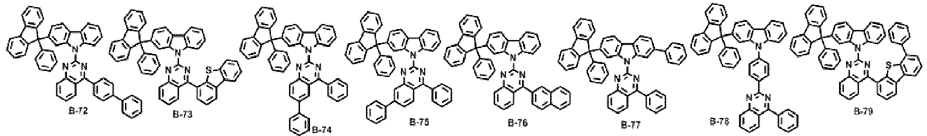

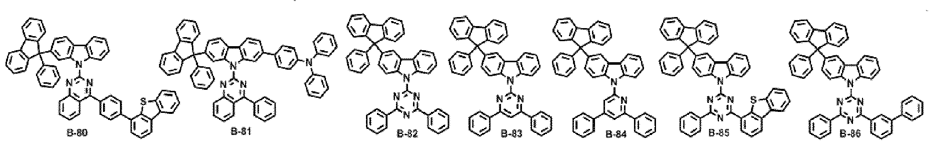

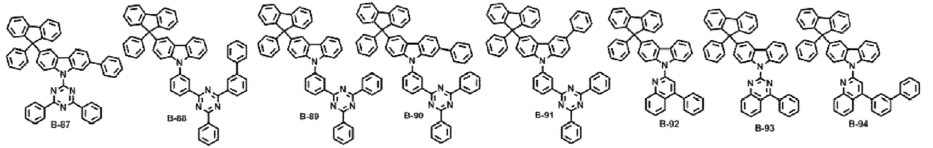

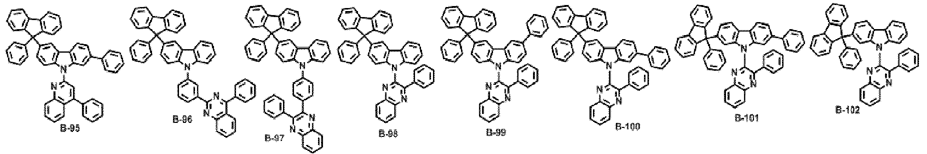

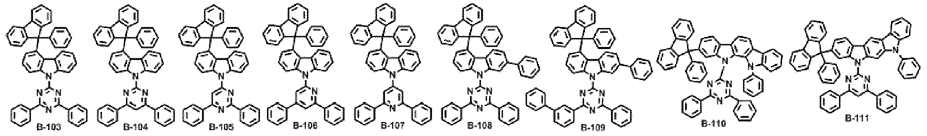

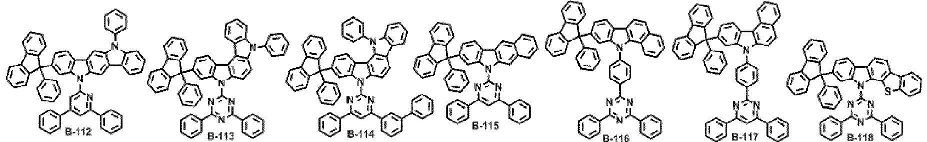

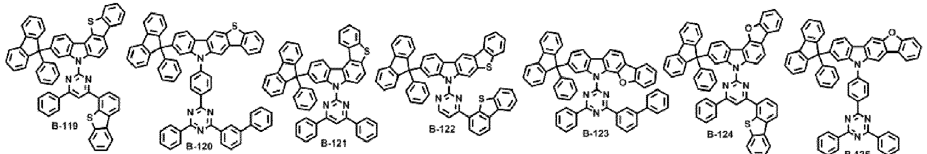

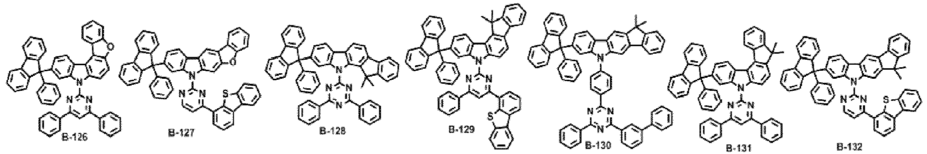

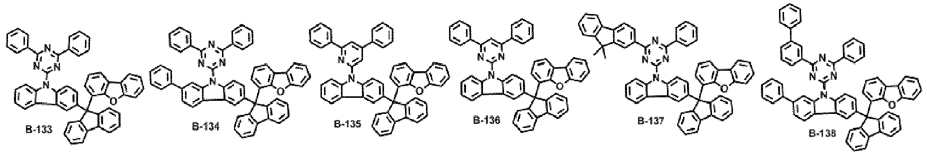

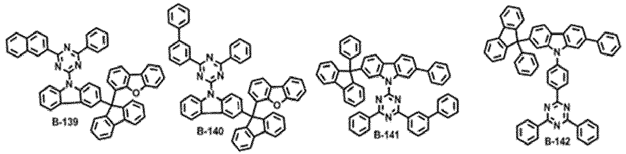

4. The electron buffering material according to claim 1, wherein a compound represented by the following formula 11 is further comprised: 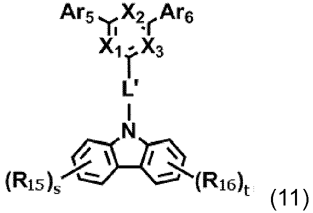 wherein
Ar₅ and Ar₆, each independently, represent a substituted or unsubstituted (5- to 30-membered)heteroaryl, or a substituted or unsubstituted (C6-C30)aryl;
L' represents a single bond, a substituted or unsubstituted (C6-C30)arylene, or a substituted or unsubstituted (3- to 30-membered)heteroarylene;
X₁ to X₃, each independently, represent N or C, with the proviso that at least one of X₁ to X₃ represents N;
R₁₅ and R₁₆, each independently, represent hydrogen, deuterium, a halogen, a cyano, a carboxy, a nitro, a hydroxy, a substituted or unsubstituted (C1-C30)alkyl, a substituted or unsubstituted (C6-C30)aryl, a substituted or unsubstituted (5- to 30-membered)heteroaryl, a substituted or unsubstituted (C3-C30)cycloalkyl, a substituted or unsubstituted (C3-C30)cycloalkenyl, a substituted or unsubstituted (3- to 7-membered)heterocycloalkyl, a substituted or unsubstituted (C1-C30)alkoxy, a substituted or unsubstituted tri(C1-C30)alkylsilyl, a substituted or unsubstituted tri(C6-C30)arylsilyl, a substituted or unsubstituted di(C1-C30)alkyl(C6-C30)arylsilyl, a substituted or unsubstituted (C1-C30)alkyldi(C6-C30)arylsilyl, a substituted or unsubstituted mono- or di-(C1-C30)alkylamino, a substituted or unsubstituted mono- or di-(C6-C30)arylamino, or a substituted or unsubstituted (C1-C30)alkyl(C6-C30)arylamino; or may be linked to an adjacent substituent(s) to form a substituted or unsubstituted (C3-C30), mono- or polycyclic, alicyclic or aromatic ring whose carbon atom(s) may be replaced with at least one hetero atom selected from nitrogen, oxygen, and sulfur;
the heteroaryl(ene) and heterocycloalkyl, each independently, contain one or more hetero atoms selected from B, N, O, S, P(=O), Si and P; and
s and t, each independently, represent an integer of 1 to 4; where s or t is an integer of 2 or more, each of R₁₅ or R₁₆ may be the same or different.

5. The electron buffering material according to claim 4, wherein Ar₅ and Ar₆, each independently, represent a substituted or unsubstituted (6- to 20-membered)heteroaryl, or a substituted or unsubstituted (C6-C20)aryl; L' represents a single bond, or a substituted or unsubstituted (C6-C20)arylene; R₁₅ and R₁₆, each independently, represent hydrogen, a substituted or unsubstituted (C6-C20)aryl, or a substituted or unsubstituted (5- to 20-membered)heteroaryl, or may be linked to an adjacent substituent(s) to form a substituted or unsubstituted (C5-C20), mono- or polycyclic aromatic ring whose carbon atom(s) may be replaced with at least one hetero atom selected from nitrogen, oxygen, and sulfur; and s and t represent 1.

6. The electron buffering material according to claim 4, wherein the compound of formula 11 is selected from the group consisting of:

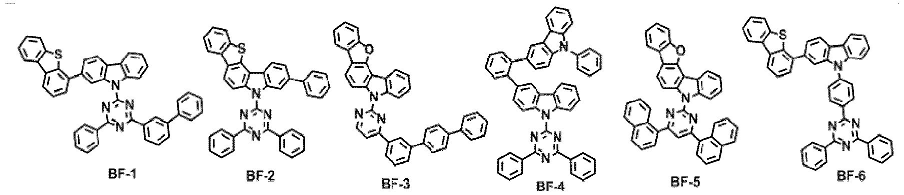

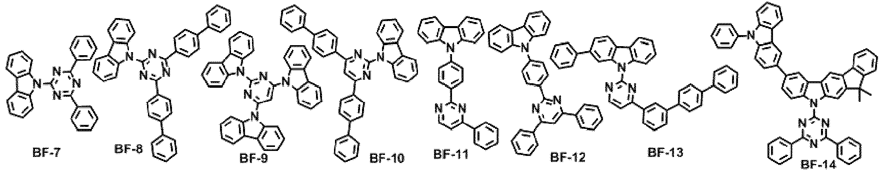

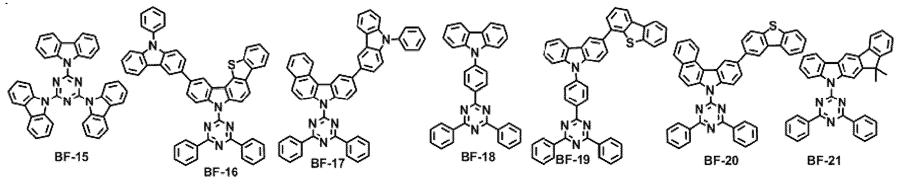

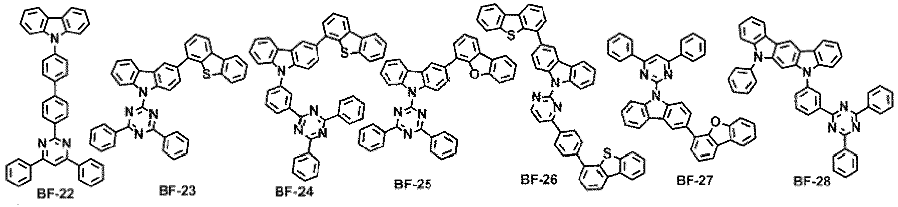

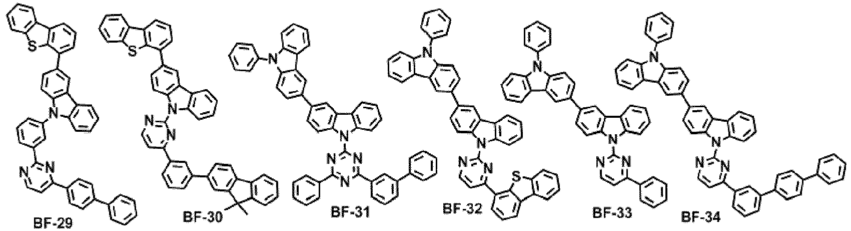

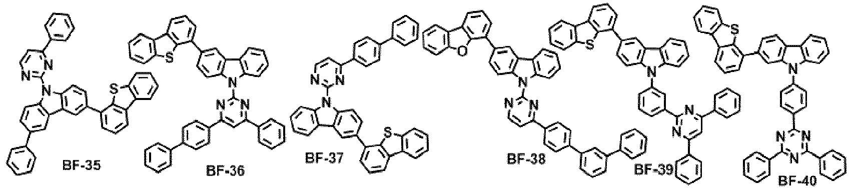

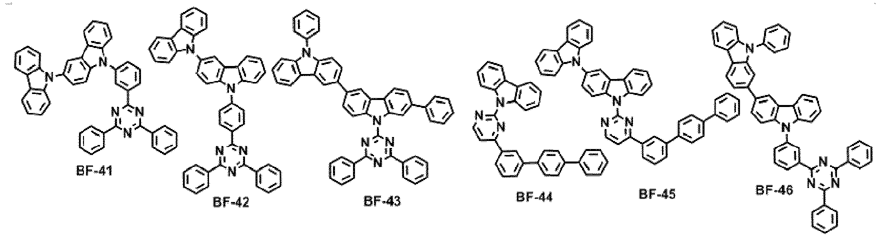

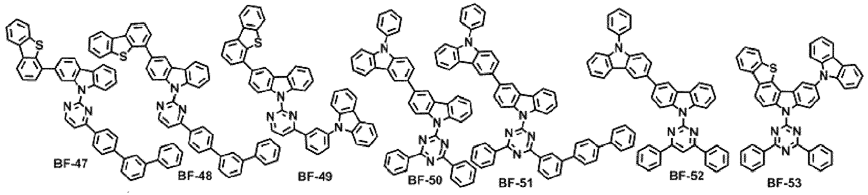

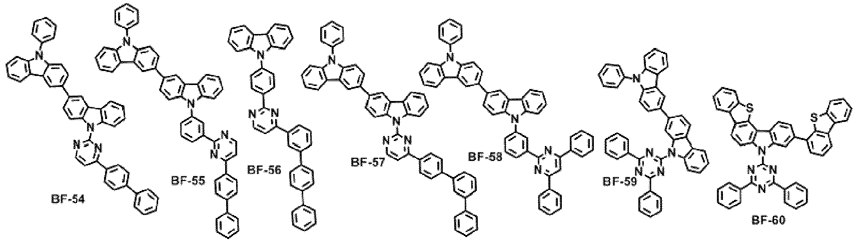

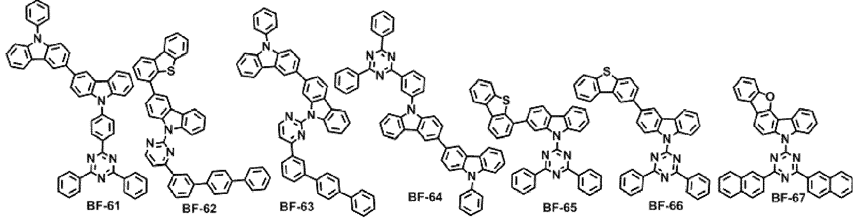

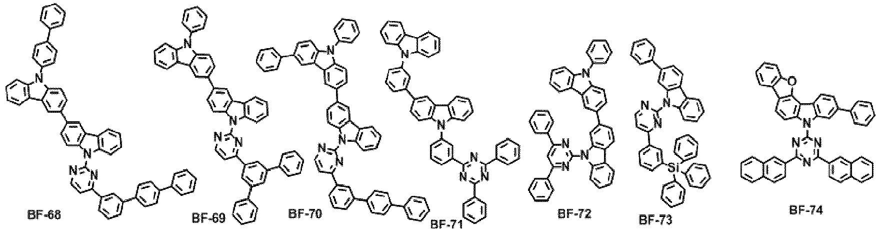

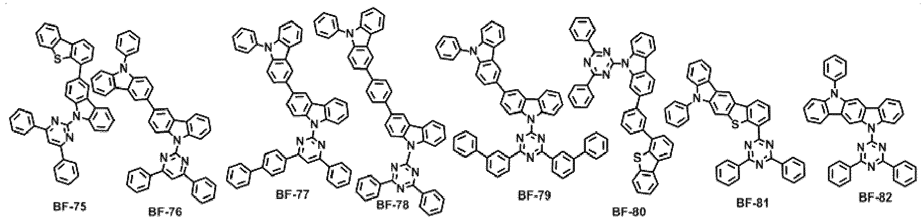

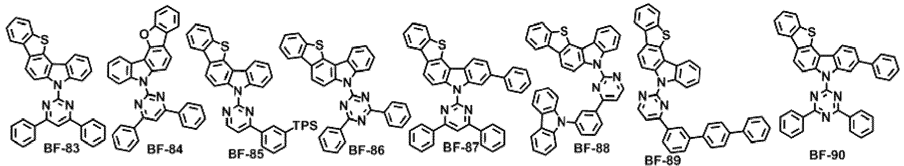

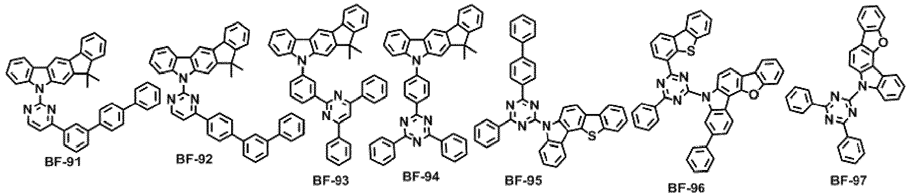

7. An organic electroluminescent device comprising a first electrode, a second electrode facing the first electrode, a light-emitting layer between the first electrode and the second electrode, and an electron transport zone and an electron buffering layer between the light-emitting layer and the second electrode, wherein the electron buffering layer comprises the compound represented by formula 1 according to claim 1.

8. The organic electroluminescent device according to claim 7, wherein the electron buffering layer further comprises the compound represented by formula 11 according to claim 4.

9. The organic electroluminescent device according to claim 8, wherein the compound represented by formula 1 and the compound represented by formula 11 of the electron buffering layer are co-evaporated or mixture-evaporated.

## Patentansprüche

1. Elektronenpuffermaterial, umfassend eine Verbindung, die dargestellt wird durch die folgende Formel 1: 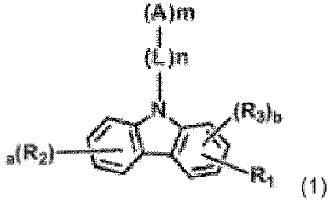 worin A ein substituiertes oder unsubstituiertes (5- bis 30-gliedriges) Heteroaryl darstellt, das ausgewählt ist aus der Gruppe bestehend aus einer substituierten oder unsubstituierten Triazinyl, Tetrazinyl-, Triazolyl-, Tetrazolyl-, Furazanyl-, Pyridyl-, Pyrazinyl-, Pyrimidinyl-, Pyridazinyl-, Benzoimidazolyl-, Benzothiazolyl-, Benzoisothiazolyl-, Benzoisoxazolyl-, Benzoxazolyl-, Chinolyl-, Isochinolyl-, Cinnolinyl-, Chinazolinyl-, Chinoxalinyl-, Naphthyridinyl- oder Phenanthridinyl-Gruppe;
L eine Einfachbindung, ein substituiertes oder unsubstituiertes (C6-C30)Arylen oder ein substituiertes oder unsubstituiertes (5- bis 30-gliedriges) Heteroarylen darstellt;
R₁ die Formel 2a darstellt: 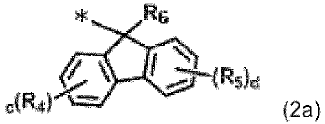
R₂ Wasserstoff, Deuterium, ein Halogen, ein Cyano, ein substituiertes oder unsubstituiertes (C1-C30) -Alkyl, ein substituiertes oder unsubstituiertes (C6-C30)Aryl, ein substituiertes oder unsubstituiertes (5- bis 30-gliedriges) Heteroaryl, ein substituiertes oder unsubstituiertes (C3-C30)Cycloalkyl, ein substituiertes oder unsubstituiertes (C1-C30)Alkoxy, ein substituiertes oder unsubstituiertes Tri(C1-C30)Alkylsilyl, ein substituiertes oder unsubstituiertes Tri(C6-C30)Arylsilyl, ein substituiertes oder unsubstituiertes Di(C1-C30)Alkyl(C6-C30)arylsilyl, ein substituiertes oder unsubstituiertes (C1-C30)Alkyl-di(C6-C30) arylsilyl, ein substituiertes oder unsubstituiertes Mono- oder Di- (C1-C30)alkylamino, ein substituiertes oder unsubstituiertes Mono- oder Di- (C6-C30)arylamino, ein substituiertes oder unsubstituiertes (C1-C30)Alkyl (C6-C30)arylamino oder die folgende Formel 3 darstellt; oder mit dem Carbazol-Grundgerüst anelliert sein kann, um ein substituiertes oder unsubstituiertes Benzocarbazol zu bilden; 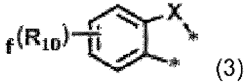
X O, S, CR₁₁R₁₂, NR₁₃ oder SiR₁₃R₁₄ darstellt;
R₃ Wasserstoff, Deuterium, ein Halogen, ein Cyano, ein substituiertes oder unsubstituiertes (C1-C30)Alkyl, ein substituiertes oder unsubstituiertes (C6-C30)Aryl, ein substituiertes oder unsubstituiertes (5- bis 30-gliedriges) Heteroaryl, ein substituiertes oder unsubstituiertes (C3-C30)Cycloalkyl, ein substituiertes oder unsubstituiertes (C1-C30)Alkoxy, ein substituiertes oder unsubstituiertes Tri(C1-C30)alkylsilyl, ein substituiertes oder unsubstituiertes Tri(C6-C30)Arylsilyl, ein substituiertes oder unsubstituiertes (C1-C30)Alkyl(C6-C30)arylsilyl, ein substituiertes oder unsubstituiertes (C1-C30)Alkyl-di(C6-C30)arylsilyl, ein substituiertes oder unsubstituiertes Mono- oder Di- (C1-C30)alkylamino, ein substituiertes oder unsubstituiertes Mono- oder Di(C6-C30)arylamino oder ein substituiertes oder unsubstituiertes (C1-C30)Alkyl (C6-C30)arylamino darstellt;
R₄, R₅ und R₁₀ jeweils unabhängig voneinander Wasserstoff, Deuterium, ein Halogen, ein Cyano, ein substituiertes oder unsubstituiertes (C1-C30)Alkyl, ein substituiertes oder unsubstituiertes (C6-C30)Aryl, ein substituiertes oder unsubstituiertes (5- bis 30-gliedriges) Heteroaryl darstellen, ein substituiertes oder unsubstituiertes (C3-C30)Cycloalkyl, ein substituiertes oder unsubstituiertes (C1-C30) Alkoxy, ein substituiertes oder unsubstituiertes Tri (C1-C30)Alkylsilyl, ein substituiertes oder unsubstituiertes Tri(C6-C30)arylsilyl, ein substituiertes oder unsubstituiertes Di(C1-C30)alkyl (C6-C30)arylsilyl, ein substituiertes oder unsubstituiertes (C1-C30)Alkyldi(C6-C30)arylsilyl, ein substituiertes oder unsubstituiertes Mono- oder Di- (C1-C30)alkylamino, ein substituiertes oder unsubstituiertes Mono- oder Di- (C6-C30)arylamino oder ein substituiertes oder unsubstituiertes (C1-C30) Alkyl(C6-C30)arylamino darstellt; oder an einem oder mehreren benachbarten Substituenten gebunden sein kann/können, um einen substituierten oder unsubstituierten (C3-C30), mono- oder polycyclischen, alicyclischen oder aromatischen Ring zu bilden, dessen Kohlenstoffatom(e) durch mindestens ein Heteroatom ersetzt werden kann, das ausgewählt ist aus Stickstoff, Sauerstoff und Schwefel;
R₆ ein substituiertes oder unsubstituiertes (C1-C30) -Alkyl, ein substituiertes oder unsubstituiertes (C6-C30)Aryl, ein substituiertes oder unsubstituiertes (5- bis 30-gliedriges) Heteroaryl, ein substituiertes oder unsubstituiertes (C3-C30)Cycloalkyl, ein substituiertes dar oder unsubstituiertes Mono- oder Di(C1-C30)Alkylamino, ein substituiertes oder unsubstituiertes Mono- oder Di- (C6-C30)arylamino oder ein substituiertes oder unsubstituiertes (C1-C30) -Alkyl (C6-C30)arylamino darstellt;
R₁₁ bis R₁₄ jeweils unabhängig voneinander ein substituiertes oder unsubstituiertes (C1-C30) -Alkyl, ein substituiertes oder unsubstituiertes (C6-C30)Aryl oder ein substituiertes oder unsubstituiertes (5- bis 30-gliedriges) Heteroaryl darstellen; oder an einem oder mehreren benachbarten Substituenten gebunden sein können, um einen substituierten oder unsubstituierten (C3-C30), mono- oder polycyclischen, alicyclischen oder aromatischen Ring zu bilden, dessen Kohlenstoffatom(e) durch mindestens ein Heteroatom ersetzt können, das ausgewählt ist aus Stickstoff, Sauerstoff und Schwefel;
a, c, d und f jeweils unabhängig voneinander eine ganze Zahl von 0 bis 4 darstellen; wobei a, c, d oder f eine ganze Zahl von 2 oder mehr darstellen, jedes R₂, R₄, R₅ oder R₁₀ gleich oder verschieden sein kann;
b eine ganze Zahl von 0 bis 3 darstellt; wobei b eine ganze Zahl von 2 oder mehr ist, jedes R₃ gleich oder verschieden sein kann;
n eine ganze Zahl von 0 oder 1 darstellt; m eine ganze Zahl von 1 oder 2 darstellt;
* eine Bindungsstelle an dem Carbazol-Grundgerüst darstellt; und
das Heteroaryl(en) ein oder mehrere Heteroatome enthält, die ausgewählt sind aus B, N, O, S, P(=O), Si und P;
wobei die Substituenten des substituierten (C1-C30) -Alkyls, des substituierten (C1-C30) -Alkoxys, des substituierten (C3-C30) -Cycloalkyls, des substituierten (C6-C30) -Aryl(en)s, des substituierten (5- bis 30)gliedrigen Heteroaryl(en)s und des substituierten (C6-C30)Aryl(C1-C30)alkyls in A, L, R2 bis R6 und R10 bis R14 der Formel 1 der vorliegenden Offenbarung jeweils unabhängig voneinander mindestens ausgewählt sind aus der Gruppe bestehend aus Deuterium, einem Halogen, einem Cyano, einem Carboxy, einem Nitro, einem Hydroxy, einem (C1-C30)Alkyl, einem Halogen(C1-C30)alkyl, einem (C2-C30)Alkenyl, einem (C2-C30)Alkinyl, einem (C1-C30)Alkoxy, einem (C1-C30)Alkylthio, einem (C3-C30)Cycloalkyl, einem (C3-C30)-Cycloalkenyl, einem (3- bis 7-gliedrigen) Heterocycloalkyl, einem (C6-C30)Aryloxy, einem (C6-C30)Arylthio, einem (3- bis 30-gliedrigen) Heteroaryl, unsubstituiert oder substituiert mit einem (C6-C30)Aryl, einem (C6-C30)Aryl, unsubstituiert oder substituiert mit einem (3- bis 30-gliedrigen) Heteroaryl, einem Tri(C1-C30)alkylsilyl, einem Tri(C6-C30)arylsilyl, einem Di(C1-C30)alkyl (C6-C30)arylsilyl, einem (C1-C30)Alkyldi(C6-C30)arylsilyl, einem Aminoeinem Mono- oder Di- (C1-C30)-Alkylamino, ein Mono- oder Di- (C6-C30)arylamino, einem (C1-C30)Alkyl (C6-C30)arylamino, einem (C1-C30)Alkylcarbonyl, einem (C1-C30) Alkoxycarbonyl, einem (C6-C30)Arylcarbonyl, einem Di(C6-C30)arylboronyl, ein Di(C1-C30)alkylboronyl, einem (C1-C30)Alkyl(C6-C30)arylboronyl, einem (C6-C30)Aryl(C1-C30)alkyl und einem (C1-C30)Alkyl(C6-C30)aryl.

2. Elektronenpuffermaterial nach Anspruch 1, wobei A ein substituiertes oder unsubstituiertes stickstoffhaltiges (5- bis 30-gliedriges) Heteroaryl darstellt, das ausgewählt ist aus der Gruppe bestehend aus einer substituierten oder unsubstituierten Triazinyl, Tetrazinyl-, Triazolyl-, Tetrazolyl-, Furazanyl-, Pyridyl-, Pyrazinyl-, Pyrimidinyl-, Pyridazinyl-, Benzoimidazolyl-, Benzothiazolyl-, Benzoisothiazolyl-, Benzoisoxazolyl-, Benzoxazolyl-, Chinolyl-, Isochinolyl-, Cinnolinyl-, Chinazolinyl-, Chinoxalinyl-, Naphthyridinyl- oder Phenanthridinyl-Gruppe; L eine Einfachbindung oder ein substituiertes oder unsubstituiertes (C6-C20)Arylen darstellt; R₁ Formel 2a darstellt; R₂ Wasserstoff, Deuterium, ein substituiertes oder unsubstituiertes (C6-C20)Aryl, ein substituiertes oder unsubstituiertes (5- bis 20-gliedriges) Heteroaryl, ein substituiertes oder unsubstituiertes Mono- oder Di(C6-C20)arylamino oder Formel 3 darstellt, oder mit dem Carbazol-Grundgerüst anneliert sein kann, um ein substituiertes oder unsubstituiertes Benzocarbazol zu bilden; X O, S, CR₁₁R₁₂ oder NR₁₃ darstellt; R₁₀ Wasserstoff oder (C1-C20)Alkyl darstellt; R₃, R₄ und R₅ jeweils unabhängig voneinander Wasserstoff oder ein (C1-C20)Alkyl darstellen, R₅ ein substituiertes oder unsubstituiertes (C1-C20)Alkyl, ein substituiertes oder unsubstituiertes (C6-C20)Aryl oder ein substituiertes oder unsubstituiertes (5- bis 20-gliedriges) Heteroaryl darstellt; R₁₁ bis R₁₄ jeweils unabhängig voneinander ein substituiertes oder unsubstituiertes (C1-C10)Alkyl, ein substituiertes oder unsubstituiertes (C6-C20)Aryl oder ein substituiertes oder unsubstituiertes (5- bis 30-gliedriges) Heteroaryl darstellen und das Heteroaryl ein oder mehrere Heteroatome enthält, ausgewählt aus N, O und S.

3. Elektronenpuffermaterial nach Anspruch 1, wobei die Verbindung von Formel 1 ausgewählt ist aus der Gruppe bestehend aus:

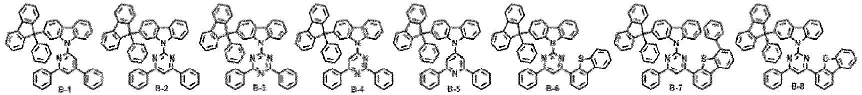

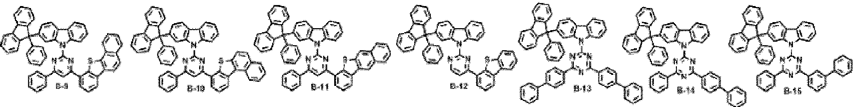

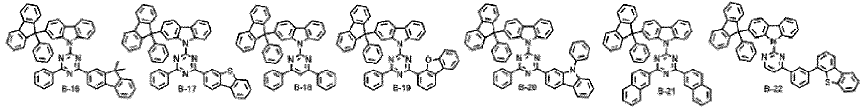

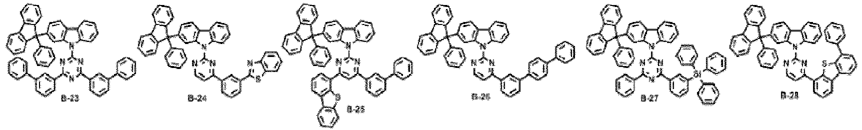

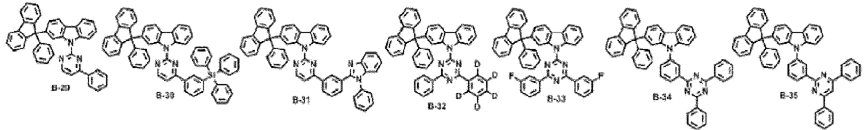

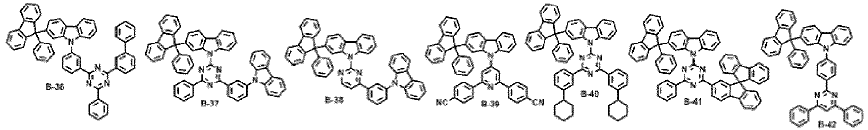

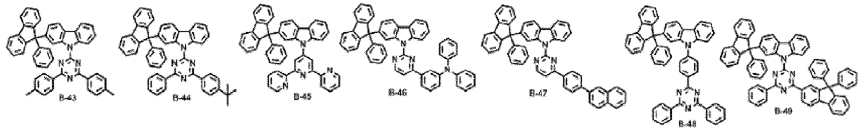

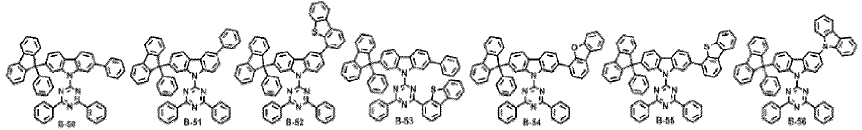

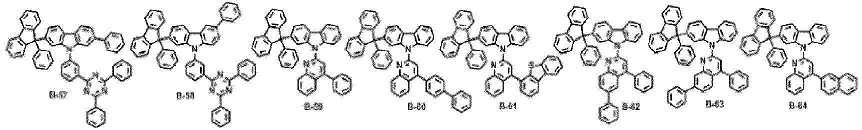

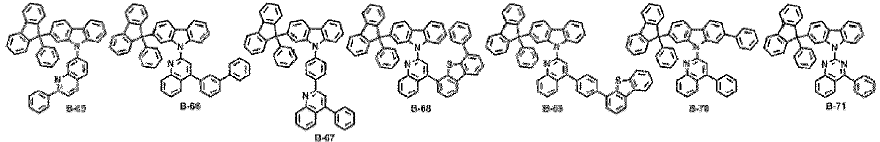

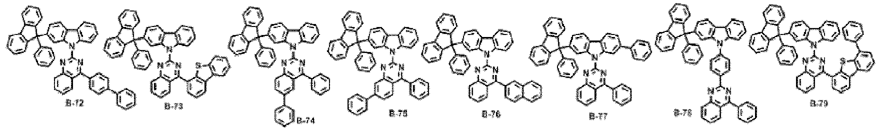

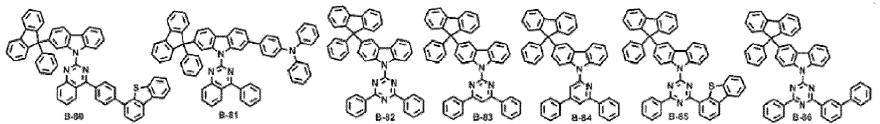

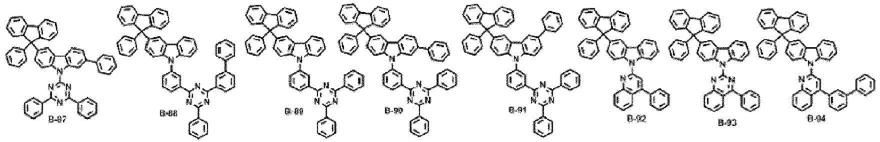

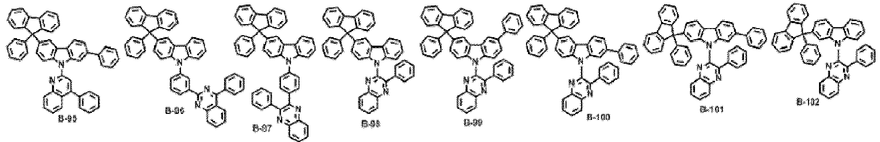

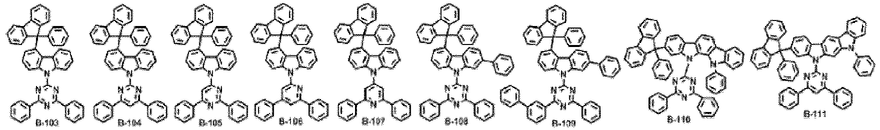

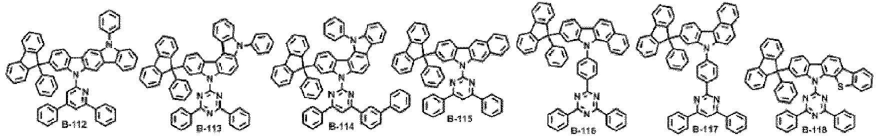

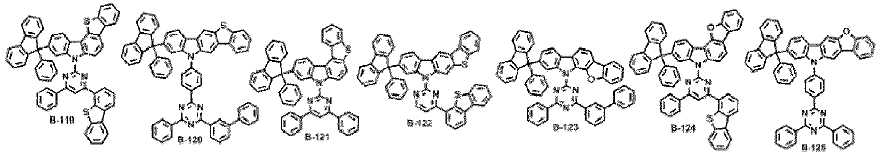

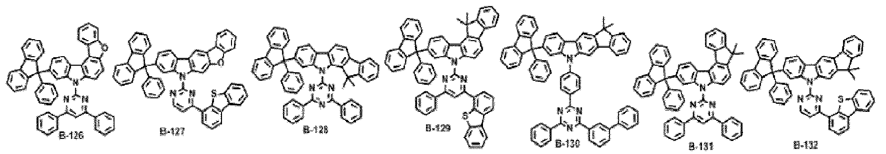

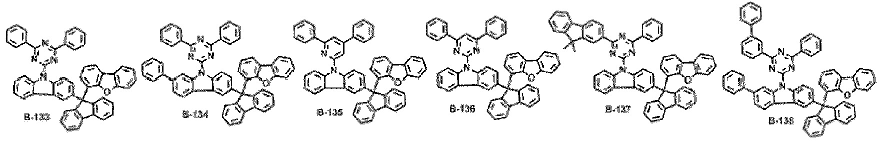

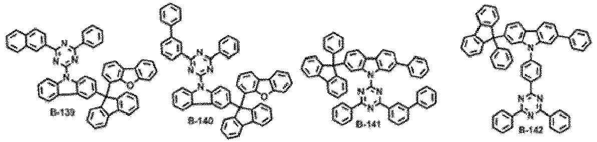

4. Elektronenpuffermaterial nach Anspruch 1, wobei eine Verbindung, die dargestellt ist durch die folgende Formel 11, ferner umfasst: 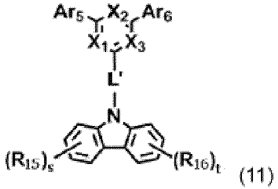 worin
Ar₅ und Ar₆, jeweils unabhängig voneinander ein substituiertes oder unsubstituiertes (5- bis 30-gliedriges) Heteroaryl, oder ein substituiertes oder unsubstituiertes (C6-C30)Aryl darstellen;
L' eine Einfachbindung, ein substituiertes oder unsubstituiertes (C6-C30)Arylen oder ein substituiertes oder unsubstituiertes (3- bis 30-gliedriges) Heteroarylen darstellt;
X₁ bis X₃ jeweils unabhängig voneinander N oder C darstellen, mit der Maßgabe, dass mindestens eines von X₁ bis X₃ N darstellen;
R₁₅ und R₁₆ jeweils unabhängig voneinander Wasserstoff, Deuterium, ein Halogen, ein Cyano, ein Carboxy, ein Nitro, ein Hydroxy, ein substituiertes oder unsubstituiertes (C1-C30)Alkyl, ein substituiertes oder unsubstituiertes (C6-C30)Aryl, ein substituiertes oder unsubstituiertes (5- bis 30-gliedriges) Heteroaryl darstellen, ein substituiertes oder unsubstituiertes (C3-C30)Cycloalkyl, ein substituiertes oder unsubstituiertes (C3-C30)Cycloalkenyl, ein substituiertes oder unsubstituiertes (3- bis 7-gliedriges) Heterocycloalkyl, ein substituiertes oder unsubstituiertes (C1-C30)Alkoxy, ein substituiertes oder unsubstituiertes Tri (C1-C30)Alkylsilyl, ein substituiertes oder unsubstituiertes Tri(C6-C30)arylsilyl, ein substituiertes oder unsubstituiertes Di(C1-C30)alkyl (C6-C30)arylsilyl, ein substituiertes oder unsubstituiertes (C1-C30)Alkyl-di(C6-C30)arylsilyl, ein substituiertes oder unsubstituiertes Mono- oder Di- (C1-C30)alkylamino, ein substituiertes oder unsubstituiertes Mono- oder Di- (C6-C30)arylamino oder ein substituiertes oder unsubstituiertes (C1-C30)Alkyl(C6-C30)arylamino darstellt; oder an einem oder mehreren benachbarten Substituenten gebunden sein kann/können, um einen substituierten oder unsubstituierten (C3-C30), mono- oder polycyclischen, alicyclischen oder aromatischen Ring zu bilden, dessen Kohlenstoffatom(e) durch mindestens ein Heteroatom ersetzt werden kann/können, ausgewählt aus Stickstoff, Sauerstoff und Schwefel;
das Heteroaryl(en) und Heterocycloalkyl jedes unabhängig ein oder mehrere Heteroatome enthält, die ausgewählt sind aus B, N, O, S, P(=O), Si und P; und
s und t jeweils unabhängig voneinander eine ganze Zahl von 1 bis 4 darstellen, wobei s oder t eine ganze Zahl von 2 oder größer ist, jedes R₁₅ oder R₁₆ gleich oder verschieden sein kann.

5. Elektronenpuffermaterial nach Anspruch 4, wobei Ar₅ oder Ar₆ jeweils unabhängig voneinander ein substituiertes oder unsubstituiertes (6- bis 20-gliedriges) Heteroaryl oder substituiertes oder unsubstituiertes (C6-C20)Aryl darstellen; L' eine Einfachbindung, oder ein substituiertes oder unsubstituiertes (C6-C20)Arylen darstellt; R₁₅ und R₁₆ jeweils unabhängig voneinander Wasserstoff, ein substituiertes oder unsubstituiertes (C6-C20)Aryl oder ein substituiertes oder unsubstituiertes (C6-C20)Aryl oder ein substituiertes oder unsubstituiertes (5- bis 20-gliedriges) Heteroaryl darstellen oder mit einem oder mehreren benachbarten Substituenten verbunden sein können, um einen substituierten oder unsubstituierten (C5-C20)-, mono- oder polycyclischen, alicyclischen oder aromatischen Ring zu bilden, dessen Kohlenstoffatom(e) durch mindestens ein Heteroatom ersetzt werden kann, das ausgewählt ist aus Stickstoff, Sauerstoff und Schwefel; und s und t 1 darstellen.

6. Elektronenpuffermaterial nach Anspruch 4, wobei die Verbindung von Formel 11 ausgewählt ist aus der Gruppe bestehend aus:

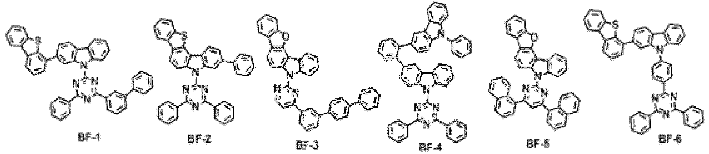

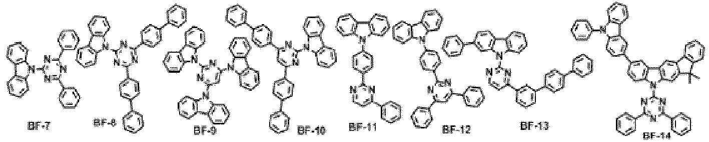

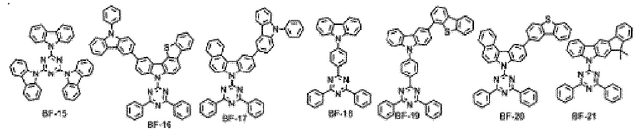

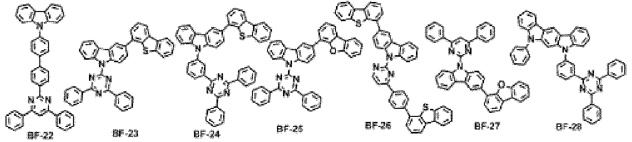

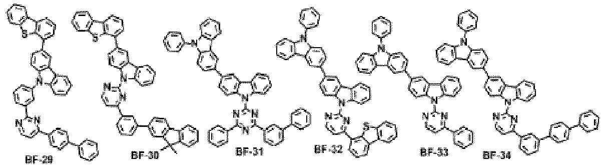

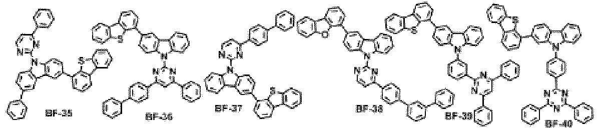

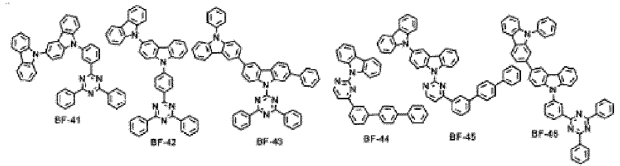

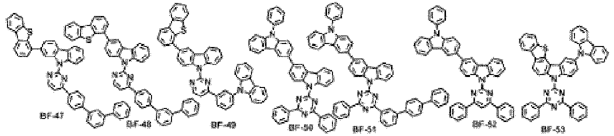

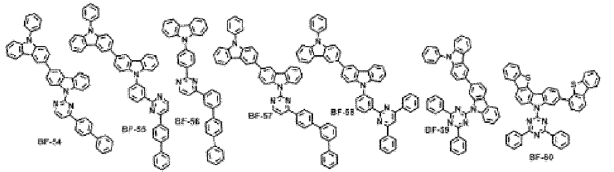

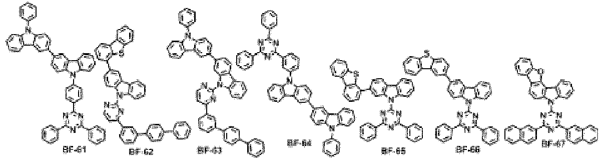

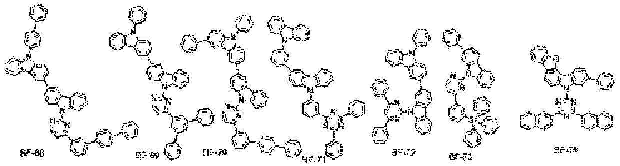

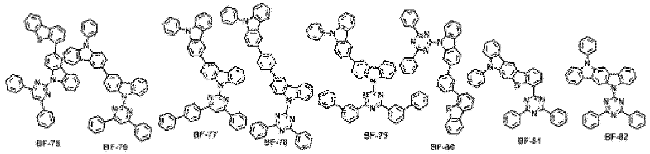

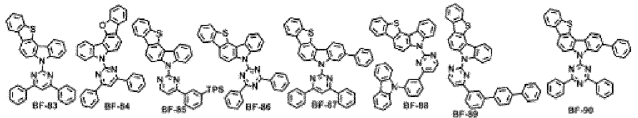

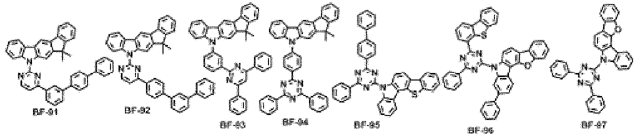

7. Organische elektrolumineszente Vorrichtung, umfassend eine erste Elektrode, eine zweite Elektrode, die der ersten Elektrode zugewandt ist, eine Licht emittierende Schicht zwischen der ersten Elektrode und der zweiten Elektrode und eine Elektronentransportzone und eine Elektronenpufferschicht zwischen der Licht emittierenden Schicht und der zweiten Elektrode, wobei die Elektronenpufferschicht die durch Formel 1 dargestellte Verbindung nach Anspruch 1 umfasst.

8. Organische elektrolumineszente Vorrichtung nach Anspruch 7, wobei die Elektronenpufferschicht ferner die durch Formel 11 nach Anspruch 4 dargestellte Verbindung umfasst.

9. Organische elektrolumineszente Vorrichtung nach Anspruch 8, wobei die durch Formel 1 dargestellte Verbindung und die durch Formel 11 dargestellte Verbindung der Elektronenpufferschicht gemeinsam verdampft oder durch Mischung aufgedampft sind.

## Revendications

1. Matériau tampon d'électrons comprenant un composé représenté par la formule 1 qui suit: 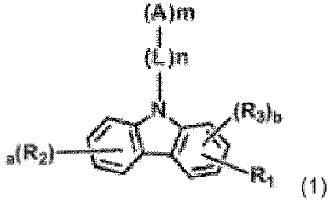 dans lequel:
A représente un (5 à 30 membres)hétéroaryle substitué ou non substitué choisi dans le groupe constitué de: un groupe triazinyle, tétrazinyle, triazolyle, tétrazolyle, furazanyle, pyridyle, pyrazinyle, pyrimidinyle, pyridazinyle, benzoimidazolyle, benzothiazolyle, benzoisothiazolyle, benzoisoxazolyle, benzoxazolyle, quinoléyle, isoquinoléyle, cinnolinyle, quinazolinyle, quinoxalinyle, naphtyridinyle ou phénanthridinyle substitué ou non substitué;
L représente une liaison simple, un (C6-C30)arylène substitué ou non substitué ou un (5 à 30 membres)hétéroarylène substitué ou non substitué;
R₁ représente la formule 2a: 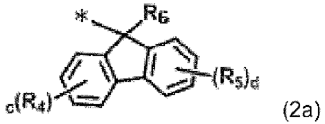
R₂ représente un hydrogène, un deutérium, un halogène, un cyano, un (C1-C30)alkyle substitué ou non substitué, un (C6-C30)aryle substitué ou non substitué, un (5 à 30 membres)hétéroaryle substitué ou non substitué, un (C3-C30)cycloalkyle substitué ou non substitué, un (C1-C30)alcoxy substitué ou non substitué, un tri(C1-C30)alkylsilyle substitué ou non substitué, un tri(C6-C30)arylsilyle substitué ou non substitué, un di(C1-C30)alkyl(C6-C30)arylsilyle substitué ou non substitué, un (C1-C30)alkyldi(C6-C30)arylsilyle substitué ou non substitué, un mono- ou di-(C1-C30)alkylamino substitué ou non substitué, un mono- ou di-(C6-C30)arylamino substitué ou non substitué, un (C1-C30)alkyl(C6-C30)arylamino substitué ou non substitué, ou la formule 3 qui suit; ou peut être condensé avec le squelette de carbazole pour former un benzocarbazole substitué ou non substitué; 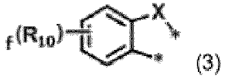
X représente O, S, CR₁₁R₁₂, NR₁₃ ou SiR₁₃R₁₄;
R₃ représente un hydrogène, un deutérium, un halogène, un cyano, un (C1-C30)alkyle substitué ou non substitué, un (C6-C30)aryle substitué ou non substitué, un (5 à 30 membres)hétéroaryle substitué ou non substitué, un (C3-C30)cycloalkyle substitué ou non substitué, un (C1-C30)alcoxy substitué ou non substitué, un tri(C1-C30)alkylsilyle substitué ou non substitué, un tri(C6-C30)arylsilyle substitué ou non substitué, un di(C1-C30)alkyl(C6-C30)arylsilyle substitué ou non substitué, un (C1-C30)alkyldi(C6-C30)arylsilyle substitué ou non substitué, un mono- ou di-(C1-C30)alkylamino substitué ou non substitué, un mono- ou di-(C6-C30)arylamino substitué ou non substitué ou un (C1-C30)alkyl(C6-C30)arylamino substitué ou non substitué;
R₄, R₅ et R₁₀, chacun indépendamment, représentent un hydrogène, un deutérium, un halogène, un cyano, un (C1-C30)alkyle substitué ou non substitué, un (C6-C30)aryle substitué ou non substitué, un (5 à 30 membres)hétéroaryle substitué ou non substitué, un (C3-C30)cycloalkyle substitué ou non substitué, un (C1-C30)alcoxy substitué ou non substitué, un tri(C1-C30)alkylsilyle substitué ou non substitué, un tri(C6-C30)arylsilyle substitué ou non substitué, un di(C1-C30)alkyl(C6-C30)arylsilyle substitué ou non substitué, un (C1-C30)alkyldi(C6-C30)arylsilyle substitué ou non substitué, un mono- ou di-(C1-C30)alkylamino substitué ou non substitué, un mono- ou di-(C6-C30)arylamino substitué ou non substitué ou un (C1-C30)alkyl(C6-C30)arylamino substitué ou non substitué; ou peuvent être liés à un ou des substituant(s) adjacent(s) pour former un cycle (C3-C30), mono- ou polycyclique, alicyclique ou aromatique substitué ou non substitué dont le ou les atome(s) de carbone peu(ven)t être remplacé(s) avec au moins un hétéroatome choisi parmi azote, oxygène et soufre;
R₆ représente un (C1-C30)alkyle substitué ou non substitué, un (C6-C30)aryle substitué ou non substitué, un (5 à 30 membres)hétéroaryle substitué ou non substitué, un (C3-C30)cycloalkyle substitué ou non substitué, un mono- ou di-(C1-C30)alkylamino substitué ou non substitué, un mono- ou di-(C6-C30)arylamino substitué ou non substitué ou un (C1-C30)alkyl(C6-C30)arylamino substitué ou non substitué;
R₁₁ à R₁₄, chacun indépendamment, représentent un (C1-C30)alkyle substitué ou non substitué, un (C6-C30)aryle substitué ou non substitué ou un (5 à 30 membres)hétéroaryle substitué ou non substitué; ou peuvent être liés à un ou des substituant(s) adjacent(s) pour former un cycle (C3-C30), mono- ou polycyclique, alicyclique ou aromatique substitué ou non substitué dont le ou les atome(s) de carbone peu(ven)t être remplacé(s) avec au moins un hétéroatome choisi parmi azote, oxygène et soufre;
a, c, d et f, chacun indépendamment, représentent un nombre entier de 0 à 4; lorsque a, c, d ou f est un nombre entier de 2 ou plus, les R₂, R₄, R₅ ou R₁₀ peuvent être chacun identiques ou différents;
b représente un nombre entier de 0 à 3; lorsque b est un nombre entier de 2 ou plus, les R₃ peuvent être chacun identiques ou différents;
n représente un nombre entier de 0 ou 1; m représente un nombre entier de 1 ou 2;
* représente un site de liaison au squelette de carbazole; et
l'hétéroaryl(ène) contient un ou plusieurs hétéroatomes choisis parmi B, N, O, S, P(=O), Si et P;
dans lequel les substituants du (C1-C30)alkyle substitué, du (C1-C30)alcoxy substitué, du (C3-C30)cycloalkyle substitué, du (C6-C30)aryl(ène) substitué, du (5 à 30 membres)hétéroaryl(ène) substitué et du (C6-C30)aryl(C1-C30)alkyle substitué dans A, L, R₂ à R₆ et R₁₀ à R₁₄ de la formule 1 du présent exposé, chacun indépendamment, sont au moins un choisi dans le groupe constitué de: un deutérium, un halogène, un cyano, un carboxy, un nitro, un hydroxy, un (C1-C30)alkyle, un halo(C1-C30)alkyle, un (C2-C30)alcényle, un (C2-C30)alcynyle, un (C1-C30)alcoxy, un (C1-C30)alkylthio, un (C3-C30)cycloalkyle, un (C3-C30)cycloalcényle, un (3 à 7 membres)hétérocycloalkyle, un (C6-C30)aryloxy, un (C6-C30)arylthio, un (3 à 30 membres)hétéroaryle non substitué ou substitué avec un (C6-C30)aryle, un (C6-C30)aryle non substitué ou substitué avec un (3 à 30 membres)hétéroaryle, un tri(C1-C30)alkylsilyle, un tri(C6-C30)arylsilyle, un di(C1-C30)alkyl(C6-C30)arylsilyle, un (C1-C30)alkyldi(C6-C30)arylsilyle, un amino, un mono- ou di-(C1-C30)alkylamino, un mono- ou di-(C6-C30)arylamino, un (C1-C30)alkyl(C6-C30)arylamino, un (C1-C30)alkylcarbonyle, un (C1-C30)alcoxycarbonyle, un (C6-C30)arylcarbonyle, un di(C6-C30)arylboronyle, un di(C1-C30)alkylboronyle, un (C1-C30)alkyl(C6-C30)arylboronyle, un (C6-C30)aryl(C1-C30)alkyle et un (C1-C30)alkyl(C6-C30)aryle.

2. Matériau tampon d'électrons selon la revendication 1, dans lequel A représente un (5 à 30 membres)hétéroaryle contenant de l'azote substitué ou non substitué choisi dans le groupe constitué de: un groupe triazinyle, tétrazinyle, triazolyle, tétrazolyle, furazanyle, pyridyle, pyrazinyle, pyrimidinyle, pyridazinyle, benzoimidazolyle, benzothiazolyle, benzoisothiazolyle, benzoisoxazolyle, benzoxazolyle, quinoléyle, isoquinoléyle, cinnolinyle, quinazolinyle, quinoxalinyle, naphtyridinyle ou phénanthridinyle substitué ou non substitué; L représente une liaison simple ou un (C6-C20)arylène substitué ou non substitué; R₁ représente la formule 2a; R₂ représente un hydrogène, un deutérium, un (C6-C20)aryle substitué ou non substitué, un (5 à 20 membres)hétéroaryle substitué ou non substitué, un mono- ou di-(C6-C20)arylamino substitué ou non substitué, ou la formule 3, ou peut être condensé avec le squelette de carbazole pour former un benzocarbazole substitué ou non substitué; X représente O, S, CR₁₁R₁₂ ou NR₁₃; R₁₀ représente un hydrogène ou un (C1-C20)alkyle; R₃, R₄ et R₅, chacun indépendamment, représentent un hydrogène ou un (C1-C20)alkyle substitué ou non substitué; R₆ représente un (C1-C20)alkyle substitué ou non substitué, un (C6-C20)aryle substitué ou non substitué ou un (6 à 20 membres)hétéroaryle substitué ou non substitué; R₁₁ à R₁₄, chacun indépendamment, représentent un hydrogène, un (C1-C10)alkyle substitué ou non substitué, un (C6-C20)aryle substitué ou non substitué ou un (5 à 20 membres)hétéroaryle substitué ou non substitué; et l'hétéroaryle contient un ou plusieurs hétéroatomes choisis parmi N, O et S.

3. Matériau tampon d'électrons selon la revendication 1, dans lequel le composé de la formule 1 est choisi dans le groupe constitué de:

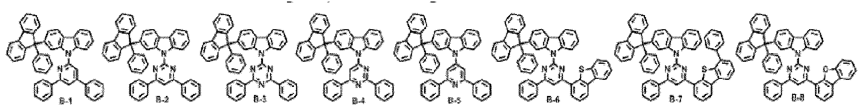

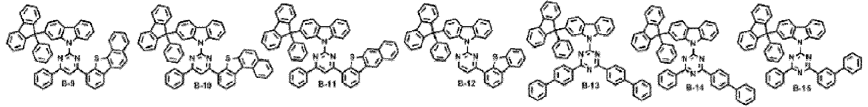

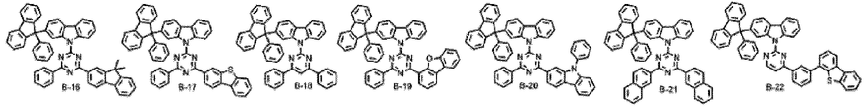

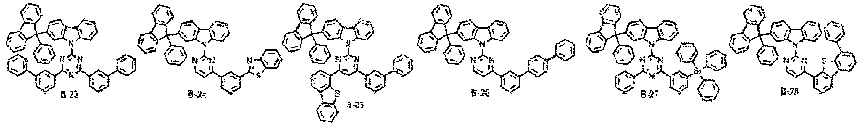

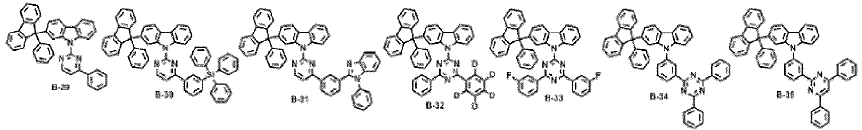

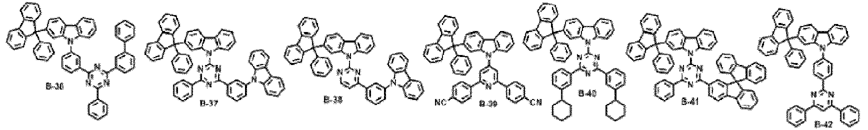

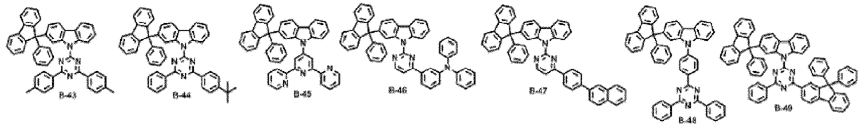

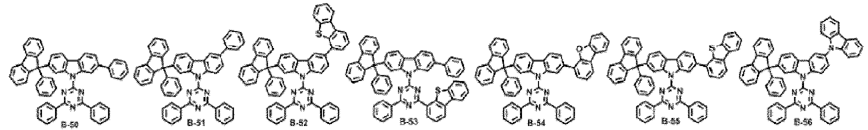

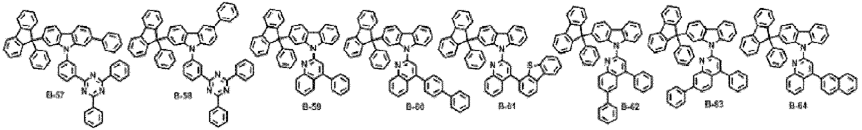

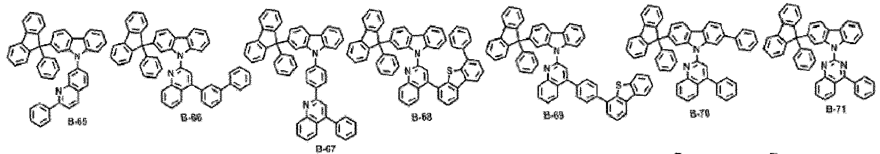

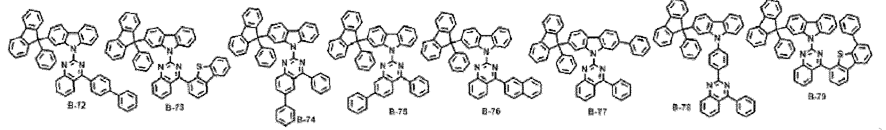

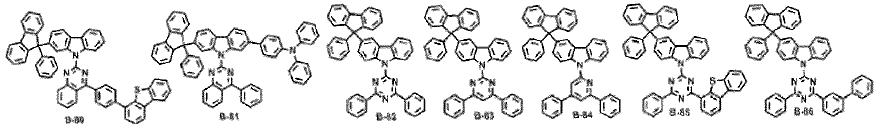

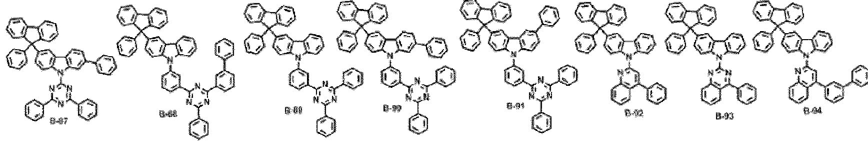

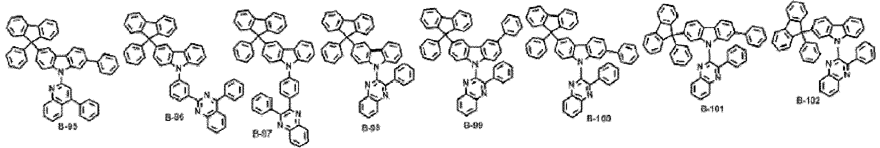

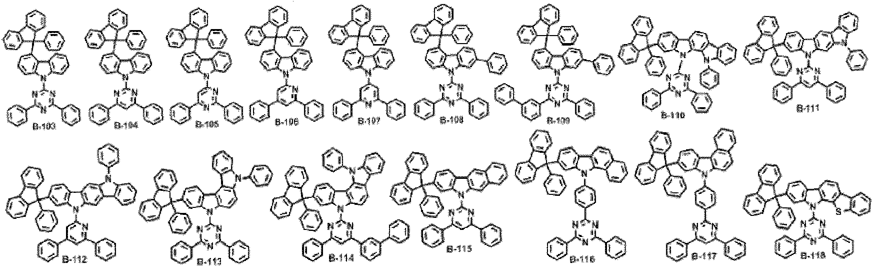

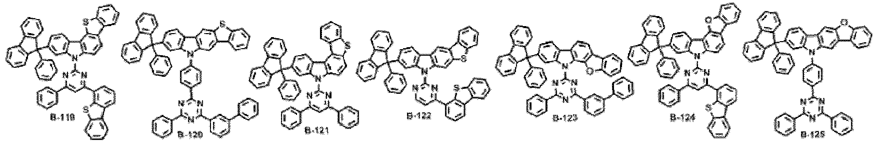

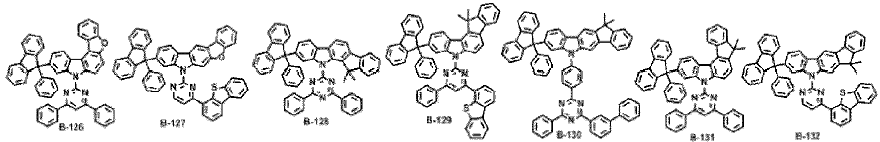

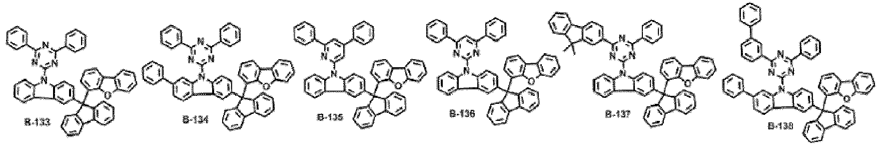

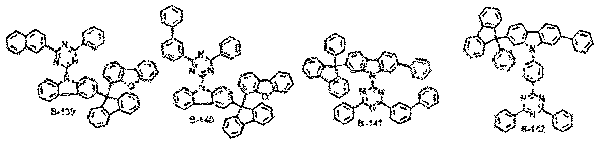

4. Matériau tampon d'électrons selon la revendication 1, dans lequel il est en outre compris un composé représenté par la formule 11 qui suit: 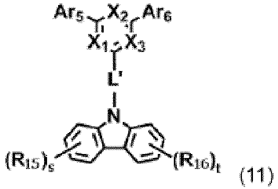 dans lequel:
Ar₅ et Ar₆, chacun indépendamment, représentent un (5 à 30 membres)hétéroaryle substitué ou non substitué ou un (C6-C30)aryle substitué ou non substitué;
L' représente une liaison simple, un (C6-C30)arylène substitué ou non substitué ou un (3 à 30 membres)hétéroarylène substitué ou non substitué;
X₁ à X₃, chacun indépendamment, représentent N ou C, sous réserve que au moins un de X₁ à X₃ représente N;
R₁₅ et R₁₆, chacun indépendamment, représentent un hydrogène, un deutérium, un halogène, un cyano, un carboxy, un nitro, un hydroxy, un (C1-C30)alkyle substitué ou non substitué, un (C6-C30)aryle substitué ou non substitué, un (5 à 30 membres)hétéroaryle substitué ou non substitué, un (C3-C30)cycloalkyle substitué ou non substitué, un (C3-C30)cycloalcényle substitué ou non substitué, un (3 à 7 membres)hétérocycloalkyle substitué ou non substitué, un (C1-C30)alcoxy substitué ou non substitué, un tri(C1-C30)alkylsilyle substitué ou non substitué, un tri(C6-C30)arylsilyle substitué ou non substitué, un di(C1-C30)alkyl(C6-C30)arylsilyle substitué ou non substitué, un (C1-C30)alkyldi(C6-C30)arylsilyle substitué ou non substitué, un mono- ou di-(C1-C30)alkylamino substitué ou non substitué, un mono- ou di-(C6-C30)arylamino substitué ou non substitué ou un (C1-C30)alkyl(C6-C30)arylamino substitué ou non substitué; ou peuvent être liés à un ou des substituant(s) adjacent(s) pour former un cycle (C3-C30), mono- ou polycyclique, alicyclique ou aromatique substitué ou non substitué dont le ou les atome(s) de carbone peu(ven)t être remplacé(s) avec au moins un hétéroatome choisi parmi azote, oxygène et soufre;
l'hétéroaryl(ène) et l'hétérocycloalkyle, chacun indépendamment, contiennent un ou plusieurs hétéroatomes choisis parmi B, N, O, S, P(=O), Si et P; et
s et t, chacun indépendamment, représentent un nombre entier de 1 à 4; lorsque s ou t est un nombre entier de 2 ou plus, les R₁₅ ou R₁₆ peuvent être chacun identiques ou différents.

5. Matériau tampon d'électrons selon la revendication 4, dans lequel Ars et Ar₆, chacun indépendamment, représentent un (6 à 20 membres)hétéroaryle substitué ou non substitué ou un (C6-C20)aryle substitué ou non substitué; L' représente une liaison simple ou un (C6-C20)arylène substitué ou non substitué; R₁₅ et R₁₆, chacun indépendamment, représentent un hydrogène, un (C6-C20)aryle substitué ou non substitué ou un (5 à 20 membres)hétéroaryle substitué ou non substitué, ou peuvent être liés à un ou des substituant(s) adjacent(s) pour former un cycle (C5-C20), mono- ou polycyclique, aromatique dont le ou les atome(s) de carbone peu(ven)t être remplacé(s) avec au moins un hétéroatome choisi parmi azote, oxygène et soufre; et s et t représentent 1.

6. Matériau tampon d'électrons selon la revendication 4, dans lequel le composé de la formule 11 est choisi dans le groupe constitué de:

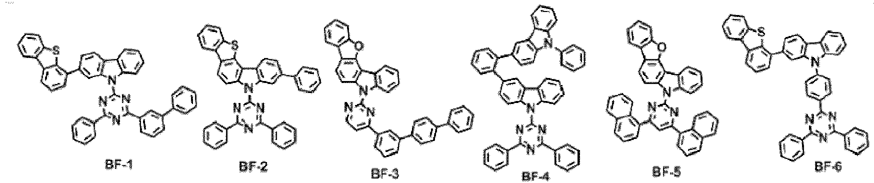

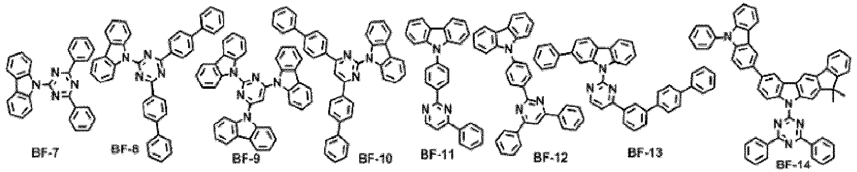

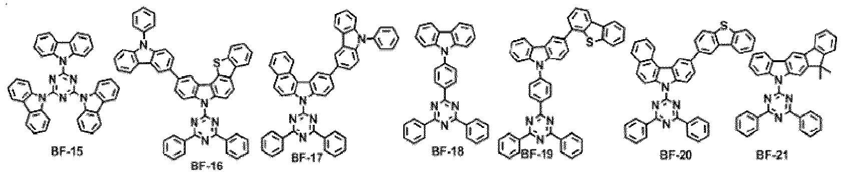

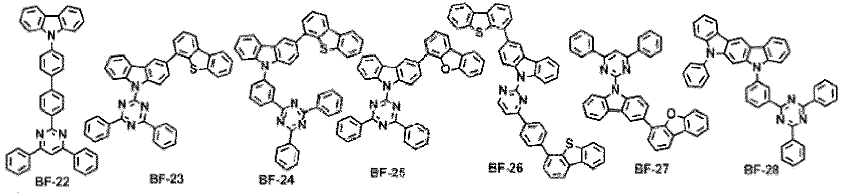

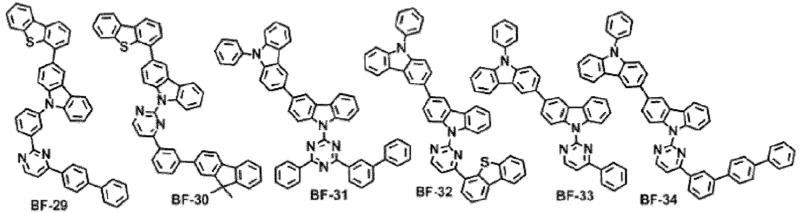

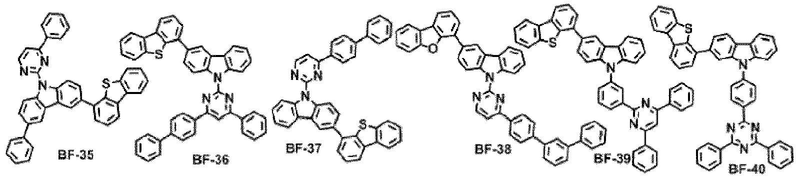

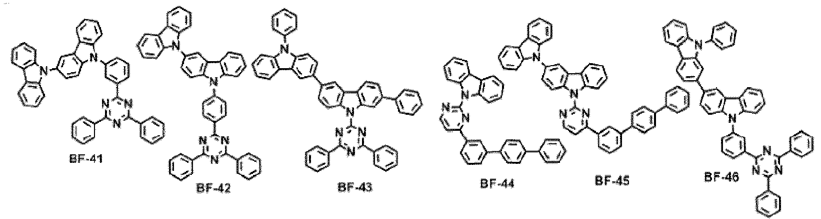

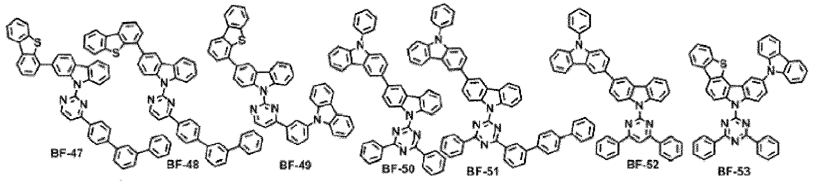

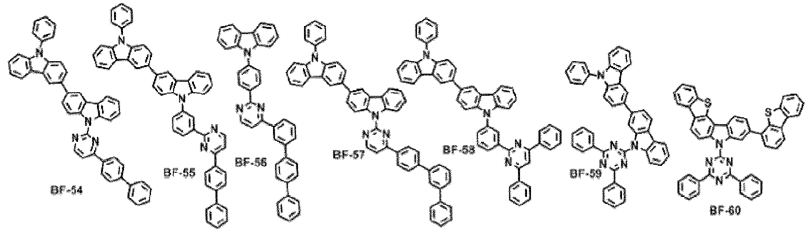

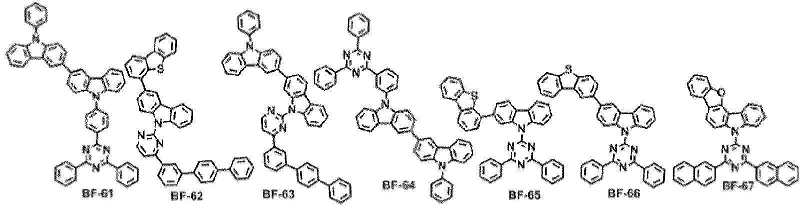

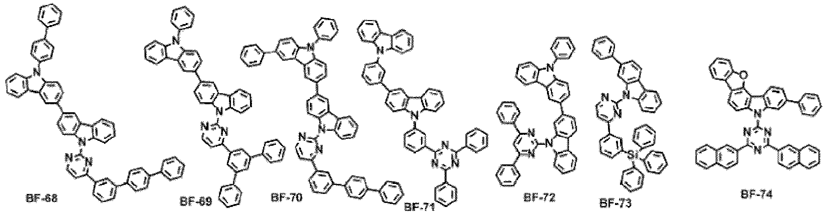

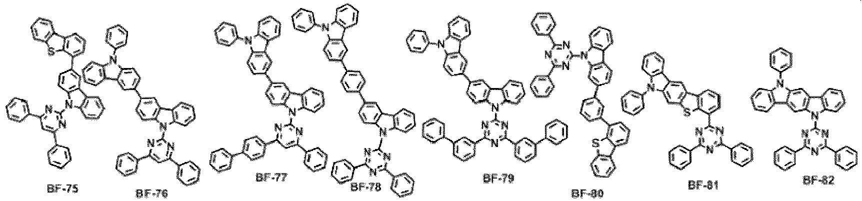

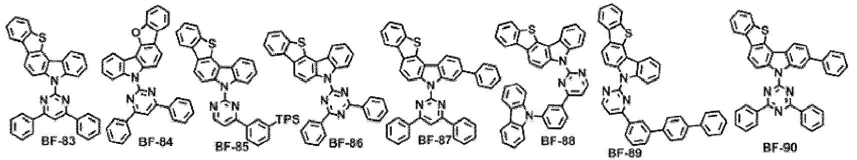

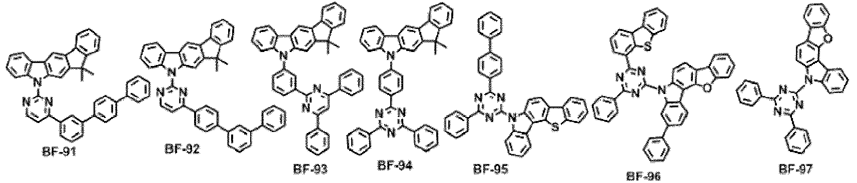

7. Dispositif électroluminescent organique comprenant une première électrode, une deuxième électrode faisant face à la première électrode, une couche émettant une lumière entre la première électrode et la deuxième électrode et une zone de transport d'électrons et une couche tampon d'électrons entre la couche émettant une lumière et la deuxième électrode, dans lequel la couche tampon d'électrons comprend le composé représenté par la formule 1 selon la revendication 1.

8. Dispositif électroluminescent organique selon la revendication 7, dans lequel la couche tampon d'électrons comprend en outre le composé représenté par la formule 11 selon la revendication 4.

9. Dispositif électroluminescent organique selon la revendication 8, dans lequel le composé représenté par la formule 1 et le composé représenté par la formule 11 de la couche tampon d'électrons sont co-évaporés ou évaporés par mélange.
